(19) Europäisches Patentamt
European Patent Office
Office européen des brevets

(11) **EP 3 727 287 B1**

(12) **EUROPEAN PATENT SPECIFICATION**


(45) Date of publication and mention of the grant of the patent:
**04.12.2024 Bulletin 2024/49**

(21) Application number: **18826717.3**

(22) Date of filing: **21.12.2018**

(51) International Patent Classification (IPC):
***A61K 8/24*** *(2006.01)* ***A61K 8/9783*** *(2017.01)*
***A61K 8/35*** *(2006.01)* ***A61K 8/49*** *(2006.01)*
***A61Q 5/06*** *(2006.01)*

(52) Cooperative Patent Classification (CPC):
**A61K 8/24; A61K 8/355; A61K 8/492; A61K 8/9783; A61Q 5/065;** A61K 2800/43; A61K 2800/884

(86) International application number:
**PCT/EP2018/086589**

(87) International publication number:
**WO 2019/122331 (27.06.2019 Gazette 2019/26)**


(54) **HAIR DYEING PROCESS COMPRISING A STEP OF DYEING WITH HENNA AND/OR INDIGO AND A TREATMENT STEP COMPRISING THE APPLICATION OF A COMPOSITION COMPRISING A BUFFER SYSTEM**

HAARFÄRBEVERFAHREN MIT EINEM SCHRITT DES FÄRBENS MIT HENNA UND/ODER INDIGO UND EINEM BEHANDLUNGSSCHRITT MIT DER ANWENDUNG EINER ZUSAMMENSETZUNG MIT EINEM PUFFERSYSTEM

PROCÉDÉ DE TEINTURE DES CHEVEUX COMPRENANT UNE ÉTAPE DE TEINTURE AU HENNÉ ET/OU INDIGO ET UNE ÉTAPE DE TRAITEMENT COMPRENANT L'APPLICATION D'UNE COMPOSITION COMPRENANT UN SYSTÈME TAMPON


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2017 FR 1763107**

(43) Date of publication of application:
**28.10.2020 Bulletin 2020/44**



(73) Proprietor: **L'OREAL**
**75008 Paris (FR)**

(72) Inventors:
- **HERCOUET, Leila**
**93400 Saint-Ouen (FR)**
- **LALLEMAN, Boris**
**93400 Saint-Ouen (FR)**
- **AGACH, Mickaël**
**93400 Saint Ouen (FR)**
- **PUECH, Julia**
**93400 Saint Ouen (FR)**
- **YAMADA, Hidetoshi**
**213-0012 Kanagawa (JP)**

(74) Representative: **L'Oreal**
**Service D.I.P.I.**
**9, rue Pierre Dreyfus**
**92110 Clichy (FR)**



(56) References cited:
**FR-A1- 3 014 315**

- **ANONYMOUS: "Palette by Nature Instruction", 13 April 2022 (2022-04-13), pages 1 - 1, XP055912752, Retrieved from the Internet <URL:http://www.palettebynature.com/v/pdf/PBN-Color-Application-Guide.pdf> [retrieved on 20220413]**
- **ANONYMOUS: "Palette by Nature", 13 April 2022 (2022-04-13), pages 1 - 2, XP055912761, Retrieved from the Internet <URL:https://www.suvarna.co.uk/user/page2_instruc.pdf> [retrieved on 20220413]**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE GNPD [online] MINTEL; 2 May 2017 (2017-05-02), ANONYMOUS: "Fashion Colore Natura", XP055502855, retrieved from https://www.gnpd.com/sinatra/recordpage/4779875/ Database accession no. 4779875
- DATABASE GNPD [online] MINTEL; 6 July 2017 (2017-07-06), ANONYMOUS: "Extra Intensive Toning Color", XP055502825, retrieved from https://www.gnpd.com/sinatra/recordpage/4930233/ Database accession no. 4930233
- DATABASE GNPD [online] MINTEL; 26 April 2011 (2011-04-26), ANONYMOUS: "All Natural Pemanent Hair Color", XP055502858, retrieved from https://www.gnpd.com/sinatra/recordpage/1522982/ Database accession no. 1522982
- DATABASE GNPD [online] MINTEL; 3 January 2013 (2013-01-03), ANONYMOUS: "Hair Colourant", XP055502853, retrieved from https://www.gnpd.com/sinatra/recordpage/1951929/ Database accession no. 1951929
- DATABASE GNPD [online] MINTEL; 2 May 2017 (2017-05-02), ANONYMOUS: "Fashion Colore Natura", XP055502855, retrieved from www.gnpd.com Database accession no. 4779875
- DATABASE GNPD [online] MINTEL; 26 April 2011 (2011-04-26), ANONYMOUS: "All Natural Pemanent Hair Color", XP055502858, retrieved from www.gnpd.com Database accession no. 1522982
- DATABASE GNPD [online] MINTEL; 3 January 2013 (2013-01-03), ANONYMOUS: "Hair Colourant", XP055502853, retrieved from www.gnpd.com Database accession no. 1951929
- DATABASE GNPD [online] MINTEL; 6 July 2017 (2017-07-06), ANONYMOUS: "Extra Intensive Toning Color", XP055502825, retrieved from www.gnpd.com Database accession no. 4930233
- ANONYMOUS: "Coloração Tonalizante", 30 August 2018 (2018-08-30), XP002784300, Retrieved from the Internet <URL:http://altamoda.com.br/wp-content/themes/thinkcyber/pdf/bula_tonalizante_17.pdf> [retrieved on 20180830]

## Description

The present invention relates to a multi-step process for dyeing keratin fibres, in particular human keratin fibres such as the hair, in which said fibres are treated with a composition comprising indigo and/or henna and a step comprising the application of an aqueous composition optionally comprising a synthetic or natural direct dye and a buffer system.

Two major methods for dyeing human keratin fibres, and in particular the hair, are known.

The first, known as oxidation dyeing or permanent dyeing, consists in using one or more oxidation dye precursors, more particularly one or more oxidation bases optionally combined with one or more couplers.

Usually, oxidation bases are selected from ortho- or para-phenylenediamines, ortho- or para-aminophenols, and heterocyclic compounds. These oxidation bases are colourless or weakly coloured compounds, which, when combined with oxidizing products, can give rise via a process of oxidative condensation to coloured species, which remain trapped within the fibre.

The shades obtained with these oxidation bases are often varied by combining them with one or more couplers, these couplers being chosen in particular from aromatic meta-diamines, meta-aminophenols, meta-diphenols and certain heterocyclic compounds, such as indole compounds.

The variety of molecules used as oxidation bases and couplers allows a wide range of colours to be obtained.

The second dyeing method, known as direct dyeing or semi-permanent dyeing, comprises the application of direct dyes, which are coloured and colouring molecules that have affinity for fibres. Given the nature of the molecules used, they tend rather to remain on the surface of the fibre and penetrate relatively little into the fibre, when compared with the small molecules of oxidation dye precursors. The main advantages of this type of dyeing are that it does not require any oxidizing agent, which limits the degradation of the fibres, and that it does not use any dyes that have particular reactivity, resulting in limitation of the intolerance risks.

The first hair dyes were semi-permanent. One of the most well-known natural dyes is that derived from the henna plant. Henna continues to be used in feminine beauty enhancement for colouring the hair or the nails, or for dyeing leather, silk and wool, etc. It is also used traditionally for various important events, celebrations and beliefs.

Red henna is constituted of leaves of shrubs of the genus *Lawsonia* from the family *Lythraceae,* which is based on the principle of dyeing with the active agent lawsone: 2-hydroxy-1,4-naphthoquinone. Lawsone [83-72-7] (CI Natural Orange 6; CI 75420), also known as isojuglone, may be found in henna shrubs (*Lawsonia alba, Lawsonia inermis*) ("Dyes, Natural", Kirk-Othmer Encyclopedia of Chemical Technology*, "*Henna" Encyclopedia Britannica*).*

This dye affords an orange-red colouration on grey hair, and a "warm" i.e. coppery to red colour on chestnut-brown hair. The dyeing process using henna is difficult to perform. A kind of "paste" (often referred to as a "poultice") is first made from ground or powdered henna leaves, which is then diluted at the time of use with warm water, and said paste is then applied to the keratin fibres.

Another very well-known natural dye is indigo (see Ullmann's Encyclopedia of Industrial Chemistry, Hair preparation, point 5.2.3, 2006 Wiley-VCH Verlag GmbH & Co. KGaA, Weinheim; 10.1002/14356007.a12 571.pub2). Indigo continues to be used for feminine beauty enhancement by dyeing the hair or the nails, or for dyeing fabrics (jeans), leather, silk, wool, etc. Indigo [482-89-3] is a natural dye, originating in particular from the indigo plant, and having the empirical formula: $C_{16}H_{10}N_2O_2$; and having the structure:

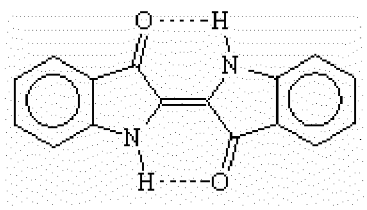

Indigo is derived from indican and may be prepared from various plants known as indigo-producing plants such as *Indigofera tinctoria, Indigo suffruticosa, Isatis tinctoria,* etc. (see Kirk-Othmer Encyclopedia of Chemical Technology, updated on 17/04/2009, DOI: 10.1002/0471238961.0425051903150618.a01.pub2). The indigo-producing plants are generally chopped and soaked in hot water, heated, fermented and oxidized in the open air to liberate the purple-blue coloured indigo (see Chem. Rev. 2011, 111, 2537-2561, pages 2537-2561). Indigo is the result of the fermentation and then oxidization of indican (glycosyl precursor). The indigo molecule is insoluble in water.

The problem is that dyeing using the indigo leaf is difficult because the uptake of the colour into the keratin fibres is very poor. This dye affords a blue colouring on grey hair, and a "cold" colour of ash to violet type on chestnut-brown hair. The dyeing process using indigo leaves is difficult to perform. A kind of "paste" (often referred to as a poultice) is first made from ground or powdered leaves of indigo plant (or Indian indigo or dyer's indigo) or dyer's pastel (or woad or *Isatis tinctoria*), which needs to have been fermented, and which is then diluted at the time of use with warm water,

and said paste is then applied to the keratin fibres.

When hair is dyed with these two types of dyes, for as much as the colour obtained on chestnut-brown hair has a natural effect, grey hair is dyed in an unaesthetic and unnatural orange colour with henna or blue with indigo. Furthermore, the colourings obtained are not homogeneous between the root and the end or from one fibre to another.

In particular, dyeing using indigo leaf is difficult since the dyeing kinetics in the keratin fibres are variable and furthermore the dyeing process is unstable. Indigo theoretically affords a blue colouring on grey hair, and a "cold" colour of ash type on chestnut-brown hair. However, the dyeing process using indigo is difficult to control due to the competing reaction for the formation of indirubin (which also involves an oxidation step with intermediate formation of yellow-coloured isatin) giving yellow to violet tints complementary to chestnut-brown hair over time.

The colourings resulting from mixtures of indigo and henna are thus generally evolutive over time in terms of colour, they have a characteristic yellow/green colour on the day of application (relatively unaesthetic "raw" colour, which dyeing consumers appreciate little) which change towards the desired colourings after a few hours (48 hours to 1 week) and may change colour over time (appearance of violet-red tints generally after 2 to 3 weeks). There is thus a need to stabilize this colouring to obtain an aesthetic colouring on the day of application (for example brown free of yellow/green tints) which changes little over time (no colour change).

Some process involving the application of a composition comprising indigo and/or henna and the application of a composition involving some acids and bases are known ( FR3014315A1).

To overcome this colour change problem and to broaden the range of shades obtained with these dyes, synthetic or natural direct dyes may be added, in particular to mask the undesirable tints. It is especially possible to use anionic synthetic direct dyes (also known as direct acid dyes), which make it possible to obtain excellent performance qualities in terms of persistence of the colouring on the fibre, but often have the drawback of also pigmenting the scalp. Natural dyes may also stain the skin and the concentrations generally used to obtain sufficient colouring of the hair amplifies the risk of staining of the scalp.

In addition, in order to improve the uptake of dyes and to mask the undesirable tints as much as possible, "hydrotropic" solvents may be used in relatively large amount or the concentration of acid dyes may be increased, but this has the drawback of giving rise to greater staining of the scalp.

Consequently, there is a need to develop a novel direct dyeing process which does not have the above drawbacks.

In particular, there is a need to develop natural dyeing processes based on henna and/or indigo which make it possible to obtain powerful, aesthetic and natural colourings from the end of application and which make it possible especially to obtain rapid colourings, of which the colouring obtained has no tints, in particular yellow/green tints, that are considered by users to be unaesthetic, and which have good colour buildup, are less aggressive to the hair and at the same time are resistant to external agents (light, bad weather, shampooing washing), which are persistent and/or homogeneous while at the same time remaining powerful and/or chromatic, and which are stable and do not change in colour over time, in particular towards red tints.

In addition, the dyeing process should not stain the scalp, or it should minimize the staining of the scalp.

The Applicant has discovered that a two-step process using a dye composition based on henna and/or indigo and a composition comprising a buffer system and optionally a direct dye makes it possible to solve the problems mentioned above.

This (these) aim(s) are achieved by the present invention, one subject of which is a process for dyeing keratin fibres, in particular human keratin fibres such as the hair, in which said fibres are treated, in several separate steps, comprising:

i) at least one step i) of dyeing said fibres using a cosmetic dye composition A, preferably an aqueous composition, comprising at least a) indigo and/or henna,
ii) at least one step ii) of treating said fibres, comprising the application to the fibres of an aqueous composition B which comprises water, b) optionally at least one direct dye, other than henna and indigo, c) a buffer system comprising a mixture of an acid and of at least one conjugate salt thereof, composition B having a pH of less than or equal to 5,
iii) optionally at least one step of intermediate rinsing of said fibres, preferably with water, between step i) and step ii) (or between step ii) and i) (depending on the order of steps i) and ii)).

Preferably, the step of treating with composition A is the first step of the dyeing process according to the invention.

Preferably, the process involves, in the following order:

- step i) of treating said fibres with a dye composition A and then
- step ii) of dyeing with composition B.

Compositions A and B are different compositions.

Preferably, the process according to the invention comprises an intermediate rinsing step iii) between step i) and step ii).

Preferably, the process according to the invention comprises a final rinsing step after performing all the steps of the process.

The process according to the invention has the advantage of dyeing keratin fibres, especially human keratin fibres, with powerful, chromatic dyeing results that are resistant to washing, perspiration, sebum and light, without impairing the fibres. Furthermore, the colourings obtained using the process according to the invention are sparingly selective, i.e. they have a uniform colour between the root and the end of the fibres. Furthermore, the dyeing process used can induce very satisfactory "buildup" and/or strength of the colouring.

The use of a step involving a composition comprising direct dyes, in particular acid dyes, and a buffer system makes it possible to maintain the acidic pH of the composition at a constant value and allows excellent dyeing performance qualities to be obtained, in particular in terms of colour buildup, while at the same time not staining the scalp, or minimizing the staining of the scalp without it being necessary to significantly increase the concentration of solvents or of acid dyes.

The process for dyeing keratin fibres according to the invention has the advantage of dyeing said fibres, especially human keratin fibres, in particular the hair, with natural dyeing results without any yellow/green tints, and/or powerful, chromatic colourings, which are resistant to washing, perspiration, sebum and light, and which are moreover long-lasting, without impairment. Furthermore, the colourings obtained using the process are uniform from the root to the end of a fibre (little colour selectivity).

The compositions used according to the invention are cosmetic compositions, i.e. they are cosmetically acceptable and are thus suitable for use for application to keratin fibres, especially for application to human keratin fibres such as the hair.

Preferably, composition A is obtained by mixing, just before use, indigo and/or henna with an aqueous composition to obtain a ready-to-use dye composition A, preferably in the form of a poultice.

Preferably, composition A is an aqueous composition.

Other subjects, characteristics, aspects and advantages of the present invention will emerge even more clearly on reading the description and the examples that follow.

***STEP i):***

Step i) of dyeing uses a cosmetic dye composition A comprising at least indigo and/or henna.

**a) *Henna and/or indigo***

According to the present invention, the term "henna" refers to a henna plant powder and/or a henna plant dye extract, preferably from a henna plant such as *Lawsonia alba* or *Lawsonia inermis.* The henna plant powder and/or dye extract especially comprises lawsone and/or a glucosyl precursor thereof.

Preferably, the henna used according to the present invention is in powder form.

According to the present invention, the term "indigo" refers to an indigo-producing plant powder and/or an indigo-producing plant dye extract, preferably *Indigofera tinctoria* or *Isatis tinctoria.* The indigo-producing plant powder and/or dye extract especially comprises indigo and/or indirubin and/or a glucosyl precursor thereof, such as indican and/or an isatan.

Preferably, the indigo used according to the present invention is in powder form.

It is understood that the henna powder and the indigo-producing plant powder are different from an extract. Specifically, an extract is a product of maceration in solvents, generally organic solvents, whereas the powder according to the invention is a natural product originating from henna or indigo-producing plants, reduced by grinding or other mechanical means, into fine particles.

Preferably, the henna used in the invention is red henna (*Lawsonia inermis, alba*). Lawsone [83-72-7] (CI Natural Orange 6; C! 75420), also known as isojuglone, may be found in henna shrubs (*Lawsonia alba, Lawsonia inermis*). Preferably, the henna is in powder form. The henna powder may be screened to obtain particles with upper limit sizes corresponding to the orifices or mesh sizes of the screen particularly between 35 and 80 mesh (US). According to a particular mode of the invention, the size of the henna powder particles is fine. According to the invention, a particle size of less than or equal to 500 μm is more particularly intended. Preferentially, the powder is constituted of fine particles with sizes inclusively between 10 and 300 μm and more particularly between 50 and 250 μm. It is understood that said henna particles preferentially have a moisture content of between 0 and 10% by weight, relative to the total weight of the powders.

Preferably, said henna particles are derived from henna leaves.

As indigo-producing plants, mention may be made of numerous species derived from the following genera:

- *Indigofera* such as *Indigofera tinctoria, Indigo suffraticosa, Indigofera articulata, Indigofera arrecta, Indigofera gerardiana, Indigofera argenta, Indigofera indica, Indigofera longiracemosa;*
- *Isatis* such as *Isatis tinctoria;*
- *Polygonum* or *Persicaria* such as *Polygonum tinctorium (Persicaria tinctoria)*;
- *Wrightia* such as *Wrightia tinctoria*;
- *Calanthe* such as *Calanthe veratrifolia*; and
- *Baphicacanthus* such as *Baphicacanthus cusia.*

Preferably, the indigo-producing plant is of the genus *Indigofera* and more particularly is *Indigofera tinctoria.*
Use may be made of all or part (in particular the leaves in particular for *Indigofera tinctoria*) of the indigo-producing plant.
The indigo-producing plant powder may be screened to obtain particles with upper limit sizes corresponding to the orifices or mesh sizes of the screen particularly between 35 and 80 mesh (US).
According to a particular mode of the invention, the size of the indigo-producing plant powder particles is fine. According to the invention, a particle size of less than or equal to 500 μm is more particularly intended. Preferentially, the powder is constituted of fine particles with sizes inclusively between 10 and 300 μm and more particularly between 50 and 250 μm.
It is understood that said indigo-producing plant particles preferentially have a moisture content of between 0 and 10% by weight relative to the total weight of the powders.
In one embodiment, the indigo is in the form of indigo-producing plant powder.
In another embodiment, the indigo is in the form of indigo-producing plant dye extract.
Composition A used in the keratin fibre dyeing process preferably comprises at least 0.1% by weight of henna and/or indigo, relative to the total weight of said composition, more preferentially from 0.2% to 50% by weight, better still from 0.3% to 40% by weight, preferably from 1% to 30% by weight relative to the total weight of composition A.
Preferably, the dye composition A is an aqueous composition and comprises at least water. Preferably, according to this embodiment, composition A comprises a water content ranging from 10% to 99% by weight, more particularly from 20% to 90% by weight and better still from 40% to 80% by weight relative to the weight of composition A.
According to a particular embodiment of the invention, composition A is obtained by mixing just before use (i.e. extemporaneously) the indigo and/or the henna with water or with an aqueous composition to obtain a ready-to-use dye composition A, preferably in the form of a poultice.

*Additional dyes*

According to one embodiment, composition A used in the dyeing process of the invention may also contain one or more additional direct dyes, in particular synthetic dyes or dyes of natural origin, other than indigo and henna; these dyes may be chosen from the dyes b) mentioned hereinbelow.
When it is (they are) present, the natural or synthetic direct dye(s), other than indigo and henna, used in the process of the invention particularly represent from 0.001 % to 10% by weight relative to the total weight of composition A and even more preferentially from 0.05% to 5% by weight relative to the total weight of the composition.
Preferably, the compositions of the invention do not contain any synthetic direct dyes, i.e. dyes that do not occur in nature.
According to one embodiment, composition A does not comprise any additional dyes other than henna and/or indigo.

***STEP ii):***

The treatment step ii) comprises the application to the fibres of an aqueous composition B which comprises a buffer system comprising a mixture of an acid and of at least one conjugate salt thereof, and optionally a synthetic or natural direct dye.

*b)* ***Direct dyes***

According to an advantageous embodiment, the aqueous composition B comprises b) one or more direct dyes chosen from synthetic direct dyes, natural dyes and mixtures thereof.
The direct dye b) is preferably other than henna and indigo as described previously.
Preferably, the dye b) is a synthetic direct dye, more preferably an anionic synthetic direct dye.

*Natural dyes*

The term "natural dyes" refers to dyes derived from natural materials (plant, mineral or animal origin), for instance extracts, ground material and decoctions, which have a greater or smaller concentration of dyes.

Included among the natural dyes according to the invention are compounds that may be present in nature and that are reproduced by chemical (semi)synthesis.

The natural dyes may be chosen especially from spinulosin, orceins, polyphenols or ortho-diphenols (also referred to as ODPs in the rest of the description), curcumin, indoles such as isatin or indole-2,3-dione, indigoids including indigo, phthalocyanines and porphyrins in particular complexed to a metal, glycosyl or non-glycosyl iridoids, chromene dyes, anthraquinone and naphthoquinone dyes, juglone, spinulosin, chromene or chroman dyes, such as neoflavanols and neoflavanones, flavanols; anthocyanidols, orceins, betalains, and mixtures thereof.

Use may also be made of extracts or decoctions containing these natural dyes and especially plant extracts or poultices containing said dyes.

These natural dyes may be added in the form of defined compounds from extracts or from plant parts. Said defined compounds from extracts or from plant parts are preferably in the form of powders, in particular fine powders whose particles have sizes identical to that of the henna and indigo-producing plant powders as defined previously.

The ODP(s) may or may not be salified. They may also be in aglycone form (without bonded sugars) or in the form of glycosylated compounds. Use may be made, for example, of ortho-diphenols such as those described in patent application FR 3 029 405. They may be synthetic or natural.

More particularly, the ODP(s) that may be used in the process of the invention are in particular:

- flavanols, for instance catechin and epicatechin gallate,
- flavonols, such as quercetin,
- anthocyanidins, for instance cyanidin, delphinidin and petunidin,
- anthocyanins or anthocyans, such as myrtillin,
- ortho-hydroxybenzoates, for example gallic acid salts,
- flavones, for instance luteolin,
- hydroxystilbenes, for example 3,3',4,5'-tetrahydroxystilbene, optionally oxylated (for example glucosylated),
- 3,4-dihydroxyphenylalanine and derivatives thereof,
- 2,3-dihydroxyphenylalanine and derivatives thereof,
- 4,5-dihydroxyphenylalanine and derivatives thereof,
- dihydroxycinnamates, such as caffeic acid and chlorogenic acid,
- ortho-polyhydroxycoumarins,
- ortho-polyhydroxyisocoumarins,
- ortho-polyhydroxycoumarones,
- ortho-polyhydroxyisocoumarones,
- ortho-polyhydroxychalcones,
- ortho-polyhydroxychromones,
- quinones,
- hydroxyxanthones,
- 1,2-dihydroxybenzene and derivatives thereof,
- 1,2,4-trihydroxybenzene and derivatives thereof,
- 1,2,3-trihydroxybenzene and derivatives thereof,
- 2,4,5-trihydroxytoluene and derivatives thereof,
- proanthocyanidins and especially the proanthocyanidins A1, A2, B1, B2, B3 and C1,
- chroman and chromene compounds,
- proathocyanins,
- tannic acid,
- ellagic acid,
- and mixtures of the preceding compounds.

Preferably, the ODPs of the invention are chromenes or chromans and are preferably chosen from haematein, haematoxylin, brazilein, brazilin and santalin A. Examples that may be mentioned include haematoxylin (Natural Black 1 according to the INCI name) and brazilin (Natural Red 24 according to the INCI name), dyes of the indochroman family, which are commercially available. The latter dyes may exist in an oxidized form and may be obtained synthetically or by extraction of plants or vegetables known to be rich in these dyes. The ODPs may be used in the form of extracts. Use may be made of the following plant extracts (genus and species): *Haematoxylon campechianum, Haematoxylon brasiletto,* Quebracho *(Schinopsis lorentsii), Caesalpinia echinata, Caesalpinia sappan, Caesalpinia spinosa* and *Cae-*

*salpinia brasiliensis.*

According to one embodiment, the natural ODPs are derived from extracts of animals, bacteria, fungi, algae, plants and fruits, used in their entirety or partially. In particular regarding plants, the extracts are derived from fruit, including citrus fruit, from vegetables, from trees and from shrubs. Use may also be made of mixtures of these extracts, which are rich in ODPs as defined above.

Preferably, the natural ODP(s) of the invention are derived from extracts of plants or plant parts.

The extracts are obtained by extraction of various plant parts, for instance the root, the wood, the bark, the leaf, the flower, the fruit, the seed, the pod or the peel.

Mention may be made, among the extracts of plants, of extracts of rose or tea leaves.

Mention may be made, among the extracts of fruits, of extracts of apple, extracts of grape (in particular of grape seed) or extracts of cocoa beans and/or pods.

Mention may be made, among the extracts of vegetables, of extracts of potato or of onion peel.

Among the extracts of tree wood, mention may preferably be made of extracts of pine bark, extracts of campeachy wood, pernambouc wood, sappan wood and brazil wood. Examples of campeachy wood extracts that may be used include the extract whose INCI name is EXTRAIT DE CAMPÊCHE OXYDE [Oxidized campeachy extract] (*Haematoxylon campechianum*) sold by SCRD under the reference HEMATINE HCK S 21.

Preferentially, the ODP(s) are chosen from catechin, quercetin, haematein, haematoxylin, brazilin, brazilein, gallic acid and tannic acid, and natural extracts containing them chosen from grape marc, pine bark, green tea, onion, cocoa bean, campeachy wood, redwood and gall nut, and mixtures thereof.

Mention may also be made of the natural dyes chosen from the compounds of formula (a) or (b) below, or mixtures thereof:

(a)

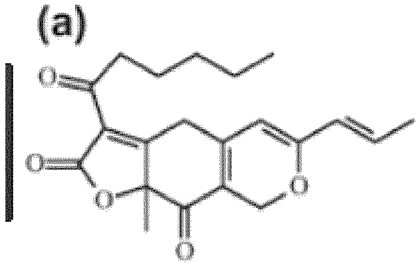

(b)

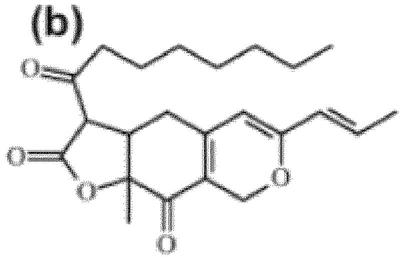

Such compounds are, for example, extracted from moulds of the species *Monascus purpureus* (synonyms: *M. albidus, M. anka, M. araneosus, M. major, M. rubiginosus* and *M. vini)-*

As preferred natural dyes, mention may be made especially of carmine, carmine ammonium, diosindigo, chlorophyllin, haematein, orcein, the following extracts: blackcurrant, blueberry, black rice, grape skin, hibiscus, red cabbage, black carrot, elderberry, rhubarb, monascus, purple sweet potato, goji, radish, orcein, gardenia, diospyros kaki, campeachy, quebracho, and mixtures thereof.

<u>*Synthetic direct dyes*</u>

The synthetic direct dyes are chosen, for example, from those conventionally used in direct dyeing, and among which mention may be made of any commonly used aromatic and/or nonaromatic dye such as neutral, acidic or cationic nitrobenzene direct dyes, neutral, acidic or cationic azo direct dyes, neutral, acidic or cationic quinone and in particular anthraquinone direct dyes, azine, triarylmethane, indoamine, methine, styryl, porphyrin, metalloporphyrin, phthalocyanine, cyanine and methine direct dyes, and fluorescent dyes.

According to a preferred embodiment of the invention, the direct dye(s), preferably synthetic dyes, that may be used according to the invention are chosen from anionic dyes, commonly referred to as "acid" direct dyes on account of their affinity for alkaline substances. The anionic direct dyes according to the invention may be natural or synthetic.

The term "anionic direct dyes" means any direct dye comprising in its structure at least one $CO_2R$ or $SO_3R$ substituent with R denoting a hydrogen atom or a cation originating from a metal or an amine, or an ammonium ion. The anionic dyes may be chosen from direct nitro acid dyes, azo acid dyes, azine acid dyes, triarylmethane acid dyes, indoamine acid dyes, anthraquinone acid dyes, indigoids and natural acid dyes.

As anionic (or acid) direct dyes that may be used according to the invention, mention may be made especially of the dyes of formulae (I), (II), (III), (IV), (V), (VI), (VII), (VIII), (IX), (X), (XI) and (XII) below:

a) the diaryl anionic azo dyes of formula (I) or (II):

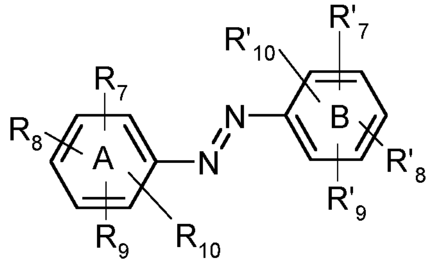

(I)

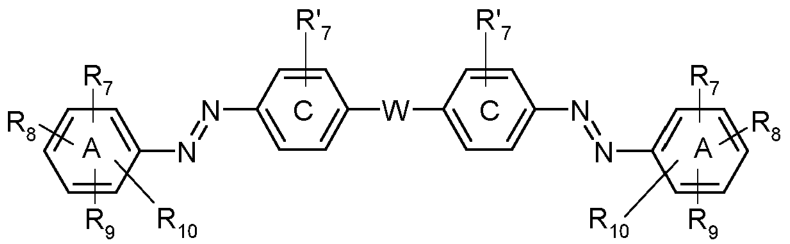

(II)

in which formulae (I) and (II):

- $R_7$, $R_8$, $R_9$, $R_{10}$, $R'_7$, $R'_8$, $R'_9$ and $R'_{10}$, which may be identical or different, represent a hydrogen atom or a group chosen from:

  - alkyl;
  - alkoxy, alkylthio;
  - hydroxyl, mercapto;
  - nitro, nitroso;
  - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
  - $(O)_2S(O^-)$-, $M^+$ with $M^+$ representing a hydrogen atom or a cationic counterion;
  - $(O)CO^-$-, $M^+$ with $M^+$ as defined previously;
  - R"-$S(O)_2$-, with R" representing a hydrogen atom or an alkyl, aryl, (di)(alkyl)amino or aryl(alkyl)amino group; preferentially a phenylamino or phenyl group;
  - R‴-$S(O)_2$-X'- with R‴ representing an alkyl or optionally substituted aryl group, X' as defined previously;
  - (di)(alkyl)amino;
  - aryl(alkyl)amino optionally substituted with one or more groups chosen from i) nitro; ii) nitroso; iii) $(O)_2S(O^-)$-, $M^+$ and iv) alkoxy, with $M^+$ as defined previously;
  - optionally substituted heteroaryl; preferentially a benzothiazolyl group;
  - cycloalkyl; in particular cyclohexyl;
  - Ar-N=N- with Ar representing an optionally substituted aryl group; preferentially a phenyl optionally substituted with one or more alkyl, $(O)_2S(O^-)$-, $M^+$ or phenylamino groups;
  - or alternatively two contiguous groups $R_7$ with $R_8$ or $R_8$ with $R_9$ or $R_9$ with $R_{10}$ together form a fused benzo group A'; and $R'_7$ with $R'_9$ or $R'_9$ with $R'_9$ or $R'_9$ with $R'_{10}$ together form a fused benzo group B'; with A' and B' optionally substituted with one or more groups chosen from i) nitro; ii) nitroso; iii) $(O)_2S(O^-)$-, $M^+$; iv) hydroxyl; v) mercapto; vi) (di)(alkyl)amino; vii) R°-C(X)-X'-; viii) R°-X'-C(X)-; ix) R°-X'-C(X)-X"-; x) Ar-N=N- and xi) optionally substituted aryl(alkyl)amino; with $M^+$, R°, X, X', X" and Ar as previously defined;

- W represents a sigma bond $\sigma$, an oxygen or sulfur atom, or a divalent radical i) - NR- with R as defined previously, or ii) methylene -$C(R_a)(R_b)$- with $R_a$ and $R_b$, which may be identical or different, representing a hydrogen atom or an aryl group, or alternatively $R_a$ and $R_b$ form, together with the carbon atom that bears them, a spiro cycloalkyl; preferentially, W represents a sulfur atom or $R_a$ and $R_b$ together form a cyclohexyl;

it being understood that formulae (I) and (II) comprise at least one sulfonate radical $(O)_2S(O^-)$-, $M^+$ or one carboxylate radical $(O)CO^-$-, $M^+$ on one of the rings A, A', B, B' or C; preferentially sodium sulfonate.

As examples of dyes of formula (I), mention may be made especially of: Acid Red 1, Acid Red 4, Acid Red 13, Acid Red 14, Acid Red 18, Acid Red 27, Acid Red 28, Acid Red 32, Acid Red 33, Acid Red 35, Acid Red 37, Acid Red 40, Acid Red 41, Acid Red 42, Acid Red 44, Pigment Red 57, Acid Red 68, Acid Red 73, Acid Red 135, Acid Red 138, Acid Red 184, Food Red 1, Food Red 13, Acid Orange 6, Acid Orange 7, Acid Orange 10, Acid Orange 19, Acid Orange 20, Acid Orange 24, Yellow 6, Acid Yellow 9, Acid Yellow 36, Acid Yellow 199, Food Yellow 3, Acid Violet 3, Acid Violet 7, Acid Violet 14, Acid Blue 113, Acid Blue 117, Acid Black 1, Acid Brown 4, Acid Brown 20, Acid Black 26, Acid Black 52, Food Black 1, Food Black 2 and Food Yellow 3 or Sunset Yellow.

As examples of dyes of formula (II), mention may be made especially of: Acid Red 111, Acid Red 134 and Acid yellow 38;

b) the pyrazolone anionic azo dyes of formulae (III) and (IV):

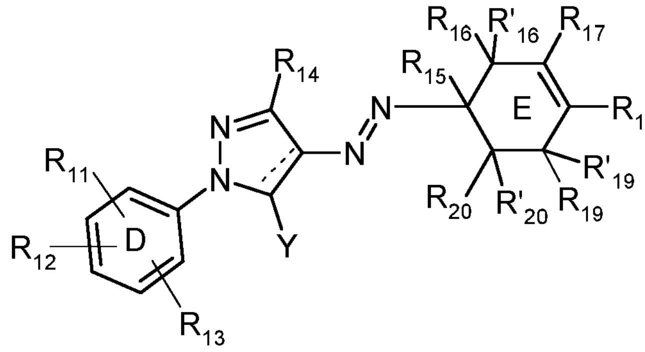

**(III)**

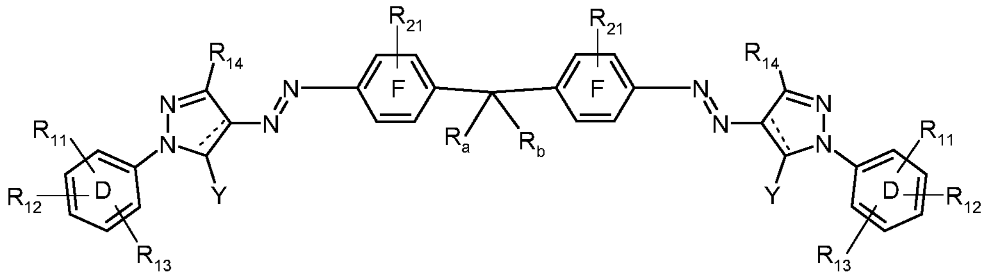

(IV)

in which formulae (III) and (IV):

- $R_{11}$, $R_{12}$ and $R_{13}$, which may be identical or different, represent a hydrogen or halogen atom, an alkyl group or $(O)_2S(O^-)$-, $M^+$ with $M^+$ as defined previously;
- $R_{14}$ represents a hydrogen atom, an alkyl group or a group $C(O)O^-$-, $M^+$ with $M^+$ as defined previously;
- $R_{15}$ represents a hydrogen atom;
- $R_{16}$ represents an oxo group, in which case $R'_{16}$ is absent, or alternatively $R_{15}$ with $R_{16}$ together form a double bond;
- $R_{17}$ and $R_{18}$, which may be identical or different, represent a hydrogen atom, or a group chosen from:
  - $(O)_2S(O^-)$-, $M^+$ with $M^+$ as defined previously;
  - Ar-O-$S(O)_2$- with Ar representing an optionally substituted aryl group; preferentially a phenyl optionally substituted with one or more alkyl groups;
- $R_{19}$ and $R_{20}$ together form either a double bond, or a benzo group D', which is optionally substituted;
- $R'_{16}$, $R'_{19}$ and $R'_{20}$, which may be identical or different, represent a hydrogen atom or an alkyl or hydroxyl group;
- $R_{21}$ represents a hydrogen atom or an alkyl or alkoxy group;
- $R_a$ and $R_b$, which may be identical or different, are as defined previously, preferentially $R_a$ represents a hydrogen atom and $R_b$ represents an aryl group;
- Y represents either a hydroxyl group or an oxo group;
- ------ represents a single bond when Y is an oxo group; and represents a double bond when Y represents a

hydroxyl group;

it being understood that formulae (III) and (IV) comprise at least one sulfonate radical $(O)_2S(O^-)$-, $M^+$ or one carboxylate radical C(O)O--, $M^+$ on one of the rings D or E; preferentially sodium sulfonate.

As examples of dyes of formula (III), mention may be made especially of: Acid Red 195, Acid Yellow 23, Acid Yellow 27 and Acid Yellow 76.

As an example of a dye of formula (IV), mention may be made especially of: Acid Yellow 17;

c) the anthraquinone dyes of formulae (V) and (VI):

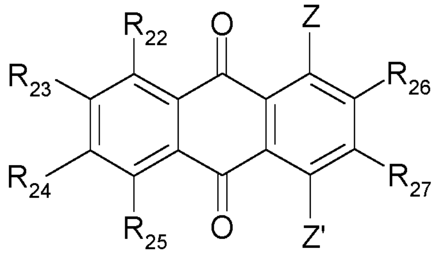

**(V)**

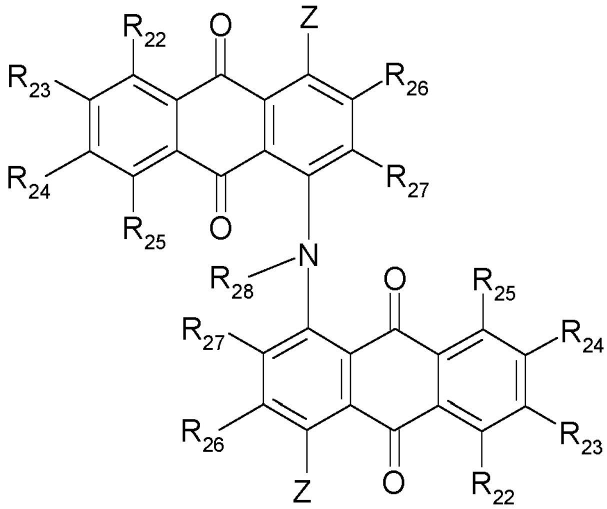

(VI)

in which formulae (V) and (VI):

- $R_{22}$, $R_{23}$, $R_{24}$, $R_{25}$, $R_{26}$ and $R_{27}$, which may be identical or different, represent a hydrogen or halogen atom, or a group chosen from:
    - alkyl;
    - hydroxyl, mercapto;
    - alkoxy, alkylthio;
    - optionally substituted aryloxy or arylthio; preferentially substituted with one or more groups chosen from alkyl and $(O)_2S(O^-)$-, $M^+$ with $M^+$ as defined previously;
    - aryl(alkyl)amino optionally substituted with one or more groups chosen from alkyl and $(O)_2S(O^-)$-, $M^+$ with $M^+$ as defined previously;
    - (di)(alkyl)amino;
    - (di)(hydroxyalkyl)amino;
    - $(O)_2S(O^-)$-, $M^+$ with $M^+$ as defined previously;
- Z' represents a hydrogen atom or a group $NR_{28}R_{29}$ with $R_{28}$ and $R_{29}$, which may be identical or different, representing a hydrogen atom or a group chosen from:

- alkyl;
- polyhydroxyalkyl such as hydroxyethyl;
- aryl optionally substituted with one or more groups, more particularly i) alkyl such as methyl, n-dodecyl, n-butyl; ii) $(O)_2S(O^-)$-, $M^+$ with $M^+$ as defined previously; iii) R°-C(X)-X'-, R°-X'-C(X)-, R°-X'-C(X)-X"- with R°, X, X' and X" as defined previously, preferentially R° represents an alkyl group;
- cycloalkyl; in particular cyclohexyl;

- Z represents a group chosen from hydroxyl and $NR'_{28}R'_{29}$ with $R'_{28}$ and $R'_{29}$, which may be identical or different, representing the same atoms or groups as $R_{28}$ and $R_{29}$ as defined previously;

it being understood that formulae (V) and (VI) comprise at least one sulfonate radical $(O)_2S(O^-)$-, $M^+$ or one carboxylate radical $C(O)O^-$-, $M^+$; preferentially sodium sulfonate.

As examples of dyes of formula (V), mention may be made especially of: Acid Blue 25, Acid Blue 43, Acid Blue 62, Acid Blue 78, Acid Blue 129, Acid Blue 138, Acid Blue 140, Acid Blue 251, Acid Green 25, Acid Green 41, Acid Violet 42, Acid Violet 43, Mordant Red 3 and EXT Violet N° 2.

As an example of a dye of formula (VI), mention may be made especially of: Acid Black 48;

d) the nitro dyes of formulae (VII) and (VIII):

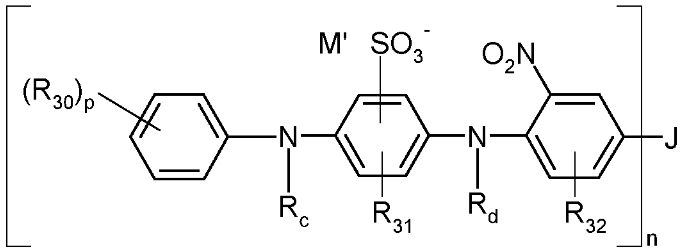

**(VII)**

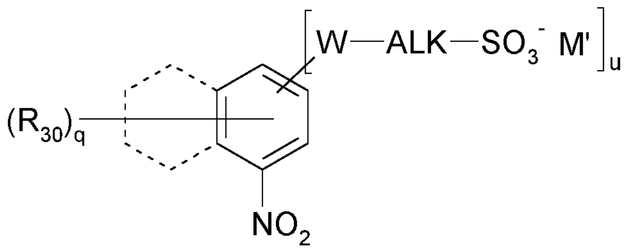

(VIII)

in which formulae (VII) and (VIII):

- $R_{30}$, $R_{31}$ and $R_{32}$, which may be identical or different, represent a hydrogen or halogen atom, or a group chosen from:

- alkyl;
- alkoxy optionally substituted with one or more hydroxyl groups, alkylthio optionally substituted with one or more hydroxyl groups;
- hydroxyl, mercapto;
- nitro, nitroso;
- polyhaloalkyl;
- R°-C(X)-X'-, R°-X'-C(X)-, R°-X'-C(X)-X"- with R°, X, X' and X" as defined previously;
- $(O)_2S(O^-)$-, $M^+$ with $M^+$ as defined previously;
- $(O)CO^-$-, $M^+$ with $M^+$ as defined previously;
- (di)(alkyl)amino;
- (di)(hydroxyalkyl)amino;
- heterocycloalkyl such as piperidino, piperazino or morpholino;

more particularly, $R_{30}$, $R_{31}$ and $R_{32}$ represent a hydrogen atom;

- $R_c$ and $R_d$, which may be identical or different, represent a hydrogen atom or an alkyl group;
- W is as defined previously; W more particularly represents an -NH- group;
- ALK represents a linear or branched divalent $C_1$-$C_6$ alkylene group; more particularly, ALK represents a $-CH_2-CH_2-$ group;
- n is 1 or 2;
- p represents an integer inclusively between 1 and 5;
- q represents an integer inclusively between 1 and 4;
- u is 0 or 1;
- when n is 1, J represents a nitro or nitroso group; more particularly nitro;
- when n is 2, J represents an oxygen or sulfur atom, or a divalent radical $-S(O)_m$-with m representing an integer 1 or 2; more preferentially, J represents a radical $-SO_2-$;
- M' represents a hydrogen atom or a cationic counterion;
- 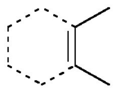 ,

  which may be present or absent, represents a benzo group optionally substituted with one or more $R_{30}$ groups as defined previously;

it being understood that formulae (VII) and (VIII) comprise at least one sulfonate radical $(O)_2S(O^-)$-, $M^+$ or one carboxylate radical $C(O)O^-$-, $M^+$; more preferentially sodium sulfonate.

As examples of dyes of formula (VII), mention may be made especially of: Acid Brown 13 and Acid Orange 3.

As examples of dyes of formula (VIII), mention may be made of: Acid Yellow 1, the sodium salt of 2,4-dinitro-1-naphthol-7-sulfonic acid, 2-piperidino-5-nitrobenzenesulfonic acid, 2-(4'-N,N(2"-hydroxyethyl)amino-2'-nitro)anilineethanesulfonic acid, 4-β-hydroxyethylamino-3-nitrobenzenesulfonic acid and EXT D&C yellow 7.

e) the triarylmethane dyes of formula (IX):

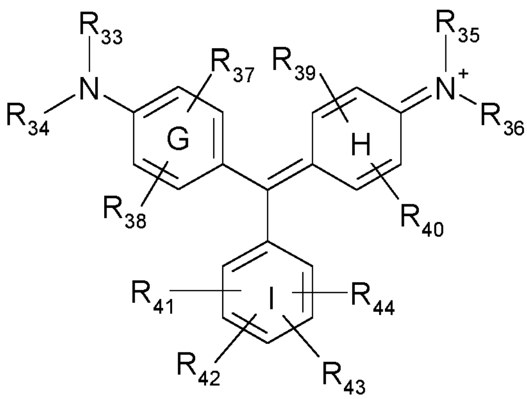

**(IX)**

in which formula (IX):

- $R_{33}$, $R_{34}$, $R_{35}$ and $R_{36}$, which may be identical or different, represent a hydrogen atom or a group chosen from alkyl, optionally substituted aryl and optionally substituted arylalkyl; more particularly an alkyl and benzyl group optionally substituted with a group $(O)_mS(O^-)$-, $M^+$ with $M^+$ and m as defined previously;
- $R_{37}$, $R_{38}$, $R_{39}$, $R_{40}$, $R_{41}$, $R_{42}$, $R_{43}$ and $R_{44}$, which may be identical or different, represent a hydrogen atom or a group chosen from:

  - alkyl;
  - alkoxy, alkylthio;
  - (di)(alkyl)amino;
  - hydroxyl, mercapto;

- nitro, nitroso;
- R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
- $(O)_2S(O^-)$-, $M^+$ with $M^+$ representing a hydrogen atom or a cationic counterion;
- (O)CO⁻-, $M^+$ with $M^+$ as defined previously;
- or alternatively two contiguous groups $R_{41}$ with $R_{42}$ or $R_{42}$ with $R_{43}$ or $R_{43}$ with $R_{44}$ together form a fused benzo group: I'; with I' optionally substituted with one or more groups chosen from i) nitro; ii) nitroso; iii) $(O)_2S(O^-)$-, $M^+$; iv) hydroxyl; v) mercapto; vi) (di)(alkyl)amino; vii) R°-C(X)-X'-; viii) R°-X'-C(X)-; ix) R°-X'-C(X)-X"-; with $M^+$, R°, X, X' and X" as defined previously;

more particularly, $R_{37}$ to $R_{40}$ represent a hydrogen atom, and $R_{41}$ to $R_{44}$, which may be identical or different, represent a hydroxyl group or $(O)_2S(O^-)$-, $M^+$; and when $R_{43}$ with $R_{44}$ together form a benzo group, it is preferentially substituted with an $(O)_2S(O^-)$- group;
it being understood that at least one of the rings G, H, I or I' comprises at least one sulfonate radical $(O)_2S(O^-)$- or one carboxylate radical C(O)O⁻-; more preferentially sulfonate.

As examples of dyes of formula (IX), mention may be made especially of: Acid Blue 1; Acid Blue 3; Acid Blue 7, Acid Blue 9; Acid Violet 49; Acid Green 3; Acid Green 5 and Acid Green 50.

f) the xanthene-based dyes of formula (X):

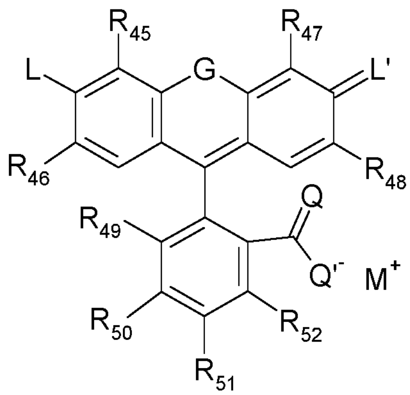

**(X)**

in which formula (X):

- $R_{45}$, $R_{46}$, $R_{47}$ and $R_{48}$, which may be identical or different, represent a hydrogen or halogen atom;
- $R_{49}$, $R_{50}$, $R_{51}$ and $R_{52}$, which may be identical or different, represent a hydrogen or halogen atom, or a group chosen from:

  - alkyl;
  - alkoxy, alkylthio;
  - hydroxyl, mercapto;
  - nitro, nitroso;
  - $(O)_2S(O^-)$-, $M^+$ with $M^+$ representing a hydrogen atom or a cationic counterion;
  - (O)CO⁻-, $M^+$ with $M^+$ as defined previously;

  preferably, $R_{49}$, $R_{50}$, $R_{51}$ and $R_{52}$ represent a hydrogen or halogen atom;
- G represents an oxygen or sulfur atom or a group $NR_e$ with $R_e$ as defined previously; more particularly G represents an oxygen atom;
- L represents an alkoxide $O^-$, $M^+$; a thioalkoxide $S^-$, $M^+$ or a group $NR_f$, with $R_f$ representing a hydrogen atom or an alkyl group and $M^+$ as defined previously; $M^+$ is particularly sodium or potassium;
- L' represents an oxygen or sulfur atom or an ammonium group: $N^+R_fR_g$, with $R_f$ and $R_g$, which may be identical or different, representing a hydrogen atom, an alkyl group or optionally substituted aryl; L' represents more particularly an oxygen atom or a phenylamino group optionally substituted with one or more alkyl or $(O)_mS(O^-$

)-, $M^+$ groups with m and $M^+$ as defined previously;

- Q and Q', which may be identical or different, represent an oxygen or sulfur atom; more particularly Q and Q' represent an oxygen atom;
- $M^+$ is as defined previously.

As examples of dyes of formula (X), mention may in particular be made of: Acid Yellow 73; Acid Red 51; Acid Red 52; Acid Red 87; Acid Red 92; Acid Red 95 and Acid Violet 9;

g) the indole-based dyes of formula (XI):

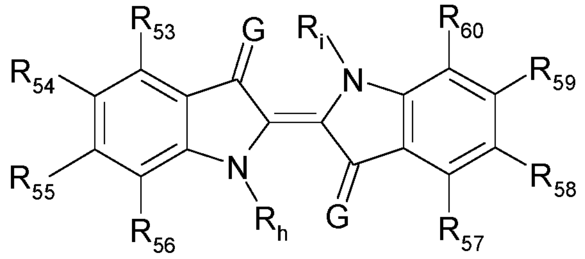

**(XI)**

in which formula (XI):

- $R_{53}$, $R_{54}$, $R_{55}$, $R_{56}$, $R_{57}$, $R_{58}$, $R_{59}$ and $R_{60}$, which may be identical or different, represent a hydrogen atom or a group chosen from:
  - alkyl;
  - alkoxy, alkylthio;
  - hydroxyl, mercapto;
  - nitro, nitroso;
  - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
  - $(O)_2S(O^-)$-, $M^+$ with $M^+$ representing a hydrogen atom or a cationic counterion;
  - $(O)CO^-$-, $M^+$ with $M^+$ as defined previously;
  - G represents an oxygen or sulfur atom or a group $NR_e$ with $R_e$ as defined previously; more particularly G represents an oxygen atom;
  - $R_j$ and $R_b$, which may be identical or different, represent a hydrogen atom or an alkyl group;

it being understood that formula (XI) comprises at least one sulfonate radical $(O)_2S(O^-)$-, $M^+$ or one carboxylate radical $C(O)O^-$-, $M^+$; more preferentially sodium sulfonate.

As an example of a dye of formula (XI), mention may be made especially of: Acid Blue 74.

h) the quinoline-based dyes of formula (XII):

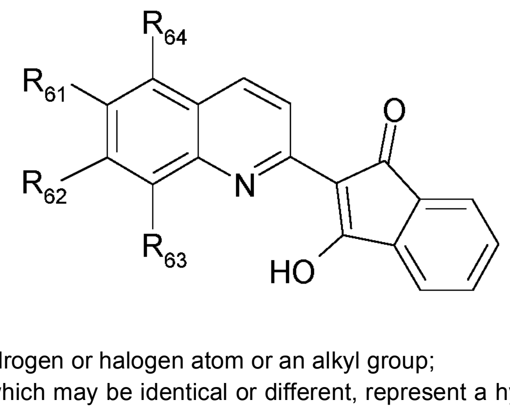

**(XII)**

- $R_{61}$ represents a hydrogen or halogen atom or an alkyl group;
- $R_{62}$, $R_{63}$, and $R_{64}$, which may be identical or different, represent a hydrogen atom or a group $(O)_2S(O^-)$-, $M^+$

with $M^+$ representing a hydrogen atom or a cationic counterion;
- or alternatively $R_{61}$ with $R_{62}$, or $R_{61}$ with $R_{64}$, together form a benzo group optionally substituted with one or more groups $(O)_2S(O^-)$-, $M^+$ with $M^+$ representing a hydrogen atom or a cationic counterion;

it being understood that formula (XII) comprises at least one sulfonate radical $(O)_2S(O^-)$-, more preferentially sodium sulfonate.

As examples of dyes of formula (XII), mention may be made especially of: Acid Yellow 2, Acid Yellow 3 and Acid Yellow 5.

The anionic direct dye(s) that may be used according to the invention are preferentially chosen from those of formulae (I), (II), (III), (IV), (V), (VI), (VII), (VIII), (IX), (X), (XI) and (XII) as defined above.

More particularly, the dyes of formulae (I) to (X) that may be used according to the invention are chosen from:

| | |
|---|---|
| (C.I. 45380) | Acid Red 87 (formula X) |
| (C.I. 10316) | Sodium salt of 2,4-dinitro-1-naphthol-7-sulfonic acid (formula VIII) |
| (C.I. 10383) | Acid Orange 3 (formula VII) |
| (C.I. 13015) | Acid Yellow 9 / Food Yellow 2 (formula I) |
| (C.I. 14780) | Direct Red 45 / Food Red 13 (formula I) |
| (C.I. 13711) | Acid Black 52 (formula I) |
| (C.I. 13065) | Acid Yellow 36 (formula I) |
| (C.I. 14700) | Sodium salt of 1-hydroxy-2-(2',4'-xylyl-5-sulfonatoazo)naphthalene-4-sulfonic acid / Food Red 1 (formula I) |
| (C.I. 14720) | Acid Red 14 / Food Red 3 / Mordant Blue 79 (formula I) |
| (C.I. 14805) | Sodium salt of 4-hydroxy-3-[(2-methoxy-5-nitrophenyl)diaza]-6-(phenylamino)naphthalene-2-sulfonic acid / Acid Brown 4 (formula I) |
| (C.I. 15510) | Acid Orange 7 / Pigment Orange 17 / Solvent Orange 49 / Orange 4 (formula I) |
| (C.I. 15985) | Food Yellow 3 / Pigment Yellow 104 (formula I) |
| (C.I. 16185) | Acid Red 27 / Food Red 9 (formula I) |
| (C.I. 16230) | Acid Orange 10 / Food Orange 4 (formula I) |
| (C.I. 16250) | Acid Red 44 (formula I) |
| (C.I. 17200) | Acid Red 33 / Food Red 12 (formula I) |
| (C.I. 15685) | Acid Red 184 (formula I) |
| (C.I. 19125) | Acid Violet 3 (formula I) |
| (C.I. 18055) | Sodium salt of 1-hydroxy-2-(4'-acetamidophenylazo)-8-acetamidonaphthalene-3,6-disulfonic acid / Acid Violet 7 / Food Red 11 (formula I) |
| (C.I. 18130) | Acid Red 135 (formula I) |
| (C.I. 19130) | Acid Yellow 27 (formula III) |
| (C.I. 19140) | Acid Yellow 23 / Food Yellow 4 / Yellow 5 (formula III) |
| (C.I. 20170) | 4'-(sulfonato-2",4"-dimethyl)bis(2,6-phenylazo)-1,3-dihydroxybenzene / Acid Orange 24 (formula I) |
| (C.I. 20470) | Sodium salt of 1-amino-2-(4'-nitrophenylazo)-7-phenylazo-8-hydroxynaphthalene-3,6-disulfonic acid / Acid Black 1 (formula I) |
| (C.I. 23266) | (4-((4-methylphenyl)sulfonyloxy)phenylazo)-2,2'-dimethyl-4-((2-hydroxy-5,8-disulfonato) naphthylazo)biphenyl / Acid Red 111 (formula II) |
| (C.I. 27755) | Food Black 2 (formula I) |
| (C.I. 25440) | 1-(4'-sulfonatophenylazo)-4-((2"-hydroxy-3"-acetylamino-6",8"-disulfonato)naphthylazo)-6-sulfonatonaphthalene (tetrasodium salt) / Food Black 1 (formula I) |

(continued)

| | |
|---|---|
| (C.I. 42090) | Acid Blue 9 (formula IX) |
| (C.I. 60730) | Acid Violet 43 / Ext Violet 2 (formula V) |
| (C.I. 61570) | Acid Green 25 (formula V) |
| (C.I. 62045) | Sodium salt of 1-amino-4-cyclohexylamino-9,10-anthraquinone-2-sulfonic acid / Acid Blue 62 (formula V) |
| (C.I. 62105) | Acid Blue 78 (formula V) |
| (C.I. 14710) | Sodium salt of 4-hydroxy-3-((2-methoxyphenyl)azo)-1-naphthalenesulfonic acid / Acid Red 4 (formula I) |
| | 2-Piperidino-5-nitrobenzenesulfonic acid (formula VIII) |
| | 2-(4'-N, N-(2"-Hydroxyethyl)amino-2'-nitro)anilineethanesulfonic acid (formula VIII) |
| | 4-β-Hydroxyethylamino-3-nitrobenzene sulfonic acid (formula VIII) |
| (C.I. 42640) | Acid Violet 49 (formula IX) |
| (C.I. 42080) | Acid Blue 7 (formula IX) |
| (C.I. 58005) | Sodium salt of 1,2-dihydroxy-3-sulfoanthraquinone / Mordant Red 3 (formula V) |
| (C.I. 62055) | Sodium salt of 1-amino-9,10-dihydro-9,10-dioxo-4-(phenylamino) 2-anthracenesulfonic acid / Acid Blue 25 (formula V) |
| (C.I. 14710) | Sodium salt of 4-hydroxy-3-((2-methoxyphenyl)azo)-1-naphthalenesulfonic acid / Acid Red 4 (formula I) |
| (C.I. 16255) | Acid Red 18 (formula II) |

Most of these dyes are described in particular in the Colour Index published by The Society of Dyers and Colourists, P.O. Box 244, Perkin House, 82 Grattan Road, Bradford, Yorkshire, BD12 JBN England.

The anionic direct dye(s) that are particularly preferred according to the invention are chosen from 1,2-dihydroxy-9,10-anthraquinone-3-sulfonic acid (C.I. 58005), the monosodium salt of 2-[(9,10-dihydro-4-hydroxy-9,10-dioxo-1-anthracenyl)amino]-5-methylbenzenesulfonic acid (C.I. 60730), the monosodium salt of 4-[(2-hydroxy-1-naphthyl)azo]benzenesulfonic acid (C.I. 15510), the disodium salt of 6-hydroxy-5-[(4-sulfophenyl)azo]-2-naphthalenesulfonic acid (C.I. 15985), the disodium salt of 5-amino-4-hydroxy-3-(phenylazo)-2,7-naphthalenedisulfonic acid (C.I. 17200), the disodium salt of 1-amino-2-(4'-nitrophenylazo)-7-phenylazo-8-hydroxy-3,6-naphthalenedisulfonic acid (C.I. 20470), the disodium salt of N-ethyl-N-[4-[[4-[ethyl[3-sulfophenyl)methyl]amino]phenyl](2-sulfophenyl)methylene]-2,5-cyclohexadien-1-ylidene]-3-sulfobenzenemethanaminium hydroxide (C.I. 42090), the disodium salt of 2,2'-[(9,10-dihydro-9,10-dioxo-1,4-anthracenediyl)diimino]bis[5-methyl]benzenesulfonic acid (C.I. 61570), the trisodium salt of 5-hydroxy-1-(4-sulfophenyl)-4-(4-sulfophenylazo)pyrazole-3-carboxylic acid (C.I. 19140), sodium 4-[(9,10-dihydro-4-hydroxy-9,10-dioxo-1-anthryl)amino]toluene-3-sulfonate (C.I. 60730), the trisodium salt of 7-hydroxy-8-[(4-sulfo-1-naphthalenyl)azo]-1,3-naphthalenedisulfonic acid (C.I. 16255), and a mixture of these compounds.

Use may also be made of compounds corresponding to the mesomeric or tautomeric forms of structures (I) to (XII).

In a preferred variant of the invention, the synthetic direct dye(s) are chosen from anionic direct dyes, preferably azo dyes, specifically those of formula (I), and anthraquinone dyes, specifically those of formula (V).

The direct dye(s) may preferably represent a total content of at least 0.05% by weight, better still at least 0.1% by weight, preferably at least 0.15% by weight, preferably ranging from 0.05% to 5%, better still from 0.1% to 3% by weight and even better still from 0.15% to 2% by weight relative to the total weight of composition B.

In particular, the anionic or natural direct dye(s) may preferably represent a total content of at least 0.05% by weight, better still at least 0.1% by weight, preferably at least 0.15% by weight, preferably ranging from 0.05% to 5%, better still from 0.1% to 3% by weight and even better still from 0.15% to 2% by weight relative to the total weight of composition B.

Composition B according to the invention has a pH of less than or equal to 5, preferably less than or equal to 4, even better still less than or equal to 3.

Preferably, the pH of composition A according to the invention ranges from 1 to 5, better still from 1.5 to 4 and even better still from 2 to 3.

*c) Buffer system*

The term "buffer system" refers to a mixture of an acid and of at least one conjugate base thereof, more specifically of at least one conjugate salt thereof.

The formulation of buffer systems is well known to those skilled in the art.

Preferably, the composition according to the invention comprises a buffer system comprising at least one mineral (inorganic) acid and at least one conjugate base thereof, i.e. the inorganic salt of said inorganic acid, preferably a conjugate base thereof.

More preferably, the composition according to the invention comprises an inorganic buffer system comprising at least one mineral (inorganic) acid and the conjugate base thereof, i.e. the inorganic salt of said inorganic acid.

Preferably, the inorganic acid is chosen from acids with a pKa (pKa1) of less than 4.5, preferably ranging from 1 to 4 and better still from 2 to 4.

The inorganic acid is chosen, for example, from phosphorus-based acids, such as phosphoric acid, halogen-based acids, such as hydrochloric acid, and sulfur-based acids, such as sulfuric acid, and mixtures thereof.

More preferentially, the inorganic acid is phosphoric acid.

The buffer capacity of the buffer system according to the invention is advantageously optimized when the acid, preferably the inorganic acid, and the conjugate salt(s) thereof are used in the composition according to the invention in an acid/conjugate salt(s) mole ratio ranging from 1:10 to 10:1, better still from 1:5 to 5:1, even better still from 1:3 to 3:1 and more preferably from 1:2 to 2:1.

The acid, preferably the inorganic acid, may represent from 0.05% to 5% by weight, preferably from 0.1% to 3% by weight and better still from 0.15% to 2% by weight relative to the total weight of the composition.

The conjugate acid salt(s), preferably the inorganic acid salt(s), may be present in an amount ranging from 0.05% to 5% by weight, preferably from 0.1% to 3% by weight and better still from 0.15% to 2% by weight, relative to the total weight of the composition.

Preferably, the composition according to the invention comprises a buffer system comprising at least phosphoric acid ($H_3PO_4$) and at least one inorganic phosphate salt, chosen in particular from potassium dihydrogen phosphate $KH_2PO_4$, sodium dihydrogen phosphate $NaH_2PO_4$, dipotassium hydrogen phosphate $K_2HPO_4$, disodium hydrogen phosphate $Na_2HPO_4$, potassium phosphate $K_3PO_4$ and sodium phosphate $Na_3PO_4$, and mixtures thereof, preferably from potassium dihydrogen phosphate $KH_2PO_4$ and sodium dihydrogen phosphate $NaH_2PO_4$.

The conjugate acid salt, in particular the conjugate inorganic acid salt, may be in solvate form, especially in hydrate form.

Preferably, phosphoric acid represents from 0.05% to 5% by weight, preferably from 0.1% to 3% by weight and better still from 0.15% to 2% by weight relative to the total weight of the composition.

Preferably, the inorganic phosphate salt, chosen in particular from potassium dihydrogen phosphate $KH_2PO_4$, sodium dihydrogen phosphate $NaH_2PO_4$, dipotassium hydrogen phosphate $K_2HPO_4$, disodium hydrogen phosphate $Na_2HPO_4$, potassium phosphate $K_3PO_4$ and sodium phosphate $Na_3PO_4$, and mixtures thereof, is present in a content ranging from 0.05% to 5% by weight, preferably from 0.1% to 3% by weight and better still from 0.15% to 2% by weight relative to the total weight of the composition.

According to an advantageous embodiment, phosphoric acid and the conjugate salt thereof (inorganic phosphate salt), in particular chosen from potassium dihydrogen phosphate $KH_2PO_4$, sodium dihydrogen phosphate $NaH_2PO_4$, dipotassium hydrogen phosphate $K_2HPO_4$, disodium hydrogen phosphate $Na_2HPO_4$, potassium phosphate $K_3PO_4$ and sodium phosphate $Na_3PO_4$, and mixtures thereof, are used in the compositions according to the invention in an acid/conjugate salt(s) mole ratio ranging from 1:10 to 10:1, better still from 1:5 to 5:1, even better still from 1:3 to 3:1 and more preferably from 1:2 to 2:1.

In a particularly preferred variant of the invention, the composition comprises phosphoric acid and an inorganic phosphate salt chosen from potassium dihydrogen phosphate $KH_2PO_4$ and sodium dihydrogen phosphate $NaH_2PO_4$, more preferentially sodium dihydrogen phosphate $NaH_2PO_4$.

*Liquid compound with a Hansen solubility parameter value $\delta H$ of less than 16 $MPa^{1/2}$*

According to an advantageous embodiment, composition B of the process according to the invention comprises at least one liquid compound with a Hansen solubility parameter value $\delta H$ of less than 16 $MPa^{1/2}$. Such a compound is also known as a hydrotropic compound.

For the purposes of the present invention, the term "hydrotropic compound" means a compound that is capable of increasing the solubility of hydrophobic compounds in aqueous phases.

Preferably, the liquid compound(s) have a Hansen solubility parameter value $\delta$H of greater than 0 and less than 16 $MPa^{1/2}$.

The liquid compound(s) more preferably have a Hansen solubility parameter $\delta$H of between 5 and 15.8 $MPa^{1/2}$,

even more preferentially between 8 and 15.8 $MPa^{1/2}$ and better still between 8 and 15 $MPa^{1/2}$.

These compounds are liquid at a temperature of 25°C and at atmospheric pressure (760 mmHg; i.e. $1.013 \times 10^5$ Pa).

The compound(s) with a Hansen solubility parameter value δH as defined previously are, for example, described in the reference publication Hansen solubility parameters: A User's Handbook by Charles M. Hansen, CRC Press, 2000, pages 167 to 185, or in the publication Handbook of Solubility Parameters and Other Cohesion Parameters, CRC Press, pages 95 to 121 and pages 177 to 185.

This solubility parameter value δH is related to the formation of hydrogen bonds. It may be recalled that there are three major types of interaction in organic compounds: nonpolar interactions, permanent dipole-dipole interactions and interactions of hydrogen bonding type, the latter forming the subject of the parameter defining the hydrotropic compound present in the composition used in accordance with the invention.

In particular, the publication Handbook of Solubility Parameters and Other Cohesion Parameters, CRC Press, pages 95 to 121 and pages 177 to 185, gives the equation $\delta H = (\Sigma\text{-}^{z}U_h/V)^{1/2}$

where $^{z}U_h$ (in $J.mol^{-1}$) describes the contributions of the functional group under consideration in the solubility parameters related to the hydrogen bonds (values in Table 14, page 183), this parameter $^{z}U_h$ also being described in the publication The relation between surface tension and solubility parameter in liquids, Bagda, E, Farbe Lack, 84, 212, 1978; and V is the volume of the molecule.

It should be noted that the solubility parameter value δH is usually given for a temperature of 25°C and at atmospheric pressure (760 mmHg, i.e. $1.013 \times 10^5$ Pa).

In particular, the liquid compounds with a Hansen solubility parameter value δH of less than 16 $MPa^{1/2}$ are nonionic organic compounds.

Said liquid compound(s) with a Hansen solubility parameter value δH of less than 16 $MPa^{1/2}$ may be chosen from:

- alcohol ethers, in particular $C_1$-$C_4$ ethers of $C_5$-$C_{30}$ alcohols, which are preferably saturated, linear or branched, optionally interrupted with one or more non-adjacent ether functions;
- aliphatic esters of $C_1$-$C_4$ carboxylic acids and of $C_3$-$C_{10}$ monoalcohols or polyhydroxylated alcohols, interrupted with one or more non-adjacent ether functions;
- aromatic ethers, in particular of $C_6$-$C_{10}$, of a $C_1$-$C_6$ alkyl optionally bearing a hydroxyl group,
- ($C_6$-$C_{10}$)aryl($C_1$-$C_6$)alkyl ethers, of a $C_1$-$C_6$ alkyl optionally bearing a hydroxyl group,
- alkanols bearing aryl substituents, preferably for which the aryl part is a $C_6$-$C_{10}$ aryl part, advantageously a $C_6$ aryl part, and the alkyl part of the alkanol is a $C_1$-$C_4$ alkyl part, it being possible for this alkyl part to be terminated or interrupted with a heteroatom, advantageously oxygen, or a hydroxyl group, preferably such as benzyl alcohol;
- lactones, preferably of formula (iii), and also mixtures thereof, with:

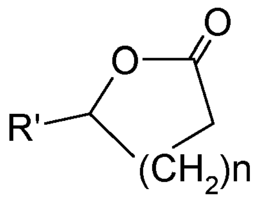
(iii)

in which R' represents a hydrogen, a linear or branched $C_1$-$C_8$ alkyl, a linear or branched $C_1$-$C_4$ hydroxyalkyl, n being equal to 1, 2 or 3, and preferably R' represents a hydrogen, a linear or branched $C_1$-$C_6$ alkyl or a linear or branched $C_1$-$C_2$ hydroxyalkyl.

A particularly advantageous example of lactones that may be mentioned is γ-butyrolactone.

Mention may also be made of certain liquid alkanols, for instance 1-pentanol.

Preferably, said liquid compound(s) with a Hansen solubility parameter value δH of less than 16 $MPa^{1/2}$ are chosen from alcohol ethers, aliphatic esters, aromatic ethers, alkanols bearing aryl substituents, and mixtures thereof.

Even more preferentially, said liquid compound(s) according to the invention are chosen from dipropylene glycol monomethyl ether acetate, dipropylene glycol methyl ether, dipropylene glycol mono-n-butyl ether (the INCI name of which is PPG-2 Butyl Ether), tripropylene glycol methyl ether, propylene glycol n-butyl ether, propylene glycol n-propyl ether, propylene glycol monomethyl ether, diethylene glycol monomethyl ether and monoethyl ether, 3-phenyl-1-propanol, 2-phenyl-1-propanol, benzyl alcohol, benzyloxyethanol, phenoxyethanol and phenylethanol, and mixtures of these compounds.

The liquid compound(s) with a Hansen solubility parameter value δH of less than 16 $MPa^{1/2}$ are even more preferentially chosen from alcohol ethers and alkanols bearing aryl substituents and even more preferentially dipropylene

glycol methyl ether, propylene glycol monobutyl ether and benzyl alcohol, phenylethanol or phenoxyethanol, better still alkanols bearing an aryl substituent, such as benzyl alcohol, phenylethanol or phenoxyethanol.

Preferentially, the liquid compound with a Hansen solubility parameter value δH of less than 16 $MPa^{1/2}$ is chosen from alkanols bearing aryl substituents.

More preferentially, the liquid compound with a Hansen solubility parameter value δH of less than 16 $MPa^{1/2}$ is benzyl alcohol.

Preferably, the liquid compound(s) with a Hansen solubility parameter value δH of less than 16 $MPa^{1/2}$ represent a total content ranging from 0.1% to 35% by weight, preferably from 0.2% to 20% by weight and better still from 0.3% to 5% by weight, relative to the total weight of composition B.

*a) Linear or branched C1-C8 alcohols*

The composition according to the invention comprises at least one alcohol, preferably a monoalcohol, other than the liquid compound with a Hansen solubility parameter value δH of less than 16 $MPa^{1/2}$, chosen from linear or branched alcohols containing from 1 to 8 carbon atoms, preferably 1 to 4 carbon atoms, preferably chosen from linear monoalcohols containing from 1 to 8 carbon atoms and better still from 1 to 4 carbon atoms.

Such alcohols may be chosen from ethanol, propanol, butanol, isopropanol and isobutanol, and mixtures thereof, preferably ethanol.

The linear or branched alcohol(s), preferably monoalcohol(s), containing from 1 to 8 carbon atoms may be present in an amount ranging from 0.5% to 20% by weight, preferably from 1% to 15% by weight and better still from 2% to 10% by weight, relative to the total weight of composition B.

The weight ratio of the total amount of liquid compound(s) with a Hansen solubility parameter value δH of less than 16 $MPa^{1/2}$ to the total amount of linear or branched alcohol(s) containing from 1 to 8 carbon atoms is less than or equal to 1, preferably less than or equal to 0.9 and more preferentially less than or equal to 0.8.

The weight ratio of the total amount of liquid compound(s) with a Hansen solubility parameter value δH of less than 16 $MPa^{1/2}$ to the total amount of linear or branched alcohol(s) containing from 1 to 8 carbon atoms may range from 0.1 to 0.9.

In a preferred variant of the invention, the weight ratio of the total amount of liquid alkanol(s) bearing aryl substituents with a Hansen solubility parameter value δH of less than 16 $MPa^{1/2}$ to the total amount of linear monoalcohol(s) containing from 1 to 4 carbon atoms ranges from 0.1 to 0.9.

*Organic acid*

According to one embodiment, the composition according to the invention also comprises an organic acid, other than the acid and the conjugate salt thereof, in particular other than the inorganic acid and the conjugate salt thereof, present in the buffer system.

The term "organic acid" means any non-polymeric organic compound including one or more acid functions chosen from carboxylic acid, sulfonic acid and phosphonic acid functions.

Preferably, the organic acid e) bears one or more carboxylic functions.

The organic acid(s) e) are preferably chosen from acetic acid, propanoic acid, butanoic acid, lactic acid, malic acid, glycolic acid, ascorbic acid, maleic acid, phthalic acid, succinic acid, serine, taurine, tartaric acid, arginine, glycine, glucuronic acid, gluconic acid and citric acid, and mixtures thereof.

Preferably, the organic acid has a $pK_a$ of less than 5, in particular a $pK_a$ of less than 4.5 and better still a $pK_a$ of less than 4, the $pK_a$ possibly ranging from 1 to 5 and better still from 2 to 4.

In particular, the acid(s) of the invention are chosen from α-hydroxy acids and α-amino acids; preferentially, the acid is chosen from citric acid, lactic acid, malic acid, tartaric acid, glycolic acid and serine, more preferentially from lactic acid and citric acid, or a mixture thereof.

The organic acid(s) e) may be present in an amount ranging from 0.5% to 20% by weight, preferentially from 1% to 15% by weight and even more preferably in an amount ranging from 1% to 10% by weight, relative to the total weight of the composition.

*Thickeners*

Composition B according to the invention may also comprise one or more thickeners.

According to the present invention, the term "thickener" means compounds which, by their presence at a concentration of 0.05% by weight, increase the viscosity of a composition into which they are introduced by at least 20 cps, preferably by at least 50 cps, at room temperature (25°C), at atmospheric pressure and at a shear rate of 1 $s^{-1}$ (the viscosity may be measured using a cone/plate viscometer, a Haake R600 rheometer or the like).

The thickener(s) are preferentially chosen from non-associative thickening polymers bearing sugar units, non-associative thickening polymers without sugar units, associative thickening polymers, and mixtures of these compounds.
For the purposes of the present invention, the term "sugar unit" means an oxygen-bearing hydrocarbon-based compound containing several alcohol functions, with or without aldehyde or ketone functions, and which includes at least 4 carbon atoms.
The sugar units may be optionally modified by substitution, and/or by oxidation and/or by dehydration.
The sugar units that may be included in the composition of the aqueous-phase thickening polymers of the invention are preferably derived from the following sugars: glucose, galactose, arabinose, rhamnose, mannose, xylose, fucose, anhydrogalactose, galacturonic acid, glucuronic acid, mannuronic acid, galactose sulfate, anhydrogalactose sulfate and fructose.
Non-associative thickening polymers bearing sugar units that may especially be mentioned include native gums such as:

a) tree or shrub exudates, including:

- gum arabic (branched polymer of galactose, arabinose, rhamnose and glucuronic acid);
- ghatti gum (polymer derived from arabinose, galactose, mannose, xylose and glucuronic acid);
- karaya gum (polymer derived from galacturonic acid, galactose, rhamnose and glucuronic acid);
- gum tragacanth (polymer of galacturonic acid, galactose, fucose, xylose and arabinose);

b) gums derived from algae, including:

- agar (polymer derived from galactose and anhydrogalactose);
- alginates (polymers of mannuronic acid and of glucuronic acid);
- carrageenans and furcellerans (polymers of galactose sulfate and of anhydrogalactose sulfate);

c) gums derived from seeds or tubers, including:

- guar gum (polymer of mannose and galactose);
- locust bean gum (polymer of mannose and galactose);
- fenugreek gum (polymer of mannose and galactose);
- tamarind gum (polymer of galactose, xylose and glucose);
- konjac gum (polymer of glucose and mannose);

d) microbial gums, including:

- xanthan gum (polymer of glucose, mannose acetate, mannose/pyruvic acid and glucuronic acid);
- gellan gum (polymer of partially acylated glucose, rhamnose and glucuronic acid);
- scleroglucan gum (glucose polymer);

e) plant extracts, including:

- cellulose (glucose polymer);
- starch (glucose polymer) and
- inulin.

These polymers may be physically or chemically modified. As physical treatment, mention may in particular be made of the temperature.
Chemical treatments that may be mentioned include esterification, etherification, amidation and oxidation reactions. These treatments make it possible to produce polymers that may especially be nonionic, anionic or amphoteric.
Preferably, these chemical or physical treatments are applied to guar gums, locust bean gums, starches and celluloses.
The nonionic guar gums that may be used according to the invention may be modified with $C_1$-$C_6$ (poly)hydroxyalkyl groups.
Among the $C_1$-$C_6$ (poly)hydroxyalkyl groups, mention may be made, by way of example, of hydroxymethyl, hydroxyethyl, hydroxypropyl and hydroxybutyl groups.
These guar gums are well known from the prior art and may be prepared, for example, by reacting corresponding alkene oxides, for instance propylene oxides, with the guar gum so as to obtain a guar gum modified with hydroxypropyl

groups.

The degree of hydroxyalkylation preferably varies from 0.4 to 1.2 and corresponds to the number of alkylene oxide molecules consumed by the number of free hydroxyl functions present on the guar gum.

Such nonionic guar gums optionally modified with hydroxyalkyl groups are sold, for example, under the trade names Jaguar HP8, Jaguar HP60 and Jaguar HP120 by the company Rhodia Chimie.

The botanical origin of the starch molecules that may be used in the present invention may be cereals or tubers. Thus, the starches are chosen, for example, from corn starch, rice starch, cassava starch, barley starch, potato starch, wheat starch, sorghum starch and pea starch.

The starches may be chemically or physically modified, in particular by one or more of the following reactions: pregelatinization, oxidation, crosslinking, esterification, etherification, amidation, heat treatments.

Distarch phosphates or compounds rich in distarch phosphate will preferentially be used, for instance the product sold under the references Prejel VA-70-T AGGL (gelatinized hydroxypropyl cassava distarch phosphate), Prejel TK1 (gelatinized cassava distarch phosphate) or Prejel 200 (gelatinized acetyl cassava distarch phosphate) by the company Avebe, or Structure Zea from National Starch (gelatinized corn distarch phosphate).

According to the invention, amphoteric starches may also be used, these amphoteric starches comprising one or more anionic groups and one or more cationic groups. The anionic and cationic groups may be bonded to the same reactive site of the starch molecule or to different reactive sites; they are preferably bonded to the same reactive site. The anionic groups may be of carboxylic, phosphate or sulfate type, preferably carboxylic. The cationic groups may be of primary, secondary, tertiary or quaternary amine type.

The starch molecules may be derived from any plant source of starch, in particular such as corn, potato, oat, rice, tapioca, sorghum, barley or wheat. It is also possible to use the hydrolysates of the starches mentioned above. The starch is preferably derived from potato.

The non-associative thickening polymers of the invention may be cellulose-based polymers not including a $C_{10}$-$C_{30}$ fatty chain in their structure.

According to the invention, the term "cellulose-based polymer" means any polysaccharide compound having in its structure sequences of glucose residues linked together via $\beta$-1,4 bonds; in addition to unsubstituted celluloses, the cellulose derivatives may be anionic, cationic, amphoteric or nonionic.

Thus, the cellulose-based polymers that may be used according to the invention may be chosen from unsubstituted celluloses, including those in a microcrystalline form, and cellulose ethers.

Among these cellulose-based polymers, cellulose ethers, cellulose esters and cellulose ester ethers are distinguished.

Among the cellulose esters are mineral esters of cellulose (cellulose nitrates, sulfates, phosphates, etc.), organic cellulose esters (cellulose monoacetates, triacetates, amidopropionates, acetatebutyrates, acetatepropionates and acetatetrimellitates, etc.), and mixed organic/mineral esters of cellulose, such as cellulose acetatebutyrate sulfates and cellulose acetatepropionate sulfates. Among the cellulose ester ethers, mention may be made of hydroxypropylmethylcellulose phthalates and ethylcellulose sulfates.

Among the nonionic cellulose ethers without a $C_{10}$-$C_{30}$ fatty chain, i.e. which are "non-associative", mention may be made of ($C_1$-$C_4$)alkylcelluloses, such as methylcelluloses and ethylcelluloses (for example, Ethocel standard 100 Premium from Dow Chemical); (poly)hydroxy($C_1$-$C_4$)alkylcelluloses, such as hydroxymethylcelluloses, hydroxyethylcelluloses (for example, Natrosol 250 HHR provided by Aqualon) and hydroxypropylcelluloses (for example, Klucel EF from Aqualon); mixed (poly)hydroxy($C_1$-$C_4$)alkyl-($C_1$-$C_4$)alkylcellulose celluloses, such as hydroxypropylmethylcelluloses (for example, Methocel E4M from Dow Chemical), hydroxyethylmethylcelluloses, hydroxyethylethylcelluloses (for example, Bermocoll E 481 FQ from AkzoNobel) and hydroxybutylmethylcelluloses.

Among the anionic cellulose ethers without a fatty chain, mention may be made of (poly)carboxy($C_1$-$C_4$)alkylcelluloses and salts thereof. By way of example, mention may be made of carboxymethylcelluloses, carboxymethylmethylcelluloses (for example Blanose 7M from the company Aqualon) and carboxymethylhydroxyethylcelluloses, and the sodium salts thereof.

Among the cationic cellulose ethers without a fatty chain, mention may be made of cationic cellulose derivatives such as cellulose copolymers or cellulose derivatives grafted with a water-soluble quaternary ammonium monomer, and described in particular in patent US 4 131 576, such as (poly)hydroxy($C_1$-$C_4$)alkylcelluloses, for instance hydroxymethyl-, hydroxyethyl- or hydroxypropylcelluloses grafted in particular with a methacryloylethyltrimethylammonium, methacrylamidopropyltrimethylammonium or dimethyldiallylammonium salt. The commercial products corresponding to this definition are more particularly the products sold under the names Celquat® L 200 and Celquat® H 100 by the company National Starch.

Among the polymers bearing non-associative sugar units, it is preferred to use non-associative cellulose-based polymers, in particular nonionic cellulose ethers not bearing a $C_{10}$-$C_{30}$ fatty chain.

Among the non-associative thickening polymers not bearing sugar units that may be used according to the invention, mention may be made of crosslinked acrylic acid or methacrylic acid homopolymers or copolymers, crosslinked

2-acrylamido-2-methylpropanesulfonic acid homopolymers and crosslinked acrylamide copolymers thereof, ammonium acrylate homopolymers, or copolymers of ammonium acrylate and of acrylamide, alone or as mixtures.

A first family of non-associative thickening polymers that is suitable for use is represented by crosslinked acrylic acid homopolymers.

Among the homopolymers of this type, mention may be made of those crosslinked with an allyl alcohol ether of the sugar series, for instance the products sold under the names Carbopol 980, 981, 954, 2984 and 5984 by the company Noveon or the products sold under the names Synthalen M and Synthalen K by the company 3 VSA. These polymers have the INCI name Carbomer.

The non-associative thickening polymers may also be crosslinked (meth)acrylic acid copolymers, such as the polymer sold under the name Aqua SF1 by the company Noveon.

The non-associative thickening polymers may be chosen from 2-acrylamido-2-methylpropanesulfonic acid homopolymers and the crosslinked or non-crosslinked copolymers thereof.

Among the 2-acrylamido-2-methylpropanesulfonic acid copolymers, mention may be made of partially or totally neutralized crosslinked copolymers of 2-acrylamido-2-methylpropanesulfonic acid and of acrylamide; mention may be made in particular of the product described in Example 1 of document EP 503 853, and reference may be made to said document as regards these polymers.

Mention may also be made of copolymers of 2-acrylamido-2-methylpropanesulfonic acid and of hydroxyethyl acrylate, such as the compound sold under the name Sepinov EMT 10 by the company SEPPIC.

The aqueous composition may similarly comprise, as non-associative thickening polymers, ammonium acrylate homopolymers or copolymers of ammonium acrylate and of acrylamide.

Among the examples of ammonium acrylate homopolymers that may be mentioned is the product sold under the name Microsap PAS 5193 by the company Hoechst. Among the copolymers of ammonium acrylate and of acrylamide that may be mentioned is the product sold under the name Bozepol C Nouveau or the product PAS 5193 sold by the company Hoechst. Reference may be made especially to FR 2 416 723, US 2 798 053 and US 2 923 692 as regards the description and preparation of such compounds.

Use may also be made of cationic thickening polymers of acrylic type.

Among the thickening polymers, mention may also be made of the associative polymers that are well known to a person skilled in the art and especially of nonionic, anionic, cationic or amphoteric nature.

It is recalled that "associative polymers" are polymers that are capable, in an aqueous medium, of reversibly associating with each other or with other molecules.

Their chemical structure more particularly comprises at least one hydrophilic region and at least one hydrophobic region.

The term "hydrophobic group" means a radical or polymer with a saturated or unsaturated, linear or branched hydrocarbon-based chain, comprising at least 10 carbon atoms, preferably from 10 to 30 carbon atoms, in particular from 12 to 30 carbon atoms and more preferentially from 18 to 30 carbon atoms.

Preferentially, the hydrocarbon-based group is derived from a monofunctional compound. By way of example, the hydrophobic group may be derived from a fatty alcohol such as stearyl alcohol, dodecyl alcohol or decyl alcohol. It may also denote a hydrocarbon-based polymer, for instance polybutadiene.

Among the associative polymers of anionic type that may be mentioned are:

- (a) those including at least one hydrophilic unit and at least one fatty-chain allyl ether unit, more particularly those of which the hydrophilic unit is constituted by an ethylenic unsaturated anionic monomer, even more particularly by a vinylcarboxylic acid and most particularly by an acrylic acid or a methacrylic acid or mixtures thereof.

Among these anionic associative polymers, the ones that are particularly preferred according to the invention are polymers formed from 20% to 60% by weight of acrylic acid and/or of methacrylic acid, from 5% to 60% by weight of lower alkyl (meth)acrylates, from 2% to 50% by weight of fatty-chain allyl ether, and from 0 to 1% by weight of a crosslinking agent which is a well-known copolymerizable unsaturated polyethylenic monomer, for instance diallyl phthalate, allyl (meth)acrylate, divinylbenzene, (poly)ethylene glycol dimethacrylate or methylenebisacrylamide.

Among the latter polymers, the ones most particularly preferred are crosslinked terpolymers of methacrylic acid, of ethyl acrylate and of polyethylene glycol (10 OE) stearyl alcohol ether (Steareth 10), especially those sold by the company CIBA under the names Salcare SC80® and Salcare SC900, which are aqueous 30% emulsions of a crosslinked terpolymer of methacrylic acid, of ethyl acrylate and of steareth-10 allyl ether (40/50/10).

- (b) those comprising i) at least one hydrophilic unit of unsaturated olefinic carboxylic acid type, and ii) at least one hydrophobic unit of the ($C_{10}$-$C_{30}$) alkyl ester of an unsaturated carboxylic acid type.

($C_{10}$-$C_{30}$) Alkyl esters of unsaturated carboxylic acids that are useful in the invention comprise, for example,

lauryl acrylate, stearyl acrylate, decyl acrylate, isodecyl acrylate and dodecyl acrylate, and the corresponding methacrylates, lauryl methacrylate, stearyl methacrylate, decyl methacrylate, isodecyl methacrylate and dodecyl methacrylate.

Anionic polymers of this type are described and prepared, for example, according to patents US 3 915 921 and US 4 509 949.

Among the anionic associative polymers of this type that will be used more particularly are those constituted of from 95% to 60% by weight of acrylic acid (hydrophilic unit), 4% to 40% by weight of $C_{10}$-$C_{30}$ alkyl acrylate (hydrophobic unit) and 0 to 6% by weight of crosslinking polymerizable monomer, or alternatively those constituted of from 98% to 96% by weight of acrylic acid (hydrophilic unit), 1% to 4% by weight of $C_{10}$-$C_{30}$ alkyl acrylate (hydrophobic unit) and 0.1% to 0.6% by weight of crosslinking polymerizable monomer such as those described above.

Among said polymers above, the ones most particularly preferred according to the present invention are the products sold by the company Goodrich under the trade names Pemulen TR1®, Pemulen TR2®, Carbopol 1382®, and even more preferentially Pemulen TR1®, and the product sold by the company SEPPIC under the name Coatex SX®.

Mention may also be made of the acrylic acid/lauryl methacrylate/vinylpyrrolidone terpolymer sold under the name Acrylidone LM by the company ISP.

- (c) maleic anhydride/$C_{30}$-$C_{38}$ $\alpha$-olefin/alkyl maleate terpolymers, such as the product (maleic anhydride/$C_{30}$-$C_{38}$ $\alpha$-olefin/isopropyl maleate copolymer) sold under the name Performa V 1608® by the company Newphase Technologies.
- (d) acrylic terpolymers comprising:

  i) about 20% to 70% by weight of an a,$\beta$-monoethylenically unsaturated carboxylic acid [A],
  ii) about 20% to 80% by weight of an a,$\beta$-monoethylenically unsaturated non-surfactant monomer other than [A],
  iii) about 0.5% to 60% by weight of a nonionic monourethane which is the product of reaction of a monohydric surfactant with a monoethylenically unsaturated monoisocyanate,

  such as those described in patent application EP-A-0 173 109 and more particularly the terpolymer described in Example 3, namely a methacrylic acid/methyl acrylate/behenyl alcohol dimethyl-meta-isopropenylbenzylisocyanate ethoxylated (40 OE) terpolymer, as an aqueous 25% dispersion.
- (e) copolymers comprising among their monomers an $\alpha$,$\beta$-monoethylenically unsaturated carboxylic acid and an ester of an a,$\beta$-monoethylenically unsaturated carboxylic acid and of an oxyalkylenated fatty alcohol.

Preferentially, these compounds also comprise as monomer an ester of an $\alpha$,$\beta$-monoethylenically unsaturated carboxylic acid and of a $C_1$-$C_4$ alcohol.

An example of a compound of this type that may be mentioned is Aculyn 22® sold by the company Röhm & Haas, which is a methacrylic acid/ethyl acrylate/oxyalkylenated stearyl methacrylate terpolymer; and also Aculyn 88, also sold by the company Röhm & Haas.

- (f) amphiphilic polymers comprising at least one ethylenically unsaturated monomer bearing a sulfonic group, in free or partially or totally neutralized form and comprising at least one hydrophobic part. These polymers may be crosslinked or non-crosslinked. They are preferably crosslinked.

The ethylenically unsaturated monomers bearing a sulfonic group are especially chosen from vinylsulfonic acid, styrenesulfonic acid, (meth)acrylamido($C_1$-$C_{22}$)alkylsulfonic acids, N-($C_1$-$C_{22}$)alkyl(meth)acrylamido($C_1$-$C_{22}$)alkylsulfonic acids such as undecylacrylamidomethanesulfonic acid, and also partially or totally neutralized forms thereof.

(Meth)acrylamido($C_1$-$C_{22}$)alkylsulfonic acids, for instance acrylamidomethanesulfonic acid, acrylamidoethanesulfonic acid, acrylamidopropanesulfonic acid, 2-acrylamido-2-methylpropanesulfonic acid, methacrylamido-2-methylpropanesulfonic acid, 2-acrylamido-n-butanesulfonic acid, 2-acrylamido-2,4,4-trimethylpentanesulfonic acid, 2-methacrylamidododecylsulfonic acid or 2-acrylamido-2,6-dimethyl-3-heptanesulfonic acid, and also partially or totally neutralized forms thereof, will more preferentially be used.

2-Acrylamido-2-methylpropanesulfonic acid (AMPS), and also partially or totally neutralized forms thereof, will more particularly be used.

The polymers of this family may be chosen especially from random amphiphilic AMPS polymers modified by reaction with a $C_6$-$C_{22}$ n-monoalkylamine or di-n-alkylamine, and such as those described in patent application WO 00/31154 (forming an integral part of the content of the description). These polymers may also contain other ethylenically unsaturated hydrophilic monomers chosen, for example, from (meth)acrylic acids, $\beta$-substituted alkyl derivatives thereof or esters thereof obtained with monoalcohols or mono- or polyalkylene glycols, (meth)acrylamides, vinylpyrrolidone, maleic anhydride, itaconic acid or maleic acid, or mixtures of these compounds.

The preferred polymers of this family are chosen from amphiphilic copolymers of AMPS and of at least one

ethylenically unsaturated hydrophobic monomer.

These same copolymers may also contain one or more ethylenically unsaturated monomers not comprising a fatty chain, such as (meth)acrylic acids, β-substituted alkyl derivatives thereof or esters thereof obtained with monoalcohols or mono- or polyalkylene glycols, (meth)acrylamides, vinylpyrrolidone, maleic anhydride, itaconic acid or maleic acid, or mixtures of these compounds.

These copolymers are described especially in patent application EP-A 750 899, patent US 5 089 578 and in the following publications from Yotaro Morishima:

- Self-assembling amphiphilic polyelectrolytes and their nanostructures, Chinese Journal of Polymer Science, Vol. 18, No. 40, (2000), 323-336;
- Micelle formation of random copolymers of sodium 2-(acrylamido)-2-methylpropanesulfonate and a nonionic surfactant macromonomer in water as studied by fluorescence and dynamic light scattering, Macromolecules, Vol. 33, No. 10, (2000), 3694-3704;
- Solution properties of micelle networks formed by nonionic moieties covalently bound to a polyelectrolyte: salt effects on rheological behavior - Langmuir, Vol. 16, No. 12, (2000), 5324-5332;
- Stimuli responsive amphiphilic copolymers of sodium 2-(acrylamido)-2-methylpropanesulfonate and associative macromonomers, Polym. Preprint, Div. Polym. Chem., 40(2), (1999), 220-221.

Among these polymers, mention may be made of:

- crosslinked or non-crosslinked, neutralized or non-neutralized copolymers, including from 15% to 60% by weight of AMPS units and from 40% to 85% by weight of ($C_8$-$C_{16}$)alkyl(meth)acrylamide or ($C_8$-$C_{16}$)alkyl(meth)acrylate units relative to the polymer, such as those described in patent application EP-A 750 899;
- terpolymers comprising from 10 mol% to 90 mol% of acrylamide units, from 0.1 mol% to 10 mol% of AMPS units and from 5 mol% to 80 mol% of n-($C_6$-$C_{18}$)alkylacrylamide units, such as those described in patent US-5 089 578.

Mention may also be made of copolymers of totally neutralized AMPS and of dodecyl methacrylate, and also crosslinked and non-crosslinked copolymers of AMPS and of n-dodecylmethacrylamide, such as those described in the Morishima articles mentioned above.

Among the cationic associative polymers, mention may be made of:

(a) cationic associative polyurethanes;
(b) the compound sold by the company Noveon under the name Aqua CC and which corresponds to the INCI name Polyacrylate-1 Crosspolymer.

Polyacrylate-1 Crosspolymer is the product of polymerization of a monomer mixture comprising:

- a di($C_1$-$C_4$ alkyl)amino($C_1$-$C_6$ alkyl) methacrylate,
- one or more $C_1$-$C_{30}$ alkyl esters of (meth)acrylic acid,
- a polyethoxylated $C_{10}$-$C_{30}$ alkyl methacrylate (20-25 mol of ethylene oxide units),
- a 30/5 polyethylene glycol/polypropylene glycol allyl ether,
- a hydroxy($C_2$-$C_6$ alkyl) methacrylate, and
- an ethylene glycol dimethacrylate.

(c) quaternized (poly)hydroxyethylcelluloses modified with groups comprising at least one fatty chain, such as alkyl, arylalkyl or alkylaryl groups comprising at least 8 carbon atoms, or mixtures thereof. The alkyl radicals borne by the above quaternized celluloses or hydroxyethylcelluloses preferably comprise from 8 to 30 carbon atoms. The aryl radicals preferably denote phenyl, benzyl, naphthyl or anthryl groups. Examples of quaternized alkylhydroxyethylcelluloses containing $C_8$-$C_{30}$ fatty chains that may be indicated include the products Quatrisoft LM 2000, Quatrisoft LM-X 529-18-A®, Quatrisoft LM-X 529-18-B® ($C_{12}$ alkyl) and Quatrisoft LM-X 529-8® ($C_{18}$ alkyl) sold by the company Aqualon, and the products Crodacel QM®, Crodacel QL® ($C_{12}$ alkyl) and Crodacel QS® ($C_{18}$ alkyl) sold by the company Croda and the product Softcat SL 1000 sold by the company Aqualon.
(d) cationic polyvinyllactam polymers.

Such polymers are described, for example, in patent application WO-00/68282.

As cationic poly(vinyllactam) polymers according to the invention, vinylpyrrolidone/dimethylaminopropylmethacrylamide/dodecyldimethylmethacryl-amidopropylammonium tosylate terpolymers, vinylpyrrolidone/dimethylaminopropylmethacrylamide/cocoyldimethylmethacrylamidoprop ylammonium tosylate terpolymers, vinylpyrrolidone/dimethylaminopropylmethacrylamide/lauryldimethylmethacrylamid-opropylammonium tosylate or chloride terpolymers are used in particular.

The amphoteric associative polymers are preferably chosen from those including at least one noncyclic cationic unit. Even more particularly, those prepared from or comprising 1 to 20 mol%, preferably 1.5 to 15 mol% and even more particularly 1.5 to 6 mol% of fatty-chain monomer relative to the total number of moles of monomers are preferred.

Amphoteric associative polymers according to the invention are described and prepared, for example, in patent application WO 98/44012.

Among the amphoteric associative polymers according to the invention, the ones that are preferred are acrylic acid/(meth)acrylamidopropyltrimethylammonium chloride/stearyl methacrylate terpolymers.

The associative polymers of nonionic type that may be used according to the invention are preferably chosen from:

(a) copolymers of vinylpyrrolidone and of fatty-chain hydrophobic monomers, of which examples that may be mentioned include:

- the products Antaron V216® or Ganex V216® (vinylpyrrolidone/hexadecene copolymer), sold by the company ISP,
- the products Antaron V220® or Ganex V220® (vinylpyrrolidone/eicosene copolymer), sold by the company ISP,

(b) copolymers of $C_1$-$C_6$ alkyl methacrylates or acrylates and of amphiphilic monomers comprising at least one fatty chain, for instance the oxyethylenated methyl acrylate/stearyl acrylate copolymer sold by the company Goldschmidt under the name Antil 208®;

(c) copolymers of hydrophilic methacrylates or acrylates and of hydrophobic monomers comprising at least one fatty chain, for instance the polyethylene glycol methacrylate/lauryl methacrylate copolymer;

(d) polyurethane polyethers comprising in their chain both hydrophilic blocks usually of polyoxyethylenated nature and hydrophobic blocks, which may be aliphatic sequences alone and/or cycloaliphatic and/or aromatic sequences;

(e) polymers with an aminoplast ether backbone containing at least one fatty chain, such as the Pure Thix® compounds sold by the company Sud-Chemie;

(f) celluloses or derivatives thereof, modified with groups comprising at least one fatty chain, such as alkyl, arylalkyl or alkylaryl groups or mixtures thereof in which the alkyl groups are of Cs, and in particular:

* nonionic alkylhydroxyethylcelluloses such as the products Natrosol Plus Grade 330 CS and Polysurf 67 ($C_{16}$ alkyl) sold by the company Aqualon;
* nonionic nonoxynylhydroxyethylcelluloses such as the product Amercell HM-1500 sold by the company Amerchol;
* nonionic alkylcelluloses such as the product Bermocoll EHM 100 sold by the company Berol Nobel;

(g) associative guar derivatives, for instance hydroxypropyl guars modified with a fatty chain, such as the product Esaflor HM 22 (modified with a $C_{22}$ alkyl chain) sold by the company Lamberti; the product Miracare XC 95-3 (modified with a $C_{14}$ alkyl chain) and the product RE 205-146 (modified with a $C_{20}$ alkyl chain) sold by Rhodia Chimie.

Preferably, the polyurethane polyethers include at least two hydrocarbon-based lipophilic chains containing from 6 to 30 carbon atoms, separated by a hydrophilic block, the hydrocarbon-based chains possibly being side chains or chains at the end of the hydrophilic block. In particular, it is possible for one or more side chains to be envisaged. In addition, the polymer may comprise a hydrocarbon-based chain at one end or at both ends of a hydrophilic block.

The polyurethane polyethers may be multiblock, in particular in triblock form. The hydrophobic blocks may be at each end of the chain (for example: triblock copolymer bearing a hydrophilic central block) or distributed both at the ends and in the chain (for example, multiblock copolymer). These same polymers may also be graft polymers or star polymers.

The nonionic fatty-chain polyurethane polyethers may be triblock copolymers, the hydrophilic block of which is a polyoxyethylene chain including from 50 to 1000 oxyethylene groups. The nonionic polyurethane polyethers include a urethane bond between the hydrophilic blocks, hence the origin of the name.

By extension, also included among the nonionic fatty-chain polyurethane polyethers are those in which the hydrophilic blocks are linked to the lipophilic blocks via other chemical bonds.

As examples of nonionic fatty-chain polyurethane polyethers that may be used in the invention, use may also be made of Rheolate 205® bearing a urea function, sold by the company Rheox, or Rheolate® 208, 204 or 212, and also Acrysol RM 184®.

Mention may also be made of the product Elfacos T210® bearing a $C_{12}$-$C_{14}$ alkyl chain, and the product Elfacos

T212® bearing a $C_{18}$ alkyl chain, from Akzo.

The product DW 1206B® from Röhm & Haas bearing a $C_{20}$ alkyl chain and a urethane bond, sold at a solids content of 20% in water, may also be used.

Use may also be made of solutions or dispersions of these polymers, especially in water or in aqueous-alcoholic medium. Examples of such polymers that may be mentioned include Rheolate® 255, Rheolate® 278 and Rheolate® 244 sold by the company Rheox. Use may also be made of the products DW 1206F and DW 1206J sold by the company Röhm & Haas.

The polyurethane polyethers that may be used according to the invention are in particular those described in the article by G. Fonnum, J. Bakke and Fk. Hansen - Colloid Polym. Sci., 271, 380-389 (1993).

It is even more particularly preferred to use a polyurethane polyether that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

Such polyurethane polyethers are sold especially by the company Röhm & Haas under the names Aculyn 46® and Aculyn 44® [Aculyn 46® is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%); Aculyn 44® is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%)].

More preferentially, the thickener(s) are chosen from non-associative cellulose-based polymers, in particular nonionic cellulose ethers not bearing a $C_{10}$-$C_{30}$ fatty chain, associative or non-associative thickening polymers bearing acrylic or methacrylic units, polymers bearing 2-acrylamido-2-methylpropanesulfonic acid units and/or the salified form thereof.

In a preferred variant of the invention, the thickener(s) are chosen from acrylic acid homopolymers or copolymers, in particular acrylic acid homopolymers, homopolymers or copolymers of 2-acrylamido-2-methylpropanesulfonic acid and/or the salified form thereof, in particular copolymers of 2-acrylamido-2-methylpropanesulfonic acid and/or the salified form thereof, more particularly copolymers of 2-acrylamido-2-methylpropanesulfonic acid and/or the salified form thereof and of acrylamide or copolymers of 2-acrylamido-2-methylpropanesulfonic acid and/or the salified form thereof and of hydroxyethyl acrylate, said polymers possibly being crosslinked or non-crosslinked.

When it is (they are) present, the thickener(s) generally represent a total content ranging from 0.1% to 20% by weight, preferably from 0.5% to 15% by weight and better still from 1.5% to 10% by weight, relative to the total weight of the composition.

Composition B according to the invention also comprises water.

Water generally represents from 40% to 99% by weight, preferably from 50% to 95% by weight and more preferentially from 60% to 90% by weight relative to the total weight of the composition.

*The adjuvants:*

The compositions used in the dyeing process in accordance with the invention may also contain various adjuvants conventionally used in hair dye compositions, such as anionic, cationic, nonionic, amphoteric or zwitterionic surfactants or mixtures thereof, anionic, cationic, nonionic, amphoteric or zwitterionic polymers or mixtures thereof, mineral or organic thickeners, other than the fatty-phase thickeners and aqueous-phase thickeners present in compositions A and B, and in particular anionic, cationic, nonionic and amphoteric polymeric associative thickeners, antioxidants, penetrants, sequestrants, fragrances, buffers, dispersants, conditioning agents, for instance volatile or non-volatile, modified or unmodified silicones, film-forming agents, ceramides, preserving agents and opacifiers.

The above additives may in general be present in an amount, for each of them, of between 0.01 and 20% by weight relative to the total weight of each composition containing them.

Needless to say, a person skilled in the art will take care to select this or these optional additional compound(s) such that the advantageous properties intrinsically associated with the composition(s) that are useful in the dyeing process in accordance with the invention are not, or are not substantially, adversely affected by the envisaged addition(s).

The cosmetic composition(s) used according to the process of the invention may be in various presentation forms, such as a powder, a lotion, a mousse, a cream or a gel, or in any other form that is suitable for dyeing keratin fibres. They may also be packaged in a propellant-free pump-action bottle or under pressure in an aerosol container in the presence of a propellant and form a foam.

***Multi-step dyeing process***

The process for dyeing keratin fibres of the invention involves several separate steps, comprising:

i) at least one step i) of dyeing said fibres using a cosmetic dye composition A, preferably an aqueous composition, comprising at least a) indigo and/or henna,

ii) at least one step ii) of treating said fibres, comprising the application to the fibres of an aqueous composition B which comprises water, b) at least one direct dye, other than henna and indigo, c) a buffer system comprising a mixture of an acid and of at least one conjugate salt thereof,

iii) optionally at least one step of intermediate rinsing of said fibres, preferably with water, between step i) and step ii) (or between step ii) and i) (depending on the order of steps i) and ii)).

According to a preferred embodiment of the invention, the step of treating with composition A is performed prior to the first step of dyeing with composition B; it is then a step of pretreatment with composition A.

Preferably, the process according to the invention comprises an intermediate rinsing step iii) between step i) and step ii).

Preferably, the process involves, in the following order:

- step i) of treating said fibres with a cosmetic composition A and then
- step ii) of dyeing with composition B.

Preferably, in this embodiment, the process according to the invention comprises an intermediate rinsing step iii) between step i) and step ii).

Preferably, the process according to the invention comprises a final rinsing step after performing all the steps of the process.

Preferably, the intermediate step(s) of rinsing the keratin fibres are preferably performed with a composition comprising water.

In particular, according to a preferred embodiment of the invention, said intermediate rinsing step(s) are performed exclusively with water, without adding an additional compound. According to another embodiment, the composition used for performing said rinsing step(s) may also comprise one or more additional compounds.

In particular, in the dyeing process of the invention, the leave-on time of composition A as defined previously on the keratin fibres is between 1 minute and 2 hours, more particularly between 5 minutes and 1 hour, preferably between 10 and 45 minutes. Preferentially, the leave-on time of composition A on the keratin fibres is performed at a temperature of between 20°C and 50°C, more preferentially between room temperature (25°C) and 40°C.

Composition B used in step ii) is preferably left to stand on the fibres for 1 to 30 minutes and preferably for 2 to 20 minutes, at a temperature of between 20°C and 50°C.

On conclusion of the dyeing process, the keratin fibres are generally rinsed with water, optionally undergo washing with a shampoo, followed by rinsing with water, before being left to dry or dried via a heat treatment by heating to a temperature of between 30 and 60°C. In practice, this operation may be performed using a styling hood, a hairdryer, an infrared ray dispenser and other standard heating appliances.

Use may also be made, as a means for both heating and for straightening the head of hair, of a heating iron at a temperature of between 60°C and 220°C and preferably between 120°C and 200°C.

According to a particular embodiment of the invention, the process according to the invention does not use any hydrogen peroxide.

According to a preferred embodiment, the process according to the invention does not use a chemical oxidizing agent.

According to a preferred embodiment, the process according to the invention does not use any oxidation base and/or any coupler.

A particular embodiment of the invention relates to a dyeing process which is performed at room temperature (25°C).

In all the particular forms and variants of the processes previously described, the compositions mentioned are ready-to-use compositions that may result from the extemporaneous mixing of two or more compositions and in particular of compositions present in dyeing kits.

The evaluation of the colouring obtained on the keratin fibres may be performed visually or with a spectrocolorimeter in the CIE L* a* b* system, for example using a Minolta CM 3600 spectrocolorimeter (illuminant D65, angle 10°, specular component included).

In this L*a*b* system, L* represents the lightness of the colour, a* indicates the green/red colour axis and b* indicates the blue/yellow colour axis. The smaller the value of L*, the darker and more powerful the colouring.

The smaller the value of a*, the greener the colour and the higher the value of a*, the redder the colour.

The smaller the value of b*, the bluer the colour and the higher the value of b*, the yellower the colour.

The colour buildup corresponds to the variation in colouring between the locks of hair before and after the treatment or dyeing and is defined by ($\Delta E^*$) according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured on locks of hair after dyeing and $L_0$*, $a_0$* and $b_0$* represent the values measured on locks of undyed hair. The higher the ΔE* value, the better is the buildup of the colour.

The stability of the colouring of the keratin fibres over time, especially after 9 days, was also evaluated by measuring the colour coordinates of the keratin fibres and comparing them with the colour coordinates immediately after performing the dyeing process according to the invention. The colour difference ΔE* between the colour at T0 and the colour after 9 days represents the stability of the colour of the hair and is calculated by means of the following equation:

$$\Delta E_{stab}^* = \sqrt{(L_{3s}^* - L_o^*)^2 + (a_{3s}^* - a_o^*)^2 + (b_{3s}^* - b_o^*)^2}$$

In this equation, $L_0$*, $a_0$* and $b_0$* represent the colorimetric coordinates measured on locks of hair at T0 immediately after performing the process and $L_{3s}$*, $a_{3s}$* and $b^*_{3s}$ represent the colorimetric coordinates 9 days after performing the process. The smaller the value of ΔE*stab, the more stable the colouring.

In particular, in the context of the invention, a colouring for which the ΔE* 9 days after dyeing is less than 2 is considered as stable over time.

Comment: the change in colouring after 3 weeks may similarly be evaluated from the colorimetric coordinates 3 weeks after dyeing the keratin fibres.

In the context of the present invention, it is thus preferably sought to obtain, immediately after the dyeing process, a value of b* that is as small as possible and/or a value of a* that is as high as possible. It is sought to obtain these results while at the same time having efficient colour buildup (large colour buildup value) and colouring that is stable over time.

The examples that follow serve to illustrate the invention.

**EXAMPLE**

The following compositions were prepared from the following ingredients in the proportions indicated in grams:

*Composition A*

| | |
|---|---|
| *Lawsonia inermis* powder sold by Intermarket Négoce Exportateur | 5 |
| Indigo plant *(Indigofera tinctoria)* leaf powder (sold by Nomade Palize) | 20 |
| Water | qs 100 |

*Dye compositions 6*

The following compositions according to the invention are prepared:

| | B1 | B1' (invention) | B2 | B2' (invention) | B3 | B3' ( invention ) |
|---|---|---|---|---|---|---|
| EXT. VIOLET 2 / CI 60730 | - | - | - | - | 0.03 | 0.03 |
| ORANGE 4 / CI 15510 | - | - | - | - | 0.05 | 0.05 |
| Carminic acid | - | - | 1 | 1 | - | - |
| Lactic acid | 2.2 | 2.2 | 2.2 | 2.2 | 2.2 | 2.2 |
| Phosphoric acid | - | 0.26 | - | 0.26 | - | 0.26 |

(continued)

| | B1 | B1' (invention) | B2 | B2' (invention) | B3 | B3' ( invention ) |
|---|---|---|---|---|---|---|
| Sodium phosphate | - | 0.26 | - | 0.26 | - | 0.26 |
| Benzyl alcohol | 1 | 1 | 1 | 1 | 1 | 1 |
| Ethanol | 3.5 | 3.5 | 3.5 | 3.5 | 3.5 | 3.5 |
| Hydroxyethyl acrylate/sodium acryloyldimethylpropanesulfonate copolymer in powder form, sold by SEPPIC under the trade name Sepinov EMT 10 | 4 | | | | | |
| Deionized water | qs 100 | | | | | |
| pH | 2.2 ± 0.3 | | | | | |

*Process*

Composition A, prepared at the time of use by mixing the dyes and water at 50°C, is applied to locks of natural hair containing 90% white hairs, at a rate of 10 g of composition per 1 g of hair, left to stand on the locks for 60 minutes at 33°C under a plastic film, followed by rinsing the locks, washing them with a standard shampoo and then wringing them dry.

Each of the compositions B1 to B3 is then applied at a rate of 5 g of mixture per gram of hair and left to stand on the hair for 20 minutes at 40°C under a plastic film.

On conclusion of the leave-on time, all the locks are then rinsed and washed with Ultra Doux Camomille shampoo, and then dried with a hairdryer.

The colorimetric measurements were performed using a Minolta CM3600D spectrocolorimeter (illuminant D65, angle 10°, specular component included) in the CIELab system.

In this system, L* represents the lightness. The smaller the value of L*, the darker and more powerful the colouring obtained. The chromaticity is represented by the values a* and b*, a* representing the red/green axis and b* the yellow/blue axis.

The colour buildup is represented by the colour difference ΔE* between the undyed lock and the dyed lock: the greater the value of ΔE*, the greater the colour buildup. This value is calculated from the following equation (i):

$$\Delta E = \sqrt{(L^* - L_o{}^*)^2 + (a^* - a_o{}^*)^2 + (b^* - b_o{}^*)^2} \quad \text{(i)}$$

In the equation (i), L*, a* and b* represent the values measured on locks of undyed hair and $L_0$*, $a_0$* and $b_0$* represent the values measured on locks of dyed hair.

| | L* | a* | b* | DE* | Colour at $T_0$ |
|---|---|---|---|---|---|
| Hair treated with A + B1 | 46.69 | 4.76 | 15.49 | 22.43 | *yellow-green beige* |
| Hair treated with A + B1' (invention) | 41.13 | 2.11 | **11.16** | **28.43** | *strong cold beige* |
| Hair treated with A + B2 | 41.93 | 5.2 | 11.83 | 27.78 | *golden beige* |
| Hair treated with A + B2' (invention) | 37.7 | 3.5 | **9.51** | **32.24** | *strong beige* |
| Hair treated with A + B3 | 33.54 | 10.13 | 12.35 | 36.73 | *brown* |
| Hair treated with A + B3' (invention) | 31.21 | 6.64 | **9.53** | **38.84** | *strong brown* |

It is observed visually on the locks that the process of the invention (using compositions B1', B2' or B3') makes it possible to very markedly intensify the colour obtained on the hair relative to the comparative processes.

This is confirmed by the colorimetric measurements showing ΔE values that are significantly higher in the case

of the process according to the invention, i.e. a greater colour buildup, and also significantly lower values of L* in the case of the process of the invention, i.e. a more intense colouring.

In addition, the process according to the invention makes it possible to reduce the evolution of the colour and especially the change towards yellow.

Furthermore, it is observed visually that the scalp is stained little or not at all with the dyeing process according to the invention.

## Claims

1. Process for dyeing keratin fibres, in particular human keratin fibres such as the hair, in which said fibres are treated, in several separate steps, comprising:

    i) at least one step i) of dyeing said fibres using a cosmetic dye composition A, preferably an aqueous composition, comprising at least a) indigo and/or henna,
    ii) at least one step ii) of treating said fibres, comprising the application to the fibres of an aqueous composition B which comprises water, b) optionally at least one direct dye, other than henna and indigo, c) a buffer system comprising a mixture of an acid and of at least one conjugate salt thereof, composition B having a pH of less than or equal to 5,
    iii) optionally at least one step of intermediate rinsing of said fibres, preferably with water, between step i) and step ii) (or between step ii) and i) (depending on the order of steps i) and ii)).

2. Process according to the preceding claim, in which composition A comprises water, preferably comprises a water content ranging from 10% to 99% by weight, more particularly from 20% to 90% by weight and better still from 40% to 80% by weight relative to the weight of composition A.

3. Process according to either of the preceding claims, in which henna and/or indigo are in powder form.

4. Process according to any one of the preceding claims, in which the indigo is derived from indigo-producing plant(s), preferably of the genus *Indigofera* et plus particulièrement *Indigofera tinctoria.*

5. Process according to any one of the preceding claims, **characterized in that** the henna is red henna.

6. Process according to any one of the preceding claims, **characterized in that** composition A comprises at least 0.1% by weight of henna and/or indigo, relative to the total weight of said composition, more preferentially from 0.2% to 50% by weight, better still from 0.3% to 40% by weight, preferably from 1% to 30% by weight relative to the total weight of composition A.

7. Dyeing process according to one of the preceding claims, **characterized in that** the dye composition B comprises at least one natural dye, preferably chosen from spinulosin, orceins, polyphenols or ortho-diphenols (also referred to as ODPs in the rest of the description), curcumin, indoles such as isatin or indole-2,3-dione, indigoids including indigo, phthalocyanines and porphyrins in particular complexed to a metal, glycosyl or non-glycosyl iridoids, chromene dyes, anthraquinone and naphthoquinone dyes, juglone, spinulosin, chromene or chroman dyes, such as neoflavanols and neoflavanones, flavanols; anthocyanidols, orceins, betalains, and mixtures thereof.

8. Process according to any one of the preceding claims, **characterized in that** the dye composition B comprises at least one natural dye chosen from carmine, carmine ammonium, diosindigo, chlorophyllin, haematein, orcein, the following extracts: blackcurrant, blueberry, black rice, grape skin, hibiscus, red cabbage, black carrot, elderberry, rhubarb, monascus, purple sweet potato, goji, radish, orcein, gardenia, diospyros kaki, campeachy, quebracho, and mixtures thereof.

9. Process according to any one of the preceding claims, **characterized in that** the dye composition B comprises at least one synthetic direct dye, preferably chosen from anionic direct dyes, better still from anionic direct dyes chosen from those of formulae (I), (II), (III), (IV), (V), (VI), (VII), (VIII), (IX), (X), (XI) and (XII) below, and the mesomeric and tautomeric forms thereof:

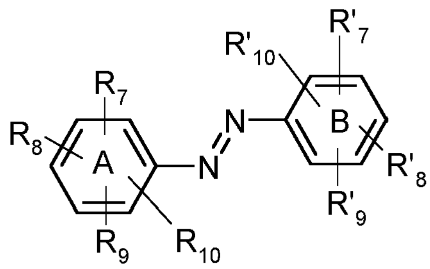

(I)

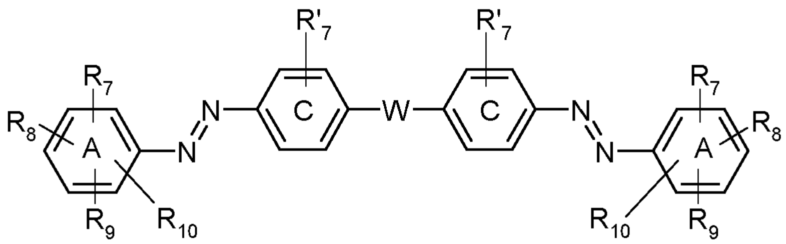

(II)

in which formulae (I) and (II):

- $R_7$, $R_8$, $R_9$, $R_{10}$, $R'_7$, $R'_8$, $R'_9$ and $R'_{10}$, which may be identical or different, represent a hydrogen atom or a group chosen from:

  - alkyl;
  - alkoxy, alkylthio;
  - hydroxyl, mercapto;
  - nitro, nitroso;
  - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
  - $(O)_2S(O^-)$-, $M^+$ with $M^+$ representing a hydrogen atom or a cationic counterion;
  - $(O)CO^-$-, $M^+$ with $M^+$ as defined previously;
  - R"-$S(O)_2$-, with R" representing a hydrogen atom or an alkyl, aryl, (di)(alkyl)amino or aryl(alkyl)amino group; preferentially a phenylamino or phenyl group;
  - R‴-$S(O)_2$-X'- with R‴ representing an alkyl or optionally substituted aryl group, X' as defined previously;
  - (di)(alkyl)amino;
  - aryl(alkyl)amino optionally substituted with one or more groups chosen from i) nitro; ii) nitroso; iii) $(O)_2S(O^-)$-, $M^+$ and iv) alkoxy, with $M^+$ as defined previously;
  - optionally substituted heteroaryl;
  - cycloalkyl;
  - Ar-N=N- with Ar representing an optionally substituted aryl group;
  - or alternatively two contiguous groups $R_7$ with $R_8$ or $R_8$ with $R_9$ or $R_9$ with $R_{10}$ together form a fused benzo group A'; and $R'_7$ with $R'_9$ or $R'_9$ with $R'_9$ or $R'_9$ with $R'_{10}$ together form a fused benzo group B'; with A' and B' optionally substituted with one or more groups chosen from i) nitro; ii) nitroso; iii) $(O)_2S(O^-)$-, $M^+$; iv) hydroxyl; v) mercapto; vi) (di)(alkyl)amino; vii) R°-C(X)-X'-; viii) R°-X'-C(X)-; ix) R°-X'-C(X)-X"-; x) Ar-N=N- and xi) optionally substituted aryl(alkyl)amino; with $M^+$, R°, X, X', X" and Ar as previously defined;

- W represents a sigma bond σ, an oxygen or sulfur atom, or a divalent radical i) - NR- with R as defined previously, or ii) methylene -$C(R_a)(R_b)$- with $R_a$ and $R_b$, which may be identical or different, representing a hydrogen atom or an aryl group, or alternatively $R_a$ and $R_b$ form, together with the carbon atom that bears them, a spiro cycloalkyl;
it being understood that formulae (I) and (II) comprise at least one sulfonate radical $(O)_2S(O^-)$-, $M^+$ or one carboxylate radical $(O)CO^-$-, $M^+$ on one of the rings A, A', B, B' or C; preferentially sodium sulfonate;

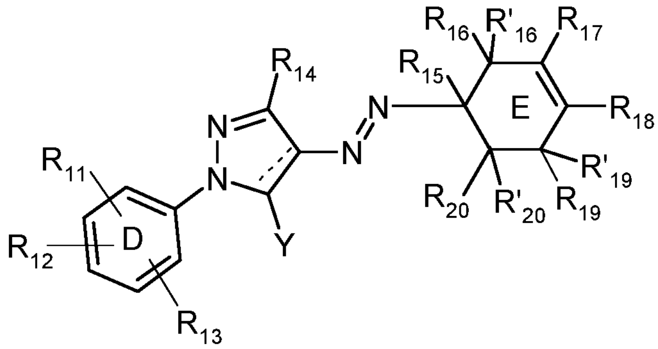

(III)

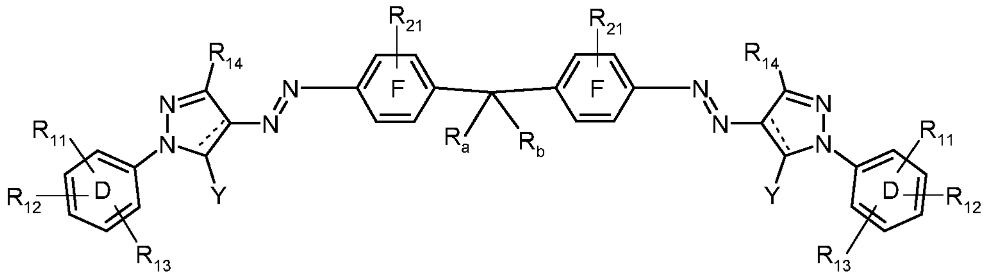

(IV)

in which formulae (III) and (IV):

- $R_{11}$, $R_{12}$ and $R_{13}$, which may be identical or different, represent a hydrogen or halogen atom, an alkyl group or $(O)_2S(O^-)$-, $M^+$ with $M^+$ as defined previously;
- $R_{14}$ represents a hydrogen atom, an alkyl group or a group $C(O)O^-$-, $M^+$ with $M^+$ as defined previously;
- $R_{15}$ represents a hydrogen atom;
- $R_{16}$ represents an oxo group, in which case $R'_{16}$ is absent, or alternatively $R_{15}$ with $R_{16}$ together form a double bond;
- $R_{17}$ and $R_{18}$, which may be identical or different, represent a hydrogen atom, or a group chosen from:

  - $(O)_2S(O^-)$-, $M^+$ with $M^+$ as defined previously;
  - Ar-O-$S(O)_2$- with Ar representing an optionally substituted aryl group, preferentially a phenyl optionally substituted with one or more alkyl groups;

- $R_{19}$ and $R_{20}$ together form either a double bond, or a benzo group D', which is optionally substituted;
- $R'_{16}$, $R'_{19}$ and $R'_{20}$, which may be identical or different, represent a hydrogen atom or an alkyl or hydroxyl group;
- $R_{21}$ represents a hydrogen atom or an alkyl or alkoxy group;
- $R_a$ and $R_b$, which may be identical or different, are as defined previously;
- Y represents either a hydroxyl group or an oxo group;
- ------- represents a single bond when Y is an oxo group; and represents a double bond when Y represents a hydroxyl group;

it being understood that formulae (III) and (IV) comprise at least one sulfonate radical $(O)_2S(O^-)$-, $M^+$ or one carboxylate radical $C(O)O^-$-, $M^+$ on one of the rings D or E; preferentially sodium sulfonate;

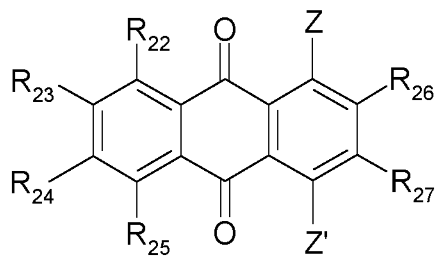

(V)

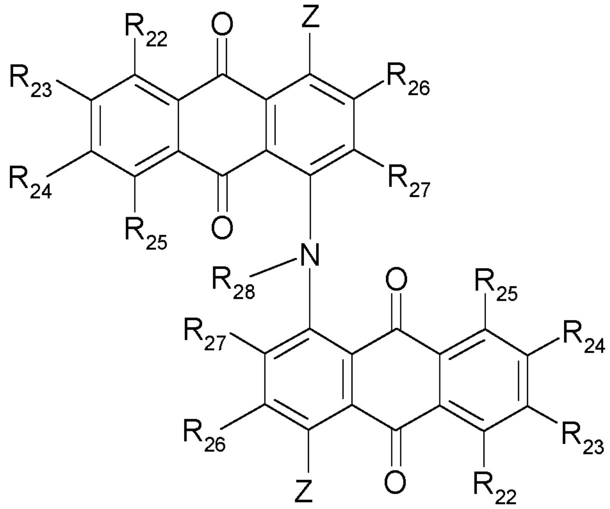

(VI)

in which formulae (V) and (VI):

- $R_{22}$, $R_{23}$, $R_{24}$, $R_{25}$, $R_{26}$ and $R_{27}$, which may be identical or different, represent a hydrogen or halogen atom, or a group chosen from:

  - alkyl;
  - hydroxyl, mercapto;
  - alkoxy, alkylthio;
  - optionally substituted aryloxy or arylthio;
  - aryl(alkyl)amino optionally substituted with one or more groups chosen from alkyl and $(O)_2S(O^-)$-, $M^+$ with $M^+$ as defined previously;
  - (di)(alkyl)amino;
  - (di)(hydroxyalkyl)amino;
  - $(O)_2S(O^-)$-, $M^+$ with $M^+$ as defined previously;

- Z' represents a hydrogen atom or a group $NR_{28}R_{29}$ with $R_{28}$ and $R_{29}$, which may be identical or different, representing a hydrogen atom or a group chosen from:

  - alkyl;
  - polyhydroxyalkyl such as hydroxyethyl;
  - aryl optionally substituted with one or more groups, more particularly i) alkyl such as methyl, n-dodecyl, n-butyl; ii) $(O)_2S(O^-)$-, $M^+$ with $M^+$ as defined previously; iii) R°-C(X)-X'-, R°-X'-C(X)-, R°-X'-C(X)-X"- with R°, X, X' and X" as defined previously;
  - cycloalkyl;

- Z represents a group chosen from hydroxyl and $NR'_{28}R'_{29}$ with $R'_{28}$ and $R'_{29}$, which may be identical or different, representing the same atoms or groups as $R_{28}$ and $R_{29}$ as defined previously;

it being understood that formulae (V) and (VI) comprise at least one sulfonate radical $(O)_2S(O^-)$-, $M^+$ or one carboxylate radical $C(O)O^-$-, $M^+$; preferentially sodium sulfonate;

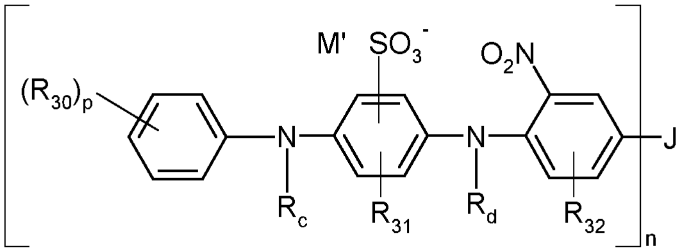

(VII)

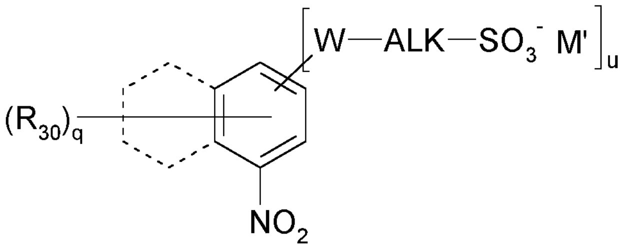

(VIII)

in which formulae (VII) and (VIII):

- $R_{30}$, $R_{31}$ and $R_{32}$, which may be identical or different, represent a hydrogen or halogen atom, or a group chosen from:

  - alkyl;
  - alkoxy optionally substituted with one or more hydroxyl groups, alkylthio optionally substituted with one or more hydroxyl groups;
  - hydroxyl, mercapto;
  - nitro, nitroso;
  - polyhaloalkyl;
  - R°-C(X)-X'-, R°-X'-C(X)-, R°-X'-C(X)-X"- with R°, X, X' and X" as defined previously;
  - $(O)_2S(O^-)$-, $M^+$ with $M^+$ as defined previously;
  - $(O)CO^-$-, $M^+$ with $M^+$ as defined previously;
  - (di)(alkyl)amino;
  - (di)(hydroxyalkyl)amino;
  - heterocycloalkyl;

- $R_c$ and $R_b$, which may be identical or different, represent a hydrogen atom or an alkyl group;
- W is as defined previously;
- ALK represents a linear or branched divalent $C_1$-$C_6$ alkylene group;
- n is 1 or 2;
- p represents an integer inclusively between 1 and 5;
- q represents an integer inclusively between 1 and 4;
- u is 0 or 1;
- when n is 1, J represents a nitro or nitroso group;
- when n is 2, J represents an oxygen or sulfur atom, or a divalent radical $-S(O)_m$-with m representing an integer 1 or 2;
- M' represents a hydrogen atom or a cationic counterion;
-

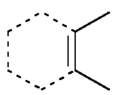,

which may be present or absent, represents a benzo group optionally substituted with one or more $R_{30}$

groups as defined previously;

it being understood that formulae (VII) and (VIII) comprise at least one sulfonate radical $(O)_2S(O^-)$-, $M^+$ or one carboxylate radical $C(O)O^-$-, $M^+$;

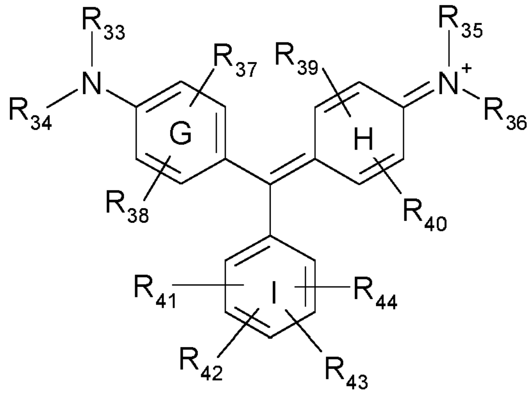

(IX)

in which formula (IX):

- $R_{33}$, $R_{34}$, $R_{35}$ and $R_{36}$, which may be identical or different, represent a hydrogen atom or a group chosen from alkyl, optionally substituted aryl and optionally substituted arylalkyl;
- $R_{37}$, $R_{38}$, $R_{39}$, $R_{40}$, $R_{41}$, $R_{42}$, $R_{43}$ and $R_{44}$, which may be identical or different, represent a hydrogen atom or a group chosen from:
  - alkyl;
  - alkoxy, alkylthio;
  - (di)(alkyl)amino;
  - hydroxyl, mercapto;
  - nitro, nitroso;
  - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
  - $(O)_2S(O^-)$-, $M^+$ with $M^+$ representing a hydrogen atom or a cationic counterion;
  - $(O)CO^-$-, $M^+$ with $M^+$ as defined previously;
  - or alternatively two contiguous groups $R_{41}$ with $R_{42}$ or $R_{42}$ with $R_{43}$ or $R_{43}$ with $R_{44}$ together form a fused benzo group: I'; with I' optionally substituted with one or more groups chosen from i) nitro; ii) nitroso; iii) $(O)_2S(O^-)$-, $M^+$; iv) hydroxyl; v) mercapto; vi) (di)(alkyl)amino; vii) R°-C(X)-X'-; viii) R°-X'-C(X)- and ix) R°-X'-C(X)-X"-; with $M^+$, R°, X, X' and X" as defined previously;

more particularly, $R_{37}$ to $R_{40}$ represent a hydrogen atom, and $R_{41}$ to $R_{44}$, which may be identical or different, represent a hydroxyl group or $(O)_2S(O^-)$-, $M^+$; and when $R_{43}$ with $R_{44}$ together form a benzo group, it is preferentially substituted with an $(O)_2S(O^-)$- group;
it being understood that at least one of the rings G, H, I or I' comprises at least one sulfonate radical $(O)_2S(O^-)$- or one carboxylate radical $C(O)O^-$-;

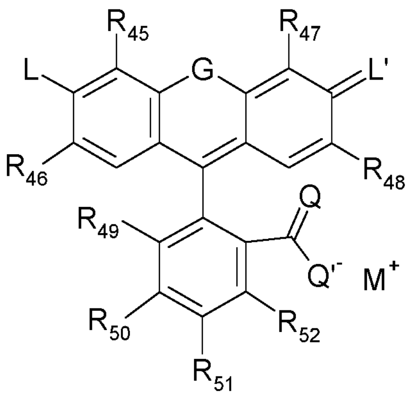

(X)

in which formula (X):

- $R_{45}$, $R_{46}$, $R_{47}$ and $R_{48}$, which may be identical or different, represent a hydrogen or halogen atom;
- $R_{49}$, $R_{50}$, $R_{51}$ and $R_{52}$, which may be identical or different, represent a hydrogen or halogen atom, or a group chosen from:
    - alkyl;
    - alkoxy, alkylthio;
    - hydroxyl, mercapto;
    - nitro, nitroso;
    - $(O)_2S(O^-)$-, $M^+$ with $M^+$ representing a hydrogen atom or a cationic counterion;
    - $(O)CO^-$-, $M^+$ with $M^+$ as defined previously;

preferably, $R_{49}$, $R_{50}$, $R_{51}$ and $R_{52}$ represent a hydrogen or halogen atom;
- G represents an oxygen or sulfur atom or a group $NR_e$ with $R_e$ as defined previously;
- L represents an alkoxide $O^-$, $M^+$; a thioalkoxide $S^-$, $M^+$ or a group $NR_f$, with $R_f$ representing a hydrogen atom or an alkyl group and $M^+$ as defined previously;
- L' represents an oxygen or sulfur atom or an ammonium group: $N^+R_fR_g$, with $R_f$ and $R_g$, which may be identical or different, representing a hydrogen atom, or an alkyl group or aryl group which is optionally substituted;
- Q and Q', which may be identical or different, represent an oxygen or sulfur atom;
- $M^+$ is as defined previously;

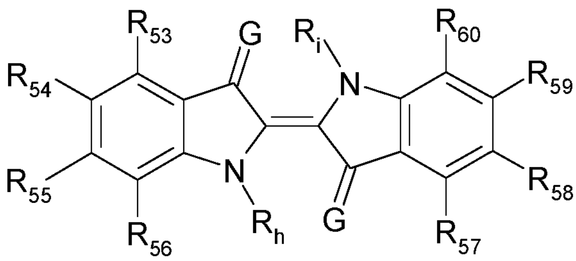

(XI)

in which formula (XI):

- $R_{53}$, $R_{54}$, $R_{55}$, $R_{56}$, $R_{57}$, $R_{58}$, $R_{59}$ and $R_{60}$, which may be identical or different, represent a hydrogen atom or a group chosen from:
    - alkyl;
    - alkoxy, alkylthio;
    - hydroxyl, mercapto;
    - nitro, nitroso;
    - R°-C(X)-X'-, R°-X'-C(X)-, R°-X'-C(X)-X"- with R° representing a hydrogen atom or an alkyl or aryl

group; X, X' and X", which may be identical or different, representing an oxygen or sulfur atom, or NR with R representing a hydrogen atom or an alkyl group;
- $(O)_2S(O^-)$-, $M^+$ with $M^+$ representing a hydrogen atom or a cationic counterion;
- $(O)CO^-$-, $M^+$ with $M^+$ as defined previously;
- G represents an oxygen or sulfur atom or a group $NR_e$ with $R_e$ as defined previously;
- $R_i$ and $R_h$, which may be identical or different, represent a hydrogen atom or an alkyl group;

it being understood that formula (XI) comprises at least one sulfonate radical $(O)_2S(O^-)$-, $M^+$ or one carboxylate radical $C(O)O^-$-, $M^+$;

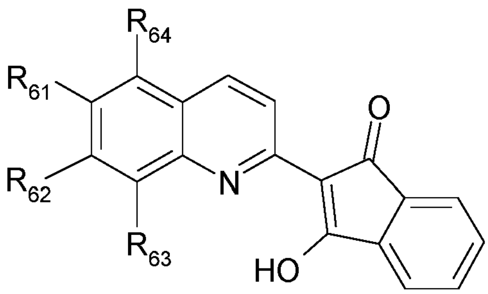

(XII)

- $R_{61}$ represents a hydrogen or halogen atom or an alkyl group;
- $R_{62}$, $R_{63}$, and $R_{64}$, which may be identical or different, represent a hydrogen atom or a group $(O)_2S(O^-)$-, $M^+$ with $M^+$ representing a hydrogen atom or a cationic counterion;
- or alternatively $R_{61}$ with $R_{62}$, or $R_{61}$ with $R_{64}$, together form a benzo group optionally substituted with one or more groups $(O)_2S(O^-)$-, $M^+$ with $M^+$ representing a hydrogen atom or a cationic counterion;

it being understood that formula (XII) comprises at least one sulfonate radical $(O)_2S(O^-)$-,
the at least one synthetic direct dye being preferably chosen from azo direct dyes, specifically those of formula (I), and anthraquinone dyes, specifically those of formula (V).

**10.** Process according to any one of the preceding claims, **characterized in that** the direct dye(s) are chosen from 1,2-dihydroxy-9,10-anthraquinone-3-sulfonic acid, the monosodium salt of 2-[(9,10-dihydro-4-hydroxy-9,10-dioxo-1-anthracenyl)amino]-5-methylbenzenesulfonic acid, the monosodium salt of 4-[(2-hydroxy-1-naphthyl)azo]benzenesulfonic acid, the disodium salt of 6-hydroxy-5-[(4-sulfophenyl)azo]-2-naphthalenesulfonic acid, the disodium salt of 5-amino-4-hydroxy-3-(phenylazo)-2,7-naphthalenedisulfonic acid, the disodium salt of 1-amino-2-(4'-nitrophenylazo)-7-phenylazo-8-hydroxy-3,6-naphthalenedisulfonic acid, the disodium salt of N-ethyl-N-[4-[[4-[ethyl[3-sulfophenyl]methyl]amino]phenyl](2-sulfophenyl)methylene]-2,5-cyclohexadien-1-ylidene]-3-sulfobenzenemethanaminium hydroxide, the disodium salt of 2,2'-[(9,10-dihydro-9,10-dioxo-1,4-anthracenediyl)diimino]bis[5-methyl]benzenesulfonic acid, the trisodium salt of 5-hydroxy-1-(4-sulfophenyl)-4-(4-sulfophenylazo)pyrazole-3-carboxylic acid, sodium 4-[(9,10-dihydro-4-hydroxy-9,10-dioxo-1-anthryl)amino]toluene-3-sulfonate, the trisodium salt of 7-hydroxy-8-[(4-sulfo-1-naphthalenyl)azo]-1,3-naphthalenedisulfonic acid, and a mixture of these compounds.

**11.** Process according to any one of the preceding claims, **characterized in that** the direct dye(s) represent a total content of at least 0.05% by weight, better still at least 0.1% by weight, preferably at least 0.15% by weight, preferably ranging from 0.05% to 5%, better still from 0.1% to 3% by weight and better still from 0.15% to 2% by weight relative to the total weight of composition B.

**12.** Process according to any one of the preceding claims, **characterized in that** the buffer system comprises an inorganic acid preferably chosen from inorganic acids with a pKa (pka1) of less than 4.5, preferably ranging from 1 to 4 and better still from 2 to 4.

**13.** Process according to any one of the preceding claims, **characterized in that** the inorganic acid is chosen from phosphorus-based acids, such as phosphoric acid, halogen-based acids, such as hydrochloric acid, and sulfur-based acids, such as sulfuric acid, and mixtures thereof.

**14.** Process according to any one of the preceding claims, **characterized in that** the inorganic acid is phosphoric acid.

**15.** Process according to any one of the preceding claims, **characterized in that** the conjugate acid salt(s) are chosen from inorganic acid salts, preferably from inorganic phosphate salts, in particular chosen from potassium dihydrogen phosphate $KH_2PO_4$, sodium dihydrogen phosphate $NaH_2PO_4$, dipotassium hydrogen phosphate $K_2HPO_4$, disodium hydrogen phosphate $Na_2HPO_4$, potassium phosphate $K_3PO_4$ and sodium phosphate $Na_3PO_4$, and mixtures thereof, preferably from potassium dihydrogen phosphate $KH_2PO_4$ and sodium dihydrogen phosphate $NaH_2PO_4$.

**16.** Process according to any one of the preceding claims, **characterized in that** the acid, preferably the inorganic acid, represents from 0.05% to 5% by weight, preferably from 0.1% to 3% by weight and better still from 0.15% to 2% by weight relative to the total weight of composition B.

**17.** Process according to any one of the preceding claims, **characterized in that** the conjugate acid salt, preferably the inorganic acid salt, is present in a content ranging from 0.05 to 5% by weight, preferably from 0.1 to 3% by weight and better still from 0.15 to 2% by weight, relative to the total weight of composition B.

**18.** Process according to any one of the preceding claims, **characterized in that** the acid, preferably the inorganic acid, and the conjugate salt(s) thereof are used in the composition according to the invention in an acid/conjugate salt(s) mole ratio ranging from 1:10 to 10:1, better still from 1:5 to 5:1, even better still from 1:3 to 3:1 and more preferably from 1:2 to 2:1.

**19.** Process according to any one of the preceding claims, **characterized in that** composition B comprises one or more liquid compounds with a Hansen solubility parameter value $\delta H$ of less than 16 $MPa^{1/2}$.

**20.** Process according to Claim 19, **characterized in that** the liquid compound(s) with a Hansen solubility parameter value $\delta H$ of less than 16 $MPa^{1/2}$ are chosen from:

• alcohol ethers, in particular $C_1$-$C_4$ ethers of $C_5$-$C_{30}$ alcohols, which are preferably saturated, linear or branched, optionally interrupted with one or more non-adjacent ether functions;
• aliphatic esters of $C_1$-$C_4$ carboxylic acids and of $C_3$-$C_{10}$ monoalcohols or polyhydroxylated alcohols, interrupted with one or more non-adjacent ether functions;
• aromatic ethers, in particular of $C_6$-$C_{10}$, of a $C_1$-$C_6$ alkyl optionally bearing a hydroxyl group,
• ($C_6$-$C_{10}$)aryl($C_1$-$C_6$)alkyl ethers, of a $C_1$-$C_6$ alkyl optionally bearing a hydroxyl group,
• alkanols bearing aryl substituents, preferably for which the aryl part is a $C_6$-$C_{10}$ aryl part, advantageously a $C_6$ aryl part, and the alkyl part of the alkanol is a $C_1$-$C_4$ alkyl part, it being possible for this alkyl part to be terminated or interrupted with a heteroatom, advantageously oxygen, or a hydroxyl group, preferably such as benzyl alcohol;
• lactones, preferably of formula (iii), and also mixtures thereof, with:

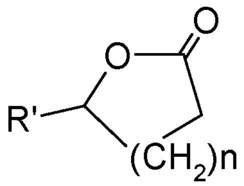
(iii)

in which R' represents a hydrogen, a linear or branched $C_1$-$C_8$ alkyl, a linear or branched $C_1$-$C_4$ hydroxyalkyl, n being equal to 1, 2 or 3, and preferably R' represents a hydrogen, a linear or branched $C_1$-$C_6$ alkyl or a linear or branched $C_1$-$C_2$ hydroxyalkyl.

**21.** Process according to either of Claims 19 and 20, **characterized in that** c) the liquid compound(s) with a Hansen solubility parameter value $\delta H$ of less than 16 $MPa^{1/2}$ are chosen from alcohol ethers, aliphatic esters, aromatic ethers, alkanols bearing aryl substituents, and mixtures thereof, preferably benzyl alcohol, phenylethanol and phenoxyethanol.

**22.** Process according to any one of Claims 19 to 21, **characterized in that** the liquid compound(s) with a Hansen solubility parameter value $\delta H$ of less than 16 $MPa^{1/2}$ represent a total content ranging from 0.1% to 35% by weight, preferably from 2% to 20% by weight and better still from 0.3% to 5% by weight, relative to the total weight of composition B.

**23.** Process according to any one of the preceding claims, **characterized in that** composition B comprises one or more linear or branched alcohols, preferably one or more linear or branched monoalcohols containing from 1 to 8 carbon atoms, preferably containing from 1 to 4 carbon atoms, and more preferably comprises one or more linear monoalcohols containing from 1 to 8 carbon atoms and better still from 1 to 4 carbon atoms.

**24.** Process according to Claim 23, **characterized in that** the linear or branched alcohol(s) containing from 1 to 8 carbon atoms are chosen from ethanol, propanol, butanol, isopropanol and isobutanol, and mixtures thereof, preferably ethanol.

**25.** Process according to any one of Claims 19 to 24, **characterized in that** the weight ratio of the total amount of liquid compound(s) with a Hansen solubility parameter value $\delta H$ of less than 16 $MPa^{1/2}$ to the total amount of linear or branched alcohol(s) containing from 1 to 8 carbon atoms is less than or equal to 1 and preferably ranges from 0.1 to 0.9.

**26.** Process according to any one of the preceding claims, **characterized in that** composition B comprises an organic acid other than the acid of the buffer system b), preferably with a $pK_a$ of less than 5, in particular a $pK_a$ of less than 4.5 and better still a $pK_a$ of less than 4, the $pK_a$ possibly ranging from 1 to 5 and better still from 2 to 4, preferably chosen from citric acid, lactic acid, malic acid, tartaric acid and glycolic acid.

**27.** Process according to any one of the preceding claims, **characterized in that** composition B has a pH of less than or equal to 4, better still less than or equal to 3, in particular a pH ranging from 1 to 5, better still from 1.5 to 4 and even better still from 2 to 3.

**28.** Process according to any one of the preceding claims, **characterized in that** water represents from 40% to 99% by weight, preferably from 50% to 95% by weight and more preferentially from 60% to 90% by weight relative to the total weight of composition B.

**29.** Dyeing process according to any one of the preceding claims, **characterized in that** it involves:

i) step i) of treating said fibres using the cosmetic composition A as defined according to any one of the preceding claims, and then
ii) step ii) of treating with composition B as defined according to any one of the preceding claims.

**30.** Dyeing process according to any one of the preceding claims, **characterized in that** it involves a rinsing step iii), preferably with water, between steps i) and ii).

**Patentansprüche**

**1.** Verfahren zum Färben von Keratinfasern, insbesondere menschlichen Keratinfasern, wie z.B. dem Haar, wobei die Fasern in mehreren getrennten Schritten behandelt werden, umfassend:

i) wenigstens einen Schritt i) des Färbens der Fasern unter Verwendung einer kosmetischen Farbstoffzusammensetzung A, vorzugsweise einer wässrigen Zusammensetzung, die wenigstens a) Indigo und/oder Henna umfasst,
ii) wenigstens einen Schritt ii) der Behandlung der Fasern, umfassend Aufbringen einer wässrigen Zusammensetzung B, die Wasser, b) gegebenenfalls wenigstens einen von Henna und Indigo verschiedenen Direktfarbstoff, c) ein Puffersystem umfassend ein Gemisch aus einer Säure und wenigstens einem Konjugatsalz davon umfasst, auf die Fasern, wobei die Zusammensetzung B einen pH-Wert von kleiner als oder gleich 5 aufweist,
iii) gegebenenfalls wenigstens einen Schritt der Zwischenspülung der Fasern, vorzugsweise mit Wasser, zwischen Schritt i) und Schritt ii) (oder zwischen Schritt ii) und i) (abhängig von der Reihenfolge der Schritte i) und ii)).

**2.** Verfahren nach dem vorstehenden Anspruch, wobei Zusammensetzung A Wasser umfasst, vorzugsweise einen Wassergehalt in dem Bereich von 10 Gew.-% bis 99 Gew.-%, insbesondere von 20 Gew.-% bis 90 Gew.-% und noch besser von 40 Gew.-% bis 80 Gew.-% bezogen auf das Gewicht der Zusammensetzung A umfasst.

**3.** Verfahren nach einem der vorstehenden Ansprüche, wobei Henna und/oder Indigo in Pulverform vorliegen.

**4.** Verfahren nach einem der vorstehenden Ansprüche, wobei das Indigo von indigoproduzierenden Pflanze(n), vorzugsweise aus der Gattung *Indigofera* und insbesondere *Indigofera tinctoria,* abgeleitet ist.

**5.** Verfahren nach einem der vorstehenden Ansprüche, **dadurch gekennzeichnet, dass** das Henna rotes Henna ist.

**6.** Verfahren nach einem der vorstehenden Ansprüche, **dadurch gekennzeichnet, dass** Zusammensetzung A wenigstens 0,1 Gew.-% Henna und/oder Indigo, bezogen auf das Gesamtgewicht der Zusammensetzung, bevorzugter von 0,2 Gew.-% bis 50 Gew.-%, noch besser von 0,3 Gew.-% bis 40 Gew.-%, vorzugsweise von 1 Gew.-% bis 30 Gew.-% bezogen auf das Gesamtgewicht der Zusammensetzung A umfasst.

**7.** Färbeverfahren nach einem der vorstehenden Ansprüche, **dadurch gekennzeichnet, dass** die Farbstoffzusammensetzung B wenigstens einen natürlichen Farbstoff umfasst, vorzugsweise ausgewählt aus Spinulosin, Orceinen, Polyphenolen oder Ortho-Diphenolen (im Rest der Beschreibung auch als ODPs bezeichnet), Curcumin, Indolen, wie z.B. Isatin oder Indol-2,3-dion, Indigoiden einschließlich Indigo, Phthalocyaninen und Porphyrinen, insbesondere im Komplex mit einem Metall, Glycosyl- oder Nichtglycosyl-Iridoiden, Chromenfarbstoffen, Anthrachinon- und Naphthochinonfarbstoffen, Juglon, Spinulosin, Chromen- oder Chromanfarbstoffen, wie z.B. Neoflavanolen und Neoflavanonen, Flavanolen; Anthocyanidolen, Orceinen, Betalainen und Gemischen davon.

**8.** Verfahren nach einem der vorstehenden Ansprüche, **dadurch gekennzeichnet, dass** die Farbstoffzusammensetzung B wenigstens einen natürlichen Farbstoff ausgewählt aus Karmin, Karminammonium, Diosindigo, Chlorophyllin, Hämatein, Orcein, den folgenden Extrakten: schwarze Johannisbeere, Heidelbeere, schwarzer Reis, Traubenhaut, Hibiskus, Rotkohl, schwarze Karotte, Holunder, Rhabarber, Monascus, lila Süßkartoffel, Goji, Rettich, Orcein, Gardenie, Diospyros kaki, Campeche, Quebracho und Gemischen davon umfasst.

**9.** Verfahren nach einem der vorstehenden Ansprüche, **dadurch gekennzeichnet, dass** die Farbstoffzusammensetzung B wenigstens einen synthetischen Direktfarbstoff umfasst, vorzugsweise ausgewählt aus anionischen Direktfarbstoffen, noch besser aus anionischen Direktfarbstoffen ausgewählt aus jenen der nachstehenden Formeln (I), (II), (III), (IV), (V), (VI), (VII), (VIII), (IX), (X), (XI) und (XII) und den mesomeren und tautomeren Formen davon:

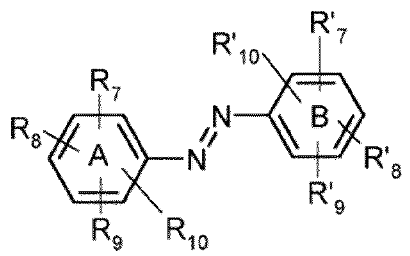

(I)

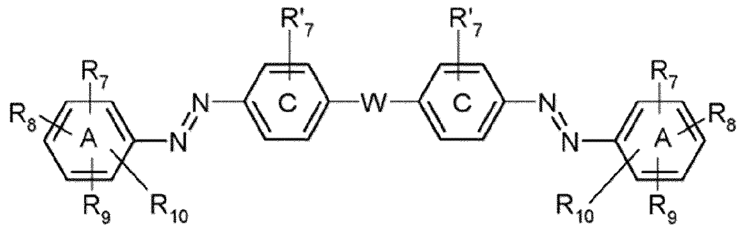

(II)

wobei in den Formeln (I) und (II):

- $R_7$, $R_8$, $R_9$, $R_{10}$, $R'_7$, $R'_8$, $R'_9$ und $R'_{10}$, die gleich oder verschieden sein können, ein Wasserstoffatom oder eine Gruppe darstellen, die ausgewählt ist aus:

  - Alkyl;
  - Alkoxy, Alkylthio;
  - Hydroxy, Mercapto;
  - Nitro, Nitroso;
  - R°-C(X)-X'-, R°-X'-C(X)-, R°-X'-C(X)-X"-, wobei R° ein Wasserstoffatom oder eine Alkyl- oder Arylgruppe darstellt; X, X' und X", die gleich oder verschieden sein können, ein Sauerstoff- oder Schwefelatom oder

NR darstellen, wobei R ein Wasserstoffatom oder eine Alkylgruppe darstellt;

- $(O)_2S(O^-)$-, $M^+$, wobei $M^+$ ein Wasserstoffatom oder ein kationisches Gegenion darstellt;
- $(O)CO^-$-, $M^+$, wobei $M^+$ wie vorstehend definiert ist;
- R"-$S(O)_2$-, wobei R" ein Wasserstoffatom oder eine Alkyl-, Aryl-, Di(alkyl)amino- oder Aryl(alkyl)aminogruppe darstellt; vorzugsweise eine Phenylamino- oder Phenylgruppe;
- R'''-$S(O)_2$-X' -, wobei R''' eine Alkyl- oder gegebenenfalls substituierte Arylgruppe darstellt, X' wie vorstehend definiert ist;
- (Di) (alkyl)amino;
- Aryl(alkyl)amino, gegebenenfalls substituiert mit einer oder mehreren Gruppen ausgewählt aus i) Nitro; ii) Nitroso; iii) $(O)_2S(O^-)$-, $M^+$ und iv) Alkoxy, wobei $M^+$ wie vorstehend definiert ist;
- gegebenenfalls substituiertem Heteroaryl;
- Cycloalkyl;
- Ar-N=N-, wobei Ar eine gegebenenfalls substituierte Arylgruppe darstellt;
- oder alternativ zwei zusammenhängende Gruppen $R_7$ mit $R_8$ oder $R_8$ mit $R_9$ oder $R_9$ mit $R_{10}$ zusammen eine verschmolzene Benzogruppe A' bilden; und $R'_7$ mit $R'_8$ oder $R'_8$ mit $R'_9$ oder $R'_9$ mit $R'_{10}$ zusammen eine verschmolzene Benzogruppe B' bilden; wobei A' und B' gegebenenfalls mit einer oder mehreren Gruppen ausgewählt aus i) Nitro; ii) Nitroso, iii) $(O)_2S(O^-)$-, $M^+$; iv) Hydroxy; v) Mercapto; vi) (Di) (alkyl)amino; vii) R°-C(X)-X'-; viii) R°-X'-C(X)-; ix) R°-X'-C(X)-X"-; x) Ar-N=N- und xi) gegebenenfalls substituiertem Aryl(alkyl)amino substituiert sind; wobei $M^+$, R°, X, X', X" und Ar wie vorstehend definiert sind;
- W eine Sigma-Bindung σ, ein Sauerstoff- oder Schwefelatom oder einen zweiwertigen Rest i) -NR-, wobei R wie vorstehend definiert ist, oder ii) Methylen - $C(R_a)(R_b)$ -, wobei $R_a$ und $R_b$, die gleich oder verschieden sein können, ein Wasserstoffatom oder eine Arylgruppe darstellen oder alternativ $R_a$ und $R_b$ zusammen mit dem Kohlenstoffatom, das sie trägt, ein Spiro-Cycloalkyl bilden, darstellt;

wobei zu beachten ist, dass die Formeln (I) und (II) wenigstens einen Sulfonatrest $(O)_2S(O^-)$ -, $M^+$ oder einen Carboxylatrest $(O)CO^-$-, $M^+$ an einem der Ringe A, A', B, B' oder C; vorzugsweise Natriumsulfonat, umfassen;

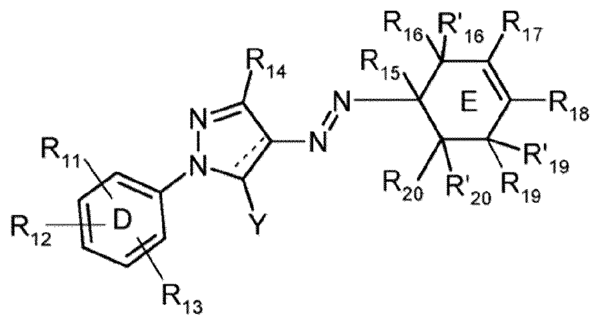

(III)

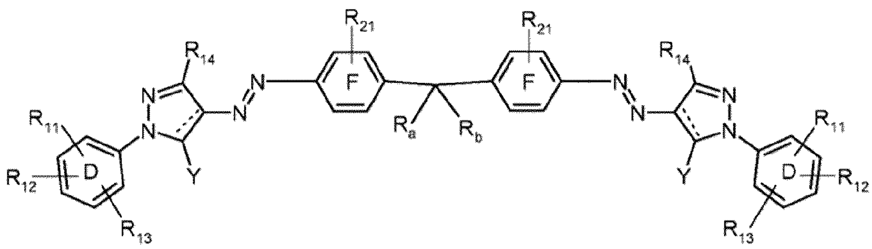

(IV)

wobei in den Formenl (III) und (IV):

- $R_{11}$, $R_{12}$ und $R_{13}$, die gleich oder verschieden sein können, ein Wasserstoff- oder Halogenatom, eine Alkylgruppe oder $(O)_2S(O^-)$-, $M^+$ darstellen, wobei $M^+$ wie vorstehend definiert ist;
- $R_{14}$ ein Wasserstoffatom, eine Alkylgruppe oder eine Gruppe $C(O)O^-$-, $M^+$ darstellt, wobei $M^+$ wie vorstehend definiert ist;
- $R_{15}$ ein Wasserstoffatom darstellt;
- $R_{16}$ eine Oxogruppe darstellt, in welchem Fall $R'_{16}$ nicht vorhanden ist, oder alternativ $R_{15}$ mit $R_{16}$ zusammen eine Doppelbindung bilden;

- $R_{17}$ und $R_{18}$, die gleich oder verschieden sein können, ein Wasserstoffatom oder eine Gruppe darstellen, die ausgewählt ist aus:
- $(O)_2S(O^-)$-, $M^+$, wobei $M^+$ wie vorstehend definiert ist;
- Ar-O-S$(O)_2$-, wobei Ar eine gegebenenfalls substituierte Arylgruppe darstellt, vorzugsweise ein Phenyl, das gegebenenfalls mit einer oder mehreren Alkylgruppen substituiert ist;
- $R_{19}$ und $R_{20}$ zusammen entweder eine Doppelbindung oder eine Benzogruppe D' bilden, die gegebenenfalls substituiert ist;
- $R'_{16}$, $R'_{19}$ und $R'_{20}$, die gleich oder verschieden sein können, ein Wasserstoffatom oder eine Alkyl- oder Hydroxygruppe darstellen;
- $R_{21}$ ein Wasserstoffatom oder eine Alkyl- oder Alkoxygruppe darstellt;
- $R_a$ und $R_b$, die gleich oder verschieden sein können, wie vorstehend definiert sind;
- Y entweder eine Hydroxygruppe oder eine Oxogruppe darstellt;
- ------ eine Einfachbindung darstellt, wenn Y eine Oxogruppe ist; und eine Doppelbindung darstellt, wenn Y eine Hydroxygruppe darstellt;

wobei zu beachten ist, dass die Formeln (III) und (IV) wenigstens einen Sulfonatrest $O_2S$ $(O^-)$ -, $M^+$ oder einen Carboxylatrest $C(O)O^-$-, $M^+$ an einem der Ringe D oder E; vorzugsweise Natriumsulfonat, umfassen;

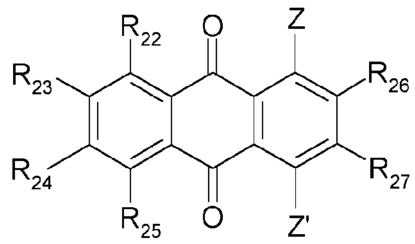

(V)

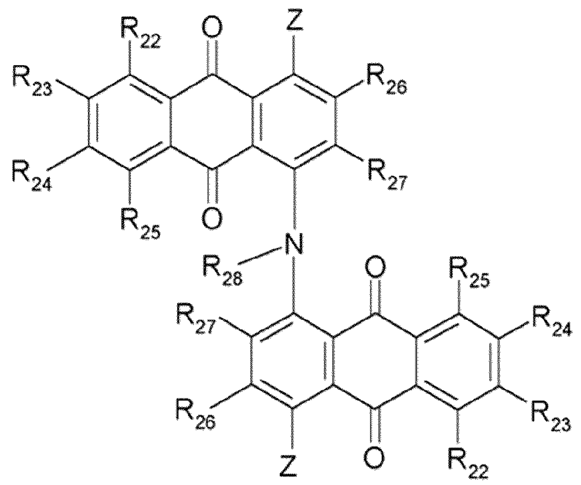

(VI)

wobei in den Formeln (V) und (VI):

- $R_{22}$, $R_{23}$, $R_{24}$, $R_{25}$, $R_{26}$ und $R_{27}$, die gleich oder verschieden sein können, ein Wasserstoff- oder Halogenatom oder eine Gruppe darstellen, die ausgewählt ist aus:
  - Alkyl;
  - Hydroxy, Mercapto;
  - Alkoxy, Alkylthio;
  - gegebenenfalls substituiertem Aryloxy oder Arylthio;
  - Aryl(alkyl)amino, gegebenenfalls substituiert mit einer oder mehreren Gruppen ausgewählt aus Alkyl und $(O)_2S(O^-)$-, $M^+$, wobei $M^+$ wie vorstehend definiert ist;
  - (Di)(alkyl)amino;
  - (Di)(hydroxyalkyl)amino;

- $(O)_2S(O^-)$-, $M^+$, wobei $M^+$ wie vorstehend definiert ist;
- Z' ein Wasserstoffatom oder eine Gruppe $NR_{28}R_{29}$ darstellt, wobei $R_{28}$ und $R_{29}$, die gleich oder verschieden sein können, ein Wasserstoffatom oder eine Gruppe darstellen, die ausgewählt ist aus:
- Alkyl;
- Polyhydroxyalkyl, wie z.B. Hydroxyethyl;
- Aryl, gegebenenfalls substituiert mit einer oder mehreren Gruppen, insbesondere i) Alkyl, wie z.B. Methyl, n-Dodecyl, n-Butyl; ii) $(O)_2S(O^-)$-, $M^+$, wobei $M^+$ wie vorstehend definiert ist; iii) R°-C(X)-X'-, R°-X'-C(X)-, R°-X'-C(X)-X"-, wobei R°, X, X' und X" wie vorstehend definiert sind;
- Cycloalkyl;
- Z eine Gruppe darstellt, die ausgewählt ist aus Hydroxy und $NR_{28}R_{29}$, wobei $R'_{28}$ und $R'_{29}$, die gleich oder verschieden sein können, die gleichen Atome oder Gruppen wie $R_{28}$ und $R_{29}$ wie vorstehend definiert darstellen;

wobei zu beachten ist, dass die Formeln (V) und (VI) wenigstens einen Sulfonatrest (O) $_2$S ($O^-$) -, $M^+$ oder einen Carboxylatrest $C(O)O^-$-, $M^+$; vorzugsweise Natriumsulfonat, umfassen;

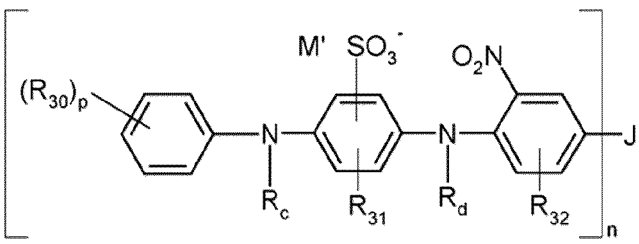

(VII)

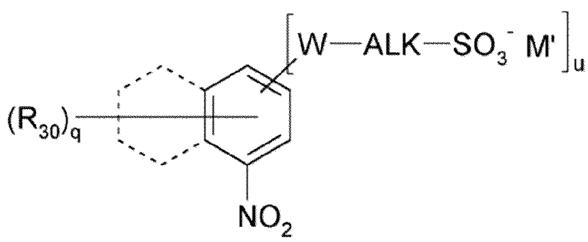

(VIII)

wobei in den Formeln (VII) und (VIII):

- $R_{30}$, $R_{31}$ und $R_{32}$, die gleich oder verschieden sein können, ein Wasserstoff- oder Halogenatom oder eine Gruppe darstellen, die ausgewählt ist aus:

- Alkyl;
- Alkoxy, gegebenenfalls substituiert mit einer oder mehreren Hydroxygruppen, Alkylthio, gegebenenfalls substituiert mit einer oder mehreren Hydroxygruppen;
- Hydroxy, Mercapto;
- Nitro, Nitroso;
- Polyhalogenalkyl;
- R°-C(X)-X'-, R°-X'-C(X)-, R°-X'-C (X)-X''-, wobei R°, X, X' und X" wie vorstehend definiert sind;
- $(O)_2S(O^-)$ -, $M^+$, wobei $M^+$ wie vorstehend definiert ist;
- $(O)CO^-$-, $M^+$, wobei $M^+$ wie vorstehend definiert ist;
- (Di) (alkyl)amino;
- (Di) (hydroxyalkyl)amino;
- Heterocycloalkyl;
- $R_c$ und $R_d$, die gleich oder verschieden sein können, ein Wasserstoffatom oder eine Alkylgruppe darstellen;
- W wie vorstehend definiert ist;
- ALK eine lineare oder verzweigte zweiwertige $C_1$-$C_6$-Alkylengruppe darstellt;
- n 1 oder 2 ist;
- p eine ganze Zahl zwischen einschließlich 1 und 5 darstellt;

- q eine ganze Zahl zwischen einschließlich 1 und 4 darstellt;
- u 0 oder 1 ist;
- wenn n 1 ist, J eine Nitro- oder Nitrosogruppe darstellt;
- wenn n 2 ist, J ein Sauerstoff- oder Schwefelatom oder einen zweiwertigen Rest $-S(O)_m-$ darstellt, wobei m eine ganze Zahl 1 oder 2 darstellt;
- M' ein Wasserstoffatom oder ein kationisches Gegenion darstellt;
-

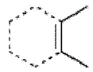,

das vorhanden oder nicht vorhanden sein kann, eine Benzogruppe darstellt, gegebenenfalls substituiert mit einer oder mehreren wie vorstehend definierten $R_{30}$-Gruppen;

wobei zu beachten ist, dass die Formeln (VII) und (VIII) wenigstens einen Sulfonatrest $(O)\ _2S\ (O^-)$ -, $M^+$ oder einen Carboxylatrest $C(O)O^-$-, $M^+$ umfassen;

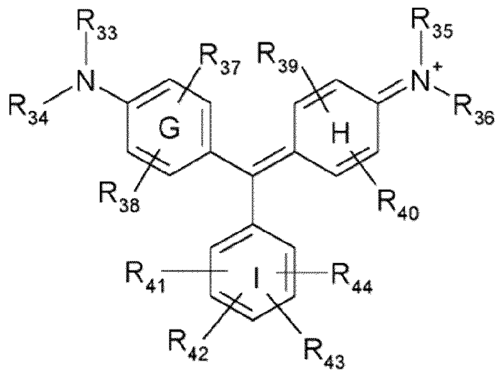

(IX)

wobei in Formel (IX):

- $R_{33}$, $R_{34}$, $R_{35}$ und $R_{36}$, die gleich oder verschieden sein können, ein Wasserstoffatom oder eine Gruppe ausgewählt aus Alkyl, gegebenenfalls substituiertem Aryl und gegebenenfalls substituiertem Arylalkyl darstellen;
- $R_{37}$, $R_{38}$, $R_{39}$, $R_{40}$, $R_{41}$, $R_{42}$, $R_{43}$ und $R_{44}$, die gleich oder verschieden sein können, ein Wasserstoffatom oder eine Gruppe darstellen, die ausgewählt ist aus:
- Alkyl;
- Alkoxy, Alkylthio;
- (Di) (alkyl)amino;
- Hydroxy, Mercapto;
- Nitro, Nitroso;
- R°-C(X)-X'-, R°-X'-C(X)-, R°-X'-C(X)-X"-, wobei R° ein Wasserstoffatom oder eine Alkyl- oder Arylgruppe darstellt; X, X' und X", die gleich oder verschieden sein können, ein Sauerstoff- oder Schwefelatom oder NR darstellen, wobei R ein Wasserstoffatom oder eine Alkylgruppe darstellt;
- $(O)_2S(O^-)$-, $M^+$, wobei $M^+$ ein Wasserstoffatom oder ein kationisches Gegenion darstellt;
- $(O)CO^-$-, $M^+$, wobei $M^+$ wie vorstehend definiert ist;
- oder alternativ zwei zusammenhängende Gruppen $R_{41}$ mit $R_{42}$ oder $R_{42}$ mit $R_{43}$ oder $R_{43}$ mit $R_{44}$ zusammen eine verschmolzene Benzogruppe: I' bilden; wobei I' gegebenenfalls mit einer oder mehreren Gruppen ausgewählt aus i) Nitro, ii) Nitroso, iii) $(O)_2S(O^-)$-, $M^+$; iv) Hydroxy; v) Mercapto; vi) Di)(alkyl)amino; vii) R°-C(X)-X'-; viii) R°-X'-C(X) - und ix) R°-X'-C(X)-X"- substituiert ist; wobei $M^+$, R°, X, X' und X" wie vorstehend definiert sind;

insbesondere $R_{37}$ bis $R_{40}$ ein Wasserstoffatom darstellen und $R_{41}$ bis $R_{44}$, die gleich oder verschieden sein können, eine Hydroxygruppe oder $(O)_2S(O^-)$-, $M^+$ darstellen; und wenn $R_{43}$ mit $R_{44}$ zusammen eine Benzogruppe bilden, diese vorzugsweise mit einer $(O)_2S(O^-)$-Gruppe substituiert ist; wobei zu beachten ist,

dass wenigstens einer der Ringe G, H, I und I' wenigstens einen Sulfonatrest (O) $_2$S ($O^-$)- oder einen Carboxylatrest C(O)$O^-$- umfasst;

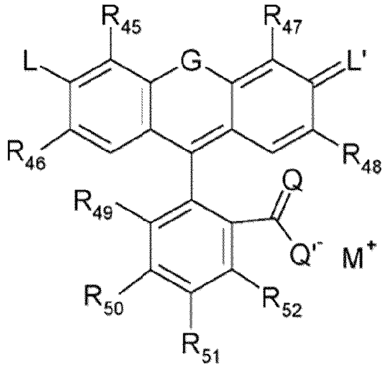

(X)

wobei in Formel (X):

- $R_{45}$, $R_{46}$, $R_{47}$ und $R_{48}$, die gleich oder verschieden sein können, ein Wasserstoff- oder Halogenatom darstellen;
- $R_{49}$, $R_{50}$, $R_{51}$ und $R_{52}$, die gleich oder verschieden sein können, ein Wasserstoff- oder Halogenatom oder eine Gruppe darstellen, die ausgewählt ist aus:
  - Alkyl;
  - Alkoxy, Alkylthio;
  - Hydroxy, Mercapto;
  - Nitro, Nitroso;
  - $(O)_2S(O^-)$-, $M^+$, wobei $M^+$ ein Wasserstoffatom oder ein kationisches Gegenion darstellt;
  - (O)$CO^-$-, $M^+$, wobei $M^+$ wie vorstehend definiert ist; wobei vorzugsweise $R_{49}$, $R_{50}$, $R_{51}$ und $R_{52}$ ein Wasserstoff- oder Halogenatom darstellen;
  - G ein Sauerstoff- oder Schwefelatom oder eine Gruppe $NR_e$ darstellt, wobei $R_e$ wie vorstehend definiert ist;
  - L ein Alkoxid $O^-$, $M^+$; ein Thioalkoxid $S^-$, $M^+$ oder eine Gruppe $NR_f$ darstellt, wobei $R_f$ ein Wasserstoffatom oder eine Alkylgruppe darstellt und $M^+$ wie vorstehend definiert ist;
  - L' ein Sauerstoff- oder Schwefelatom oder eine Ammoniumgruppe: $N^+R_fR_g$ darstellt, wobei $R_f$ und $R_g$, die gleich oder verschieden sein können, ein Wasserstoffatom oder eine Alkylgruppe oder Arylgruppe, die gegebenenfalls substituiert ist, darstellen;
  - Q und Q', die gleich oder verschieden sein können, ein Sauerstoff- oder Schwefelatom darstellen;
  - $M^+$ wie vorstehend definiert ist;

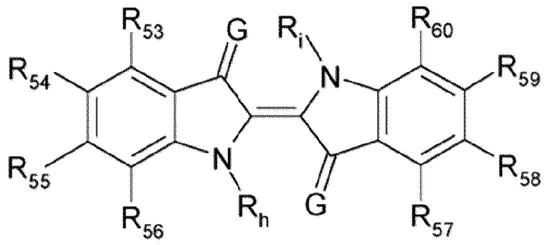

(XI)

wobei in Formel (XI):

- $R_{53}$ , $R_{54}$ , $R_{55}$ , $R_{56}$ , $R_{57}$ , $R_{58}$ , $R_{59}$ und $R_{60}$, die gleich oder verschieden sein können, ein Wasserstoffatom oder eine Gruppe darstellen, die ausgewählt ist aus:
  - Alkyl;
  - Alkoxy, Alkylthio;

- Hydroxy, Mercapto;
- Nitro, Nitroso;
- R°-C(X)-X'-, R°-X'-C(X)-, R°-X'-C(X)-X"-, wobei R° ein Wasserstoffatom oder eine Alkyl- oder Arylgruppe darstellt; X, X' und X", die gleich oder verschieden sein können, ein Sauerstoff- oder Schwefelatom oder NR darstellen, wobei R ein Wasserstoffatom oder eine Alkylgruppe darstellt;
- $(O)_2S(O^-)$-, $M^+$, wobei $M^+$ ein Wasserstoffatom oder ein kationisches Gegenion darstellt;
- $(O)CO^-$-, $M^+$, wobei $M^+$ wie vorstehend definiert ist;
- G ein Sauerstoff- oder Schwefelatom oder eine Gruppe $NR_e$ darstellt, wobei $R_e$ wie vorstehend definiert ist;
- $R_i$ und $R_h$, die gleich oder verschieden sein können, ein Wasserstoffatom oder eine Alkylgruppe darstellen;

wobei zu beachten ist, dass die Formel (XI) wenigstens einen Sulfonatrest $(O)_2S(O^-)$-, $M^+$ oder einen Carboxylatrest $C(O)O^-$-, $M^+$ umfasst;

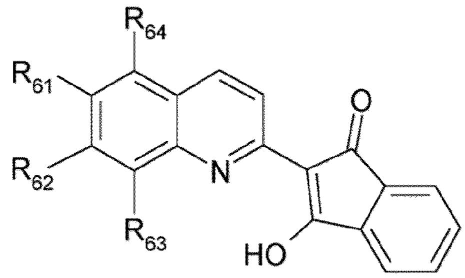

(XII)

- $R_{61}$ ein Wasserstoff- oder Halogenatom oder eine Alkylgruppe darstellt;
- $R_{62}$, $R_{63}$ und $R_{64}$, die gleich oder verschieden sein können, ein Wasserstoffatom oder eine Gruppe $(O)_2S(O^-)$-, $M^+$ darstellen, wobei $M^+$ ein Wasserstoffatom oder ein kationisches Gegenion darstellt;
- oder alternativ $R_{61}$ mit $R_{62}$ oder $R_{61}$ mit $R_{64}$ zusammen eine Benzogruppe bilden, gegebenenfalls substituiert mit einer oder mehreren Gruppen $(O)_2S(O^-)$-, $M^+$, wobei $M^+$ ein Wasserstoffatom oder ein kationisches Gegenion darstellt;

wobei zu beachten ist, dass die Formel (XII) wenigstens einen Sulfonatrest $(O)_2S(O^-)$- umfasst,
wobei der wenigstens eine synthetische Direktfarbstoff vorzugsweise ausgewählt ist aus Azo-Direktfarbstoffen, insbesondere jenen der Formel (I), und Anthrachinonfarbstoffen, insbesondere jenen der Formel (V) .

**10.** Verfahren nach einem der vorstehenden Ansprüche, **dadurch gekennzeichnet, dass** die Direktfarbstoff(e) ausgewählt sind aus 1,2-Dihydroxy-9,10-anthrachinon-3-sulfonsäure, dem Mononatriumsalz von 2-[(9,10-Dihydro-4-hydroxy-9,10-dioxo-1-anthracenyl)amino]-5-methylbenzolsulfonsäure, dem Mononatriumsalz von 4-[(2-Hydroxy-1-naphthyl)azo]benzolsulfonsäure, dem Dinatriumsalz von 6-Hydroxy-5-[(4-sulfophenyl)azo]-2-naphthalinsulfonsäure, dem Dinatriumsalz von 5-Amino-4-hydroxy-3-(phenylazo)-2,7-naphthalindisulfonsäure, dem Dinatriumsalz von 1-Amino-2-(4'-nitrophenylazo)-7-phenylazo-8-hydroxy-3,6-naphthalindisulfonsäure, dem Dinatriumsalz von N-Ethyl-N-[4-[[4-[ethyl[3-sulfophenyl]methyl]amino]phenyl](2-sulfophenyl)methylen]-2,5-cyclohexadien-1-yliden]-3-sulfobenzolmethanaminiumhydroxid, dem Dinatriumsalz von 2,2'-[(9,10-Dihydro-9,10-dioxo-1,4-anthracendiyl)diimino]bis[5-methyl]benzolsulfonsäure, dem Trinatriumsalz von 5-Hydroxy-1-(4-sulfophenyl)-4-(4-sulfophenylazo)pyrazol-3-carbonsäure, Natrium-4-[(9,10-Dihydro-4-hydroxy-9,10-dioxo-1-anthryl)amino]toluol-3-sulfonat, dem Trinatriumsalz von 7-Hydroxy-8-[(4-sulfo-1-naphthalenyl)azo]-1,3-naphthalindisulfonsäure und einem Gemisch dieser Verbindungen.

**11.** Verfahren nach einem der vorstehenden Ansprüche, **dadurch gekennzeichnet, dass** die Direktfarbstoff(e) einen Gesamtgehalt von wenigstens 0,05 Gew.-%, noch besser wenigstens 0,1 Gew.-%, vorzugsweise wenigstens 0,15 Gew.-%, vorzugsweise in dem Bereich von 0,05 Gew.-% bis 5 Gew.-%, noch besser von 0,1 Gew.-% bis 3 Gew.-% und noch besser von 0,15 Gew.-% bis 2 Gew.-% bezogen auf das Gesamtgewicht der Zusammensetzung B darstellen.

**12.** Verfahren nach einem der vorstehenden Ansprüche, **dadurch gekennzeichnet, dass** das Puffersystem eine anorganische Säure umfasst, vorzugsweise ausgewählt aus anorganischen Säuren mit einem pKa (pKa1) von weniger als 4,5, vorzugsweise in dem Bereich von 1 bis 4 und noch besser von 2 bis 4.

**13.** Verfahren nach einem der vorstehenden Ansprüche, **dadurch gekennzeichnet, dass** die anorganische Säure ausgewählt ist aus Säuren auf Phosphorbasis, wie z.B. Phosphorsäure, Säuren auf Halogenbasis, wie z.B. Salzsäure, und Säuren auf Schwefelbasis, wie z.B. Schwefelsäure, und Gemischen davon.

**14.** Verfahren nach einem der vorstehenden Ansprüche, **dadurch gekennzeichnet, dass** die anorganische Säure Phosphorsäure ist.

**15.** Verfahren nach einem der vorstehenden Ansprüche, **dadurch gekennzeichnet, dass** die Konjugatsäuresalz(e) ausgewählt sind aus anorganischen Säuresalzen, vorzugsweise aus anorganischen Phosphatsalzen, insbesondere ausgewählt aus Kaliumdihydrogenphosphat $KH_2PO_4$, Natriumdihydrogenphosphat $NaH_2PO_4$, Dikaliumhydrogenphosphat $K_2HPO_4$, Dinatriumhydrogenphosphat $Na_2HPO_4$, Kaliumphosphat $K_3PO_4$ und Natriumphosphat $Na_3PO_4$ und Gemischen davon, vorzugsweise aus Kaliumdihydrogenphosphat $KH_2PO_4$ und Natriumdihydrogenphosphat $NaH_2PO_4$.

**16.** Zusammensetzung nach einem der vorstehenden Ansprüche, **dadurch gekennzeichnet, dass** die Säure, vorzugsweise die anorganische Säure, von 0,05 Gew.-% bis 5 Gew.-%, vorzugsweise von 0,1 Gew.-% bis 3 Gew.-% und noch besser von 0,15 Gew.-% bis 2 Gew.-% bezogen auf das Gesamtgewicht der Zusammensetzung B darstellt.

**17.** Verfahren nach einem der vorstehenden Ansprüche, **dadurch gekennzeichnet, dass** das Konjugatsäuresalz, vorzugsweise das anorganische Säuresalz, in einem Gehalt in dem Bereich von 0,05 bis 5 Gew.-%, vorzugsweise von 0,1 bis 3 Gew.-% und noch besser von 0,15 bis 2 Gew.-% bezogen auf das Gesamtgewicht der Zusammensetzung B vorhanden ist.

**18.** Verfahren nach einem der vorstehenden Ansprüche, **dadurch gekennzeichnet, dass** die Säure, vorzugsweise die anorganische Säure, und die Konjugatsalz(e) davon in der Zusammensetzung gemäß der Erfindung in einem Säure/Konjugatsalz(e)-Molverhältnis in dem Bereich von 1:10 bis 10:1, noch besser von 1:5 bis 5:1, sogar noch besser von 1:3 bis 3:1 und bevorzugter von 1:2 bis 2:1 verwendet werden.

**19.** Verfahren nach einem der vorstehenden Ansprüche, **dadurch gekennzeichnet, dass** die Zusammensetzung B eine oder mehrere flüssige Verbindungen mit einem Hansen-Löslichkeitsparameterwert $\delta H$ von kleiner als 16 $MPa^{1/2}$ umfasst.

**20.** Verfahren nach Anspruch 19, **dadurch gekennzeichnet, dass** die flüssigen Verbindung(en) mit einem Hansen-Löslichkeitsparameterwert $\delta H$ von kleiner 16 $MPa^{1/2}$ ausgewählt sind aus:

- Alkoholethern, insbesondere $C_1$-$C_4$-Ethern von $C_5$-$C_{30}$-Alkoholen, die vorzugsweise gesättigt, linear oder verzweigt sind, gegebenenfalls unterbrochen durch eine oder mehrere nichtbenachbarte Etherfunktionen;
- aliphatischen Estern von $C_1$-$C_4$-Carbonsäuren und von $C_3$-$C_{10}$-Monoalkoholen oder polyhydroxylierten Alkoholen, unterbrochen durch eine oder mehrere nichtbenachbarte Etherfunktionen;
- aromatischen Ethern, insbesondere $C_6$-$C_{10}$, eines $C_1$-$C_6$-Alkyls, gegebenenfalls mit einer Hydroxygruppe,
- ($C_6$-$C_{10}$) Aryl ($C_1$-$C_6$) alkylethern eines $C_1$-$C_6$-Alkyls, gegebenenfalls mit einer Hydroxygruppe,
- Alkanolen, die Arylsubstituenten tragen, wobei deren Arylteil vorzugsweise ein $C_6$-$C_{10}$-Arylteil ist, vorteilhafterweise ein $C_6$-Arylteil, und der Alkylteil des Alkanols ein $C_1$-$C_4$-Alkylteil ist, wobei es möglich ist, dass dieser Alkylteil mit einem Heteroatom, vorteilhaft Sauerstoff, oder einer Hydroxygruppe beendet oder davon unterbrochen ist, vorzugsweise wie z.B. Benzylalkohol;
- Lactonen, vorzugsweise der Formel (iii), sowie Gemischen davon, mit:

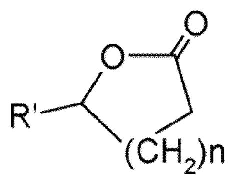

(iii)

wobei R' einen Wasserstoff, ein lineares oder verzweigtes $C_1$-$C_8$-Alkyl, ein lineares oder verzweigtes $C_1$-$C_4$-Hydroxyalkyl darstellt, wobei n gleich 1, 2 oder 3 ist, und vorzugsweise R' einen Wasserstoff, ein lineares oder verzweigtes $C_1$-$C_6$-Alkyl oder ein lineares oder verzweigtes $C_1$-$C_2$-Hydroxyalkyl darstellt.

**21.** Verfahren nach einem der Ansprüche 19 und 20, **dadurch gekennzeichnet, dass** c) die flüssigen Verbindung(en) mit einem Hansen-Löslichkeitsparameterwert $\delta H$ von kleiner als 16 $MPa^{1/2}$ ausgewählt sind aus Alkoholethern, aliphatischen Estern, aromatischen Ethern, Alkanolen mit Arylsubstituenten und Gemischen davon, vorzugsweise Benzylalkohol, Phenylethanol und Phenoxyethanol.

**22.** Verfahren nach einem der Ansprüche 19 bis 21, **dadurch gekennzeichnet, dass** die flüssigen Verbindung(en) mit einem Hansen-Löslichkeitsparameterwert $\delta H$ von kleiner als 16 $MPa^{1/2}$ einen Gesamtgehalt in dem Bereich von 0,1 Gew.-% bis 35 Gew.-%, vorzugsweise von 2 Gew.-% bis 20 Gew.-% und noch besser von 0,3 Gew.-% bis 5 Gew.-% bezogen auf das Gesamtgewicht der Zusammensetzung B darstellen.

**23.** Verfahren nach einem der vorstehenden Ansprüche, **dadurch gekennzeichnet, dass** die Zusammensetzung B einen oder mehrere lineare oder verzweigte Alkohole, vorzugsweise einen oder mehrere lineare oder verzweigte Monoalkohole, die von 1 bis 8 Kohlenstoffatome enthalten, vorzugsweise von 1 bis 4 Kohlenstoffatome enthalten, und bevorzugter einen oder mehrere lineare Monoalkohole, die von 1 bis 8 Kohlenstoffatome und noch besser von 1 bis 4 Kohlenstoffatome enthalten, umfasst.

**24.** Verfahren nach Anspruch 23, **dadurch gekennzeichnet, dass** die linearen oder verzweigten Alkohol(e), die von 1 bis 8 Kohlenstoffatome enthalten, ausgewählt sind aus Ethanol, Propanol, Butanol, Isopropanol und Isobutanol und Gemischen davon, vorzugsweise Ethanol.

**25.** Verfahren nach einem der Ansprüche 19 bis 24, **dadurch gekennzeichnet, dass** das Gewichtsverhältnis der Gesamtmenge von flüssigen Verbindung(en) mit einem Hansen-Löslichkeitsparameterwert $\delta H$ von kleiner als 16 $MPa^{1/2}$ zu der Gesamtmenge von linearen oder verzweigten Alkohol(en), die von 1 bis 8 Kohlenstoffatome enthalten, kleiner als oder gleich 1 ist und vorzugsweise in dem Bereich von 0,1 bis 0,9 liegt.

**26.** Verfahren nach einem der vorstehenden Ansprüche, **dadurch gekennzeichnet, dass** die Zusammensetzung B eine von der Säure des Puffersystems b) verschiedene organische Säure umfasst, vorzugsweise mit einem $pK_A$ von kleiner als 5, insbesondere einem $pK_A$ von kleiner als 4,5 und noch besser einem $pK_A$ von kleiner als 4, wobei der $pK_A$ gegebenenfalls in dem Bereich von 1 bis 5 und noch besser von 2 bis 4 liegt, vorzugsweise ausgewählt aus Citronensäure, Milchsäure, Apfelsäure, Weinsäure und Glycolsäure.

**27.** Verfahren nach einem der vorstehenden Ansprüche, **dadurch gekennzeichnet, dass** die Zusammensetzung B einen pH-Wert von kleiner oder gleich 4, noch besser kleiner oder gleich 3, insbesondere einen pH-Wert in dem Bereich von 1 bis 5, noch besser von 1,5 bis 4 und sogar noch besser von 2 bis 3 aufweist.

**28.** Verfahren nach einem der vorstehenden Ansprüche, **dadurch gekennzeichnet, dass** Wasser von 40 Gew.-% bis 99 Gew.-%, vorzugsweise von 50 Gew.-% bis 95 Gew.-% und bevorzugter von 60 Gew.-% bis 90 Gew.-% bezogen auf das Gesamtgewicht der Zusammensetzung B darstellt.

**29.** Färbeverfahren nach einem der vorstehenden Ansprüche, **dadurch gekennzeichnet, dass** es umfasst:

i) Schritt i) des Behandelns der Fasern unter Verwendung der kosmetischen Zusammensetzung A nach einem der vorstehenden Ansprüche, und dann
ii) Schritt ii) des Behandelns mit Zusammensetzung B nach einem der vorstehenden Ansprüche.

**30.** Färbeverfahren nach einem der vorstehenden Ansprüche, **dadurch gekennzeichnet, dass** es einen Spülschritt iii), vorzugsweise mit Wasser, zwischen den Schritten i) und ii) umfasst.

## Revendications

**1.** Procédé de teinture de fibres kératiniques, en particulier de fibres kératiniques humaines telles que les cheveux, dans lequel lesdites fibres sont traitées, en plusieurs étapes distinctes, comprenant :

i) au moins une étape i) de teinture desdites fibres à l'aide d'une composition cosmétique tinctoriale A, de préférence une composition aqueuse, comprenant au moins a) de l'indigo et/ou du henné,
ii) au moins une étape ii) de traitement desdites fibres, comprenant l'application sur les fibres d'une composition aqueuse B qui comprend de l'eau, b) éventuellement au moins un colorant direct autre que le henné et l'indigo, c) un système de tampon comprenant un mélange d'un acide et d'au moins un sel conjugué correspondant, la composition B ayant un pH inférieur ou égal à 5,
iii) éventuellement au moins une étape de rinçage intermédiaire desdites fibres, de préférence avec de l'eau, entre l'étape i) et l'étape ii) (ou entre les étapes ii) et i) (selon l'ordre des étapes i) et ii)).

2. Procédé selon la revendication précédente, dans lequel la composition A comprend de l'eau, comprend de préférence une teneur en eau allant de 10 % à 99 % en poids, plus particulièrement de 20 % à 90 % en poids et encore mieux de 40 % à 80 % en poids par rapport au poids de la composition A.

3. Procédé selon l'une quelconque des revendications précédentes, dans lequel le henné et/ou l'indigo sont sous forme de poudre.

4. Procédé selon l'une quelconque des revendications précédentes, dans lequel l'indigo est dérivé de plante(s) productrice(s) d'indigo, de préférence du genre *Indigofera* et plus particulièrement *Indigofera tinctoria.*

5. Procédé selon l'une quelconque des revendications précédentes, **caractérisé en ce que** le henné est du henné rouge.

6. Procédé selon l'une quelconque des revendications précédentes, **caractérisé en ce que** la composition A comprend au moins 0,1 % en poids de henné et/ou d'indigo, par rapport au poids total de ladite composition, plus préférentiellement de 0,2 % à 50 % en poids, encore mieux de 0,3 % à 40 % en poids, de préférence de 1 % à 30 % en poids par rapport au poids total de la composition A.

7. Procédé de teinture selon l'une des revendications précédentes, **caractérisé en ce que** la composition tinctoriale B comprend au moins un colorant naturel, de préférence choisi parmi la spinulosine, les orcéines, les polyphénols ou les orthodiphénols (également appelés ODP dans le reste de la description), la curcumine, les indoles tels que l'isatine ou l'indole-2,3-dione, les indigoïdes y compris l'indigo, les phtalocyanines et les porphyrines notamment complexées à un métal, les iridoïdes glycosylés ou non, les colorants de chromène, les colorants anthraquinoniques et naphtoquinoniques, la juglone, la spinulosine, les colorants de chromène ou chromane, tels que les néoflavanols et les néoflavanones, les flavanols ; les anthocyanidols, les orcéines, les bétalaïnes et des mélanges correspondants.

8. Procédé selon l'une quelconque des revendications précédentes, **caractérisé en ce que** la composition tinctoriale B comprend au moins un colorant naturel choisi parmi le carmin, le carmin d'ammonium, le diosindigo, la chlorophylline, l'hématéine, l'orcéine, les extraits suivants : cassis, myrtille, riz noir, peau de raisin, hibiscus, chou rouge, carotte noire, sureau, rhubarbe, monascus, patate douce violette, goji, radis, orcéine, gardénia, diospyros kaki, campeachy, quebracho, et des mélanges correspondants.

9. Procédé selon l'une quelconque des revendications précédentes, **caractérisé en ce que** la composition tinctoriale B comprend au moins un colorant direct synthétique, de préférence choisi parmi les colorants directs anioniques, encore mieux parmi les colorants directs anioniques choisis parmi ceux de formules (I), (II), (III), (IV), (V), (VI), (VII), (VIII), (IX), (X), (XI) et (XII) ci-dessous, ainsi que leurs formes mésomères et tautomères :

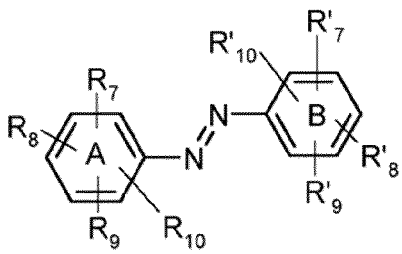

(I)

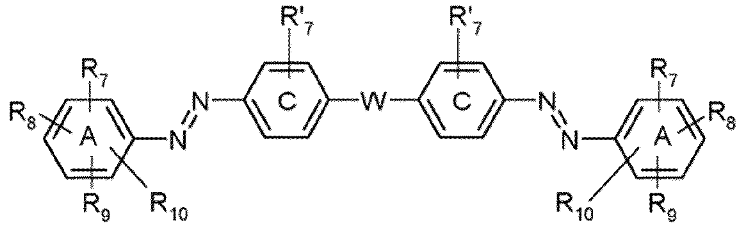

(II)

dans lesquelles formules (I) et (II) :

- $R_7$, $R_8$, $R_9$, $R_{10}$, $R'_7$, $R'_8$, $R'_9$ et $R'_{10}$, qui peuvent être identiques ou différents, représentant un atome d'hydrogène ou un groupe choisi parmi :
- alkyle ;
- alcoxy, alkylthio ;
- hydroxyle, mercapto ;
- nitro, nitroso R°-X'-C(X)-, R°-X'-C(X)-X"-, R°
- représentant un atome d'hydrogène ou un groupe alkyle ou aryle ; X, X' et X", qui peuvent être identiques ou différents, représentant un atome d'oxygène ou de soufre, ou NR, R représentant un atome d'hydrogène ou un groupe alkyle ;
- $(O)_2S(O^-)$-, $M^+$, $M^+$ représentant un atome d'hydrogène ou un contre-ion cationique ;
- (O) $CO^-$-, $M^+$, $M^+$ étant tel que défini précédemment ;
- R"-$S(O)_2$-, R" représentant un atome d'hydrogène ou un groupe alkyle, aryle, (di) (alkyl)amino ou aryl(alkyl)amino ; préférentiellement un groupe phénylamino ou phényle ;
- R‴ -$S(O)_2$-X'-, R‴ représentant un alkyle ou un groupe aryle éventuellement substitué, X' étant tel que défini précédemment ;
- (di) (alkyl)amino ;
- aryl(alkyl)amino éventuellement substitué par un ou plusieurs groupes choisis parmi i) nitro ; ii) nitroso ; iii) $(O)_2S(O^-)$-, $M^+$ et iv) alcoxy, $M^+$ étant tel que défini précédemment ;
- hétéroaryle éventuellement substitué ;
- cycloalkyle ;
- Ar-N=N-, Ar représentant un groupe aryle éventuellement substitué ;
- ou en variante deux groupes contigus $R_7$ avec $R_8$ ou $R_8$ avec $R_9$ ou $R_9$ avec $R_{10}$ formant ensemble un groupe benzo condensé A' ; et $R'_7$ avec $R'_8$ ou $R'_8$ avec $R'_9$ ou $R'_9$ avec $R'_{10}$ formant ensemble un groupe benzo condensé B' ; avec A' et B' éventuellement substitués par un ou plusieurs groupes choisis parmi i) nitro ; ii) nitroso ; iii) $(O)_2S$ $(O^-)$-, $M^+$ ; iv) hydroxyle ; v) mercapto ; vi) (di) (alkyl)amino ; vii) R°-C(X)-X'- ; viii) R°-X'-C(X)- ; ix) R°-X'-C(X)-X"- ; x) Ar-N=N- et xi) aryl (alkyl)amino éventuellement substitué ; $M^+$, R°, X, X', X" et Ar étant tels que définis précédemment ;
- W représentant une liaison sigma $\sigma$, un atome d'oxygène ou de soufre, ou un radical divalent i) -NR-, R étant tel que défini précédemment, ou ii) méthylène - $C(R_a)$ $(R_b)$-, $R_a$ et $R_b$, qui peuvent être identiques ou différents, représentant un atome d'hydrogène ou un groupe aryle, ou en variante $R_a$ et $R_b$ formant, conjointement avec l'atome de carbone qui les porte, un spiro cycloalkyle ;

étant entendu que les formules (I) et (II) comprennent au moins un radical sulfonate $(O)_2S(O^-)$-, $M^+$ ou un radical carboxylate $(O)CO^-$-, $M^+$ sur l'un des cycles A, A', B, B' ou C ; préférentiellement sulfonate de sodium ;

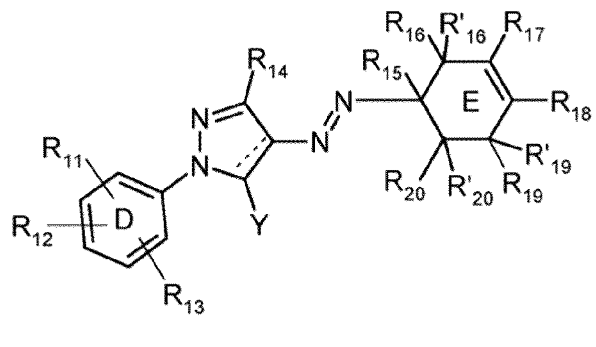

(III)

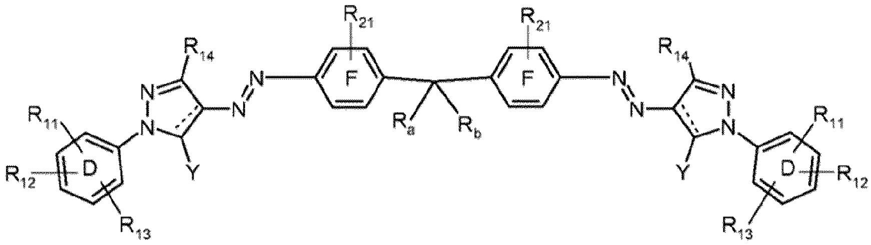

(IV)

dans lesquelles formules (III) et (IV) :

- $R_{11}$ , $R_{12}$ et $R_{13}$, qui peuvent être identiques ou différents, représentant un atome d'hydrogène ou d'halogène, un groupe alkyle ou $(O)_2S(O^-)$-, $M^+$, $M^+$ étant tel que défini précédemment ;
- $R_{14}$ représentant un atome d'hydrogène, un groupe alkyle ou un groupe $C(O)O^-$-, $M^+$, $M^+$ étant tel que défini précédemment ;
- $R_{15}$ représentant un atome d'hydrogène ;
- $R_{16}$ représentant un groupe oxo, auquel cas $R'_{16}$ est absent, ou en variante $R_{15}$ avec $R_{16}$ formant ensemble une double liaison ;
- $R_{17}$ et $R_{18}$, qui peuvent être identiques ou différents, représentant un atome d'hydrogène, ou un groupe choisi parmi :
  - $(O)_2S(O^-)$-, $M^+$, $M^+$ étant tel que défini précédemment ;
  - Ar-O-$S(O)_2$-, Ar représentant un groupe aryle éventuellement substitué, préférentiellement un phényle éventuellement substitué par un ou plusieurs groupes alkyle ;
  - $R_{19}$ et $R_{20}$ formant ensemble soit une double liaison, soit un groupe benzo D', qui est éventuellement substitué ;
  - $R'_{16}$, $R'_{19}$ et $R'_{20}$, qui peuvent être identiques ou différents, représentant un atome d'hydrogène ou un groupe alkyle ou hydroxyle ;
  - $R_{21}$ représentant un atome d'hydrogène ou un groupe alkyle ou alcoxy ;
  - $R_a$ et $R_b$, qui peuvent être identiques ou différents, étant tels que définis précédemment ;
  - Y représentant soit un groupe hydroxyle, soit un groupe oxo ;
  - ------ représentant une simple liaison lorsque Y est un groupe oxo ; et représentant une double liaison lorsque Y représente un groupe hydroxyle ;

étant entendu que les formules (III) et (IV) comprennent au moins un radical sulfonate $(O)_2S(O^-)$-, $M^+$ ou un radical carboxylate $C(O)O^-$-, $M^+$ sur l'un des cycles D ou E ; préférentiellement sulfonate de sodium ;

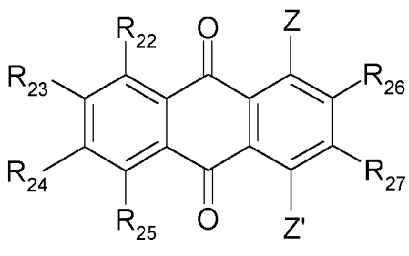

(V)

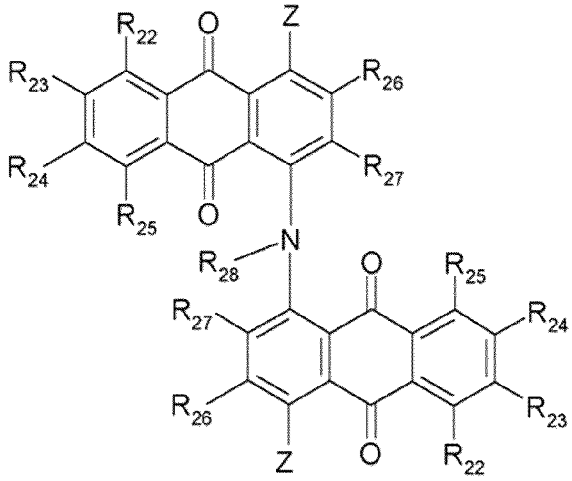

(VI)

dans lesquelles formules (V) et (VI) :

- $R_{22}$, $R_{23}$, $R_{24}$, $R_{25}$, $R_{26}$ et $R_{27}$, qui peuvent être identiques ou différents, représentant un atome d'hydrogène ou d'halogène, ou un groupe choisi parmi :

  - alkyle ;
  - hydroxyle, mercapto ;
  - alcoxy, alkylthio ;
  - aryloxy ou arylthio éventuellement substitué ;
  - aryl(alkyl)amino éventuellement substitué par un ou plusieurs groupes choisis parmi alkyle et $(O)_2S(O^-)$-, $M^+$, $M^+$ étant tel que défini précédemment ;
  - (di) (alkyl)amino ;
  - (di) (hydroxyalkyl)amino ;
  - $(O)_2S(O^-)$-, $M^+$, $M^+$ étant tel que défini précédemment ;
  - Z' représentant un atome d'hydrogène ou un groupe $NR_{28}R_{29}$, $R_{28}$ et $R_{29}$, qui peuvent être identiques ou différents, représentant un atome d'hydrogène ou un groupe choisi parmi :

    - alkyle ;
    - polyhydroxyalkyle tel que hydroxyéthyle ;
    - aryle éventuellement substitué par un ou plusieurs groupes, plus particulièrement i) alkyle tel que méthyle, n-dodécyle, n-butyle ; ii) $(0)\ _2S\ (O^-)$ -, $M^+$, $M^+$ étant tel que défini précédemment ; iii) R°-C(X)-X'-, R°-X'-C(X)-, R°-X'-C(X)-X"-, R°, X, X' et X" étant tels que définis précédemment ;
    - cycloalkyle ;
    - Z représentant un groupe choisi parmi hydroxyle et $NR'_{28}R'_{29}$, $R'_{28}$ et $R'_{29}$, qui peuvent être identiques ou différents, représentant les mêmes atomes ou groupes que $R_{28}$ et $R_{29}$ tels que définis précédemment ;

étant entendu que les formules (V) et (VI) comprennent au moins un radical sulfonate $(O)_2S(O^-)$-, $M^+$ ou un radical carboxylate $C(O)O^-$-, $M^+$ ; préférentiellement sulfonate de sodium ;

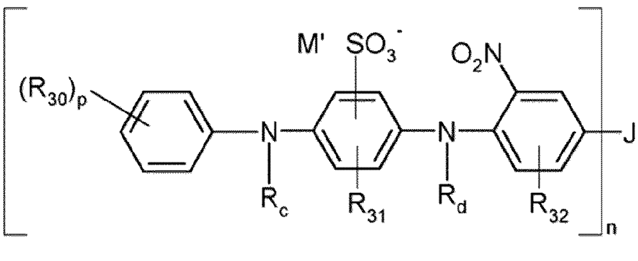

(VII)

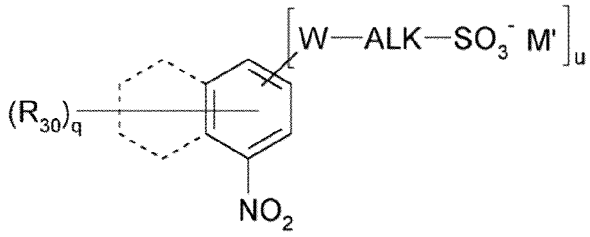

(VIII)

dans lesquelles formules (VII) et (VIII) :

- $R_{30}$, $R_{31}$ et $R_{32}$, qui peuvent être identiques ou différents, représentant un atome d'hydrogène ou d'halogène, ou un groupe choisi parmi :

  - alkyle ;
  - alcoxy éventuellement substitué par un ou plusieurs groupes hydroxyle, alkylthio éventuellement substitué par un ou plusieurs groupes hydroxyle ;
  - hydroxyle, mercapto ;
  - nitro, nitroso ;
  - polyhalogénoalkyle ;
  - R°-C(X)-X'-, R°-X'-C(X)-, R°-X'-C (X)-X"-, R°, X, X' et X" étant tels que définis précédemment ;
  - $(O)_2S(O^-)$-, $M^+$, $M^+$ étant tel que défini précédemment ;
  - (O) $CO^-$-, $M^+$, $M^+$ étant tel que défini précédemment ;
  - (di) (alkyl)amino ;
  - (di) (hydroxyalkyl)amino ;
  - hétérocycloalkyle ;
  - $R_c$ et $R_d$, qui peuvent être identiques ou différents, représentant un atome d'hydrogène ou un groupe alkyle ;
  - W étant tel que défini précédemment ;
  - ALK représentant un groupe $C_1$-$C_6$ alkylène divalent linéaire ou ramifié ;
  - n étant 1 ou 2 ;
  - p représentant un entier inclusivement entre 1 et 5 ;
  - q représentant un entier inclusivement entre 1 et 4 ;
  - u étant 0 ou 1 ;
  - lorsque n est 1, J représentant un groupe nitro ou nitroso ;
  - lorsque n est 2, J représentant un atome d'oxygène ou de soufre, ou un radical divalent $-S(O)_m-$, m représentant un entier 1 ou 2 ;
  - M' représentant un atome d'hydrogène ou un contre-ion cationique ;
  -

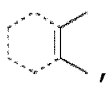,

qui peut être présent ou absent, représentant un groupe benzo éventuellement substitué par un ou plusieurs groupes $R_{30}$ tels que définis précédemment ;

étant entendu que les formules (VII) et (VIII) comprennent au moins un radical sulfonate $(O)_2S(O^-)$-, $M^+$ ou un radical carboxylate $C(O)O^-$-, $M^+$ ;

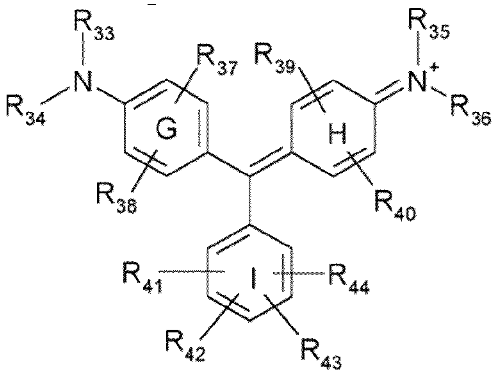

(IX)

dans laquelle formule (IX) :

- $R_{33}$, $R_{34}$, $R_{35}$ et $R_{36}$, qui peuvent être identiques ou différents, représentant un atome d'hydrogène ou un groupe choisi parmi alkyle, aryle éventuellement substitué et arylalkyle éventuellement substitué ;
- $R_{37}$, $R_{38}$, $R_{39}$, $R_{40}$, $R_{41}$, $R_{42}$, $R_{43}$ et $R_{44}$, qui peuvent être identiques ou différents, représentant un atome d'hydrogène ou un groupe choisi parmi :

  - alkyle ;
  - alcoxy, alkylthio ;
  - (di) (alkyl)amino ;
  - hydroxyle, mercapto ;
  - nitro, nitroso R°-X'-C(X)-, R°-X'-C(X)-X"-, R°
  - représentant un atome d'hydrogène ou un groupe alkyle ou aryle ; X, X' et X", qui peuvent être identiques ou différents, représentant un atome d'oxygène ou de soufre, ou NR, R représentant un atome d'hydrogène ou un groupe alkyle ;
  - $(O)_2S(O^-)$-, $M^+$, $M^+$ représentant un atome d'hydrogène ou un contre-ion cationique ;
  - $(O)CO^-$-, $M^+$, $M^+$ étant tel que défini précédemment ;
  - ou en variante deux groupes contigus $R_{41}$ avec $R_{42}$ ou $R_{42}$ avec $R_{43}$ ou $R_{43}$ avec $R_{44}$ formant ensemble un groupe benzo condensé : I' ; I' étant éventuellement substitué par un ou plusieurs groupes choisis parmi i) nitro ; ii) nitroso ; iii) $(O)_2S(O^-)$-, $M^+$ ; iv) hydroxyle ; v) mercapto ;

vi) (di) (alkyl)amino ; vii) R°-C(X)-X'- ; viii) R°-X'-C(X)- et ix) R°-X'-C(X)-X"- ; $M^+$, R°, X, X' et X" étant tels que définis précédemment ;
plus particulièrement, $R_{37}$ à $R_{40}$ représentant un atome d'hydrogène, et $R_{41}$ à $R_{44}$, qui peuvent être identiques ou différents, représentant un groupe hydroxyle ou $(O)_2S(O^-)$-, $M^+$ ; et lorsque $R_{43}$ avec $R_{44}$ forment ensemble un groupe benzo, il est préférentiellement substitué par un groupe $(O)_2S(O^-)$- ;
étant entendu qu'au moins un des cycles G, H, I ou I' comprend au moins un radical sulfonate $(O)_2S(O^-)$- ou un radical carboxylate $C(O)O^-$- ;

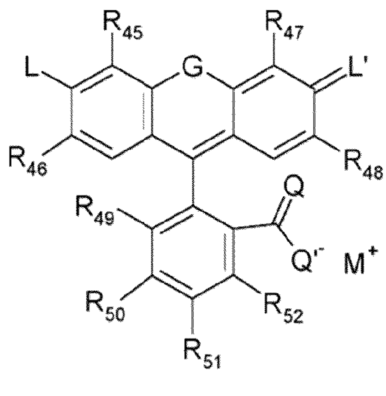

(X)

dans laquelle formule (X) :

- $R_{45}$, $R_{46}$, $R_{47}$ et $R_{48}$, qui peuvent être identiques ou différents, représentant un atome d'hydrogène ou d'halogène ;
- $R_{49}$, $R_{50}$, $R_{51}$ et $R_{52}$, qui peuvent être identiques ou différents, représentant un atome d'hydrogène ou d'halogène, ou un groupe choisi parmi :

  - alkyle ;
  - alcoxy, alkylthio ;
  - hydroxyle, mercapto ;
  - nitro, nitroso ;
  - $(O)_2S(O^-)$-, $M^+$, $M^+$ représentant un atome d'hydrogène ou un contre-ion cationique ;
  - $(O)CO^-$-, $M^+$, $M^+$ étant tel que défini précédemment ;

préférablement, $R_{49}$, $R_{50}$, $R_{51}$ et $R_{52}$ représentant un atome d'hydrogène ou d'halogène ;

- G représentant un atome d'oxygène ou de soufre ou un groupe $NR_e$, $R_e$ étant tel que défini précédemment ;
- L représentant un alcoxyde $O^-$, $M^+$ ; un thioalcoxyde $S^-$, $M^+$ ou un groupe $NR_f$, $R_f$ représentant un atome d'hydrogène ou un groupe alkyle et $M^+$ étant tel que défini précédemment ;
- L' représentant un atome d'oxygène ou de soufre ou un groupe ammonium : $N^+R_fR_g$, $R_f$ et $R_g$, qui peuvent être identiques ou différents, représentant un atome d'hydrogène, ou un groupe alkyle ou un groupe aryle qui est éventuellement substitué ;
- Q et Q', qui peuvent être identiques ou différents, représentant un atome d'oxygène ou de soufre ;
- $M^+$ étant tel que défini précédemment ;

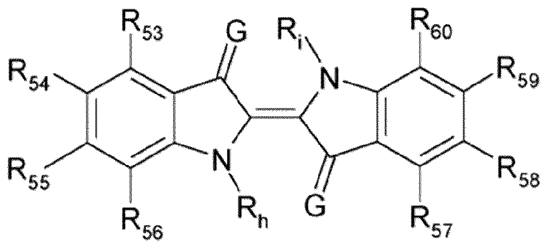

(XI)

dans laquelle formule (XI) :

- $R_{53}$, $R_{54}$, $R_{55}$, $R_{56}$, $R_{57}$, $R_{58}$, $R_{59}$ et $R_{60}$, qui peuvent être identiques ou différents, représentant un atome d'hydrogène ou un groupe choisi parmi :

  - alkyle ;
  - alcoxy, alkylthio ;
  - hydroxyle, mercapto ;
  - nitro, nitroso ;R°-X'-C(X)-X"-, R°
  - représentant un atome d'hydrogène ou un groupe alkyle ou aryle ; X, X' et X' ' , qui peuvent être identiques ou différents, représentant un atome d'oxygène ou de soufre, ou NR, R représentant un atome d'hydrogène ou un groupe alkyle ;
  - $(O)_2S(O^-)$-, $M^+$, $M^+$ représentant un atome d'hydrogène ou un contre-ion cationique ;
  - (O) $CO^-$-, $M^+$, $M^+$ étant tel que défini précédemment ;
  - G représentant un atome d'oxygène ou de soufre ou un groupe $NR_e$, $R_e$ étant tel que défini précédemment ;
  - $R_i$ et $R_h$, qui peuvent être identiques ou différents, représentant un atome d'hydrogène ou un groupe alkyle ;

étant entendu que la formule (XI) comprend au moins un radical sulfonate $(O)_2S(O^-)$-, $M^+$ ou un radical carboxylate $C(O)O^-$-, $M^+$ ;

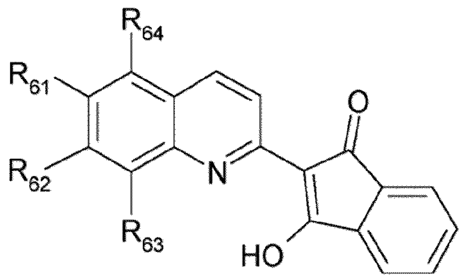

(XII)

- $R_{61}$ représentant un atome d'hydrogène ou d'halogène ou un groupe alkyle ;
- $R_{62}$, $R_{63}$, et $R_{64}$, qui peuvent être identiques ou différents, représentant un atome d'hydrogène ou un groupe $(O)_2S(O^-)$-, $M^+$, $M^+$ représentant un atome d'hydrogène ou un contre-ion cationique ;
- ou en variante $R_{61}$ avec $R_{62}$, ou $R_{61}$ avec $R_{64}$, formant ensemble un groupe benzo éventuellement substitué par un ou plusieurs groupes $(O)_2S(O^-)$-, $M^+$, $M^+$ représentant un atome d'hydrogène ou un contre-ion cationique ;

étant entendu que la formule (XII) comprend au moins un radical sulfonate $(O)_2S(O^-)$-,
l'au moins un colorant direct synthétique étant préférablement choisi parmi des colorants directs azoïques, spécifiquement ceux de formule (I), et des colorants anthraquinoniques, spécifiquement ceux de formule (V).

**10.** Procédé selon l'une quelconque des revendications précédentes, **caractérisé en ce que** le ou les colorants directs sont choisis parmi l'acide 1,2-dihydroxy-9,10-anthraquinone-3-sulfonique, le sel monosodique de l'acide 2-[(9,10-dihydro-4-hydroxy-9,10-dioxo-1-anthracényl)amino]-5-méthylbenzènesulfonique, le sel monosodique de l'acide 4-[(2-hydroxy-1-naphtyl)azo]benzènesulfonique, le sel disodique de l'acide 6-hydroxy-5-[(4-sulfophényl)azol-2-naphtalènesulfonique, le sel disodique de l'acide 5-amino-4-hydroxy-3-(phénylazo)-2,7-naphtalènedisulfonique, le sel disodique de l'acide 1-amino-2-(4'-nitrophénylazo)-7-phénylazo-8-hydroxy-3,6-naphtalènedisulfonique, le sel disodique de hydroxyde de N-éthyl-N-[4-[[4-[éthyl[3-sulfophényl]méthyl]amino]phényl](2-sulfophényl)méthylène]-2,5-cyclohexadiène-1-ylidène]-3-sulfobenzèneméthanaminium, le sel disodique de l'acide 2,2'-[(9,10-dihydro-9,10-dioxo-1,4-anthracènediyl)diimino]bis[5-méthyl]benzènesulfonique, le sel trisodique de l'acide 5-hydroxy-1-(4-sulfophényl)-4-(4-sulfophénylazo)pyrazole-3-carboxylique, le 4-[(9,10-dihydro-4-hydroxy-9,10-dioxo-1-anthryl)amino]toluène-3-sulfonate de sodium, le sel trisodique de l'acide 7-hydroxy-8-[(4-sulfo-1-naphtalényl)azo]-1,3-naphtalènedisulfonique, et un mélange de ces composés.

**11.** Procédé selon l'une quelconque des revendications précédentes, **caractérisé en ce que** le ou les colorants directs représentent une teneur totale d'au moins 0,05 % en poids, encore mieux d'au moins 0,1 % en poids, de préférence d'au moins 0,15 % en poids, de préférence allant de 0,05 % à 5 %, encore mieux de 0,1 % à 3 % en poids et encore mieux de 0,15 % à 2 % en poids par rapport au poids total de la composition B.

**12.** Procédé selon l'une quelconque des revendications précédentes, **caractérisé en ce que** le système de tampon comprend un acide inorganique choisi de préférence parmi les acides inorganiques dotés d'un pKa (pka1) inférieur à 4,5, de préférence allant de 1 à 4 et encore mieux de 2 à 4.

**13.** Procédé selon l'une quelconque des revendications précédentes, **caractérisé en ce que** l'acide inorganique est choisi parmi les acides à base de phosphore, tels que l'acide phosphorique, les acides à base d'halogène, tels que l'acide chlorhydrique, les acides à base de soufre, tels que l'acide sulfurique, et des mélanges correspondants.

**14.** Procédé selon l'une quelconque des revendications précédentes, **caractérisé en ce que** l'acide inorganique est l'acide phosphorique.

**15.** Procédé selon l'une quelconque des revendications précédentes, **caractérisé en ce que** le ou les sels d'acides conjugués sont choisis parmi les sels d'acides inorganiques, de préférence parmi les sels de phosphates inorganiques, en particulier choisis parmi le dihydrogénophosphate de potassium $KH_2PO_4$, le dihydrogénophosphate de sodium $NaH_2PO_4$, l'hydrogénophosphate dipotassique $K_2HPO_4$, l'hydrogénophosphate disodique $Na_2HPO_4$, le phosphate de potassium $K_3PO_4$ et le phosphate de sodium $Na_3PO_4$ et des mélanges correspondants, de préférence parmi le dihydrogénophosphate de potassium $KH_2PO_4$ et le dihydrogénophosphate de sodium $NaH_2PO_4$.

**16.** Procédé selon l'une quelconque des revendications précédentes, **caractérisé en ce que** l'acide, de préférence l'acide inorganique, représente de 0,05 % à 5 % en poids, de préférence de 0,1 % à 3 % en poids et encore mieux de 0,15 % à 2 % en poids par rapport au poids total de la composition B.

**17.** Procédé selon l'une quelconque des revendications précédentes, **caractérisé en ce que** le sel d'acide conjugué, de préférence le sel d'acide inorganique, est présent en une teneur allant de 0,05 à 5 % en poids, de préférence de 0,1 à 3 % en poids et encore mieux de 0,15 à 2 % en poids, par rapport au poids total de la composition B.

**18.** Procédé selon l'une quelconque des revendications précédentes, **caractérisé en ce que** l'acide, de préférence l'acide inorganique, et son ou ses sels conjugués sont utilisés dans la composition selon l'invention en un rapport molaire d'acide/sel(s) conjugué(s) dans la plage de 1:10 à 10:1, encore mieux de 1:5 à 5:1, même encore mieux de 1:3 à 3:1 et plus préférablement de 1:2 à 2:1.

**19.** Procédé selon l'une quelconque des revendications précédentes, **caractérisé en ce que** la composition B comprend un ou plusieurs composés liquides dotés d'une valeur de paramètre de solubilité Hansen $\delta H$ inférieure à 16 $MPa^{1/2}$.

**20.** Procédé selon la revendication 19, **caractérisé en ce que** le ou les composés liquides dotés d'une valeur de paramètre de solubilité Hansen $\delta H$ inférieure à 16 $MPa^{1/2}$ sont choisis parmi :

- éthers d'alcool, en particulier $C_1$-$C_4$ éthers de $C_5$-$C_{30}$ alcools, qui sont préférablement saturés, linéaires ou ramifiés, éventuellement interrompus par une ou plusieurs fonctions éther non adjacentes ;
- esters aliphatiques d'acides carboxyliques en $C_1$-$C_4$ et de $C_3$-$C_{10}$ monoalcools ou alcools polyhydroxylés, interrompus par une ou plusieurs fonctions éther non adjacentes ;
- éthers aromatiques, en particulier de $C_6$-$C_{10}$, d'un $C_1$-$C_6$ alkyle portant éventuellement un groupe hydroxyle,
- éthers de ($C_6$-$C_{10}$) aryl ($C_1$-$C_6$) alkyle, d'un $C_1$-$C_6$ alkyle portant éventuellement un groupe hydroxyle,
- alcanols portant des substituants aryle, préférablement pour lesquels la partie aryle est une partie $C_6$-$C_{10}$ aryle, avantageusement une partie $C_6$ aryle, et la partie alkyle de l'alcanol est une partie $C_1$-$C_4$ alkyle, cette partie alkyle pouvant être terminée ou interrompue par un hétéroatome, avantageusement l'oxygène, ou un groupe hydroxyle, de préférence tel que l'alcool benzylique ;
- lactones, préférablement de formule (iii), et également des mélanges correspondants, avec :

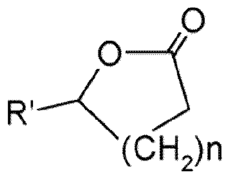

(iii)

dans laquelle R' représente un hydrogène, un $C_1$-$C_8$ alkyle linéaire ou ramifié, un $C_1$-$C_4$ hydroxyalkyle linéaire ou ramifié, n est égal à 1, 2 ou 3, et préférablement R' représente un hydrogène, un $C_1$-$C_6$ alkyle linéaire ou ramifié ou un $C_1$-$C_2$ hydroxyalkyle linéaire ou ramifié.

**21.** Procédé selon l'une ou l'autre des revendications 19 ou 20, **caractérisé en ce que** c) le ou les composés liquides dotés d'une valeur de paramètre de solubilité Hansen $\delta H$ inférieure à 16 $MPa^{1/2}$ sont choisis parmi les éthers d'alcool, les esters aliphatiques, les éthers aromatiques, les alcanols porteurs de substituants aryle, et leurs mélanges, de préférence l'alcool benzylique, le phényléthanol et le phénoxyéthanol.

**22.** Procédé selon l'une quelconque des revendications 19 à 21, **caractérisé en ce que** le ou les composés liquides dotés d'une valeur de paramètre de solubilité Hansen $\delta H$ inférieure à 16 $MPa^{1/2}$ représentent une teneur totale allant de 0,1 % à 35 % en poids, de préférence de 2 % à 20 % en poids et encore mieux de 0,3 % à 5 % en poids, par rapport au poids total de la composition B.

**23.** Procédé selon l'une quelconque des revendications précédentes, **caractérisé en ce que** la composition B comprend un ou plusieurs alcools linéaires ou ramifiés, de préférence un ou plusieurs monoalcools linéaires ou ramifiés contenant de 1 à 8 atomes de carbone, de préférence ayant de 1 à 4 atomes de carbone, et plus préférablement comprend un ou plusieurs monoalcools linéaires contenant de 1 à 8 atomes de carbone et encore mieux de 1 à 4 atomes de carbone.

**24.** Procédé selon la revendication 23, **caractérisé en ce que** l'alcool ou les alcools linéaires ou ramifiés contenant de 1 à 8 atomes de carbone sont choisis parmi l'éthanol, le propanol, le butanol, l'isopropanol, l'isobutanol et leurs mélanges, de préférence l'éthanol.

**25.** Procédé selon l'une quelconque des revendications 19 à 24, **caractérisé en ce que** le rapport pondéral de la quantité totale de composé(s) liquide(s) doté(s) d'une valeur de paramètre de solubilité Hansen $\delta H$ inférieure à 16 $MPa^{1/2}$ sur la quantité totale d'alcool(s) linéaire(s) ou ramifié (s) contenant de 1 à 8 atomes de carbone est inférieur ou égal à 1 et de préférence se situe dans la plage de 0,1 à 0,9.

**26.** Procédé selon l'une quelconque des revendications précédentes, **caractérisé en ce que** la composition B comprend un acide organique différent de l'acide du système de tampon b), de préférence doté d'un $pK_a$ inférieur à 5, en particulier un $pK_a$ inférieur à 4,5 et encore mieux un $pK_a$ inférieur à 4, le $pK_a$ pouvant se situer dans la plage de 1 à 5 et encore mieux de 2 à 4, de préférence choisi parmi l'acide citrique, l'acide lactique, l'acide malique, l'acide tartrique et l'acide glycolique.

**27.** Procédé selon l'une quelconque des revendications précédentes, **caractérisé en ce que** la composition B a un pH inférieur ou égal à 4, encore mieux inférieur ou égal à 3, en particulier un pH se situant dans la plage de 1 à 5, encore mieux de 1,5 à 4 et encore mieux de 2 à 3.

**28.** Procédé selon l'une quelconque des revendications précédentes, **caractérisé en ce que** l'eau représente de 40 à 99 % en poids, de préférence de 50 à 95 % en poids et plus préférentiellement de 60 à 90 % en poids par rapport au poids total de la composition B.

**29.** Procédé de teinture selon l'une quelconque des revendications précédentes, **caractérisé en ce qu'**il implique :

i) une étape i) de traitement desdites fibres en utilisant la composition cosmétique A telle que définie selon l'une quelconque des revendications précédentes, et ensuite
ii) une étape ii) de traitement par la composition B telle que définie selon l'une quelconque des revendications précédentes.

**30.** Procédé de teinture selon l'une quelconque des revendications précédentes, **caractérisé en ce qu'**il implique une étape iii) de rinçage, de préférence avec de l'eau, entre les étapes i) et ii).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 3014315 A1
- FR 3029405
- US 4131576 A
- EP 503853 A
- FR 2416723
- US 2798053 A
- US 2923692 A
- US 3915921 A
- US 4509949 A
- EP 0173109 A
- WO 0031154 A
- EP 750899 A
- US 5089578 A
- WO 0068282 A
- WO 9844012 A


### Non-patent literature cited in the description


- Dyes, Natural. *Kirk-Othmer Encyclopedia of Chemical Technology*
- Henna. *Encyclopedia Britannica*
- Hair preparation. Ullmann's Encyclopedia of Industrial Chemistry. Wiley-VCH Verlag GmbH & Co. KGaA, 2006
- *Kirk-Othmer Encyclopedia of Chemical Technology,* 17 April 2009
- *Chem. Rev.,* 2011, vol. 111, 2537-2561
- Colour Index. The Society of Dyers and Colourists
- **CHARLES M. HANSEN.** Hansen solubility parameters: A User's Handbook. CRC Press, 2000, 167-185
- Handbook of Solubility Parameters and Other Cohesion Parameters. CRC Press, 95-121, 177-185
- *The relation between surface tension and solubility parameter in liquids,* 1978
- Self-assembling amphiphilic polyelectrolytes and their nanostructures. *Chinese Journal of Polymer Science,* 2000, vol. 18 (40), 323-336
- Micelle formation of random copolymers of sodium 2-(acrylamido)-2-methylpropanesulfonate and a nonionic surfactant macromonomer in water as studied by fluorescence and dynamic light scattering. *Macromolecules,* 2000, vol. 33 (10), 3694-3704
- Solution properties of micelle networks formed by nonionic moieties covalently bound to a polyelectrolyte: salt effects on rheological behavior. *Langmuir,* 2000, vol. 16 (12), 5324-5332
- Stimuli responsive amphiphilic copolymers of sodium 2-(acrylamido)-2-methylpropanesulfonate and associative macromonomers. *Polym. Preprint, Div. Polym. Chem.,* 1999, vol. 40 (2), 220-221
- **G. FONNUM ; J. BAKKE ; FK. HANSEN.** *Colloid Polym. Sci.,* 1993, vol. 271, 380-389